# EUROPEAN PATENT APPLICATION

(11) **EP 4 180 460 A1**
(43) Date of publication of application: **17.05.2023**
(21) Application number: 21838472.5
(22) Date of filing: 09.07.2021
(51) Int. Cl.: C07K 19/00, C12N 9/22, C12N 15/113, C12N 15/62, C12N 15/90

(54) **SYSTEM AND METHOD FOR EDITING NUCLEIC ACID**

(30) Priority: 10.07.2020 CN 202010663076
(71) Applicant: Institute Of Zoology, Chinese Academy Of Sciences, Beijing 100101 (CN); Beijing Institute for Stem Cell and Regenerative Medicine, Beijing 100101 (CN)
(72) Inventor: LI, Wei, Beijing 100101 (CN); ZHOU, Qi, Beijing 100101 (CN); WANG, Chenxin, Beijing 100101 (CN); FANG, Sen, Beijing 100101 (CN); JIAO, Guanyi, Beijing 100101 (CN)
(74) Representative: Bottero, Carlo
(86) International application number: PCT/CN2021/105564
(87) International publication number: WO 2022/007959

(57) **Abstract**

Provided are a system for use in editing a nucleic acid, a reagent kit, uses thereof, and a method for editing a nucleic acid. The system, the reagent kit and the method are applicable in fracturing a double-stranded target nucleic acid and forming an overhangs on a distal end thereof, and are applicable in inserting or substituting a target nucleic acid into a nucleic acid molecule (such as a genomic DNA) of interest.

## Description

### Technical Field

The present application relates to the field of genetic engineering and molecular biology. In particular, the present application relates to a system and kit for editing nucleic acid and use thereof, and a method of editing nucleic acid. The system, kit and method of the present application can be used to fragment a double-stranded target nucleic acid and form an overhang at an end thereof (particularly the cleaved end), and can be used to insert the target nucleic acid in a nucleic acid molecule of interest (e.g., a genomic DNA) or to substitute a nucleotide fragment in a nucleic acid molecule of interest (e.g., a genomic DNA) with the target nucleic acid.

### Background Art

Gene editing technology is a hot field of biomedical research, and has broad application prospects in the clinical treatment of genetic diseases, the construction of animal models, and the genetic breeding of crops. Gene editing technology includes deletion, addition and substitution of a single nucleotide or DNA sequence at a specific site in the genome. Site-directed knock-in of exogenous genes can be achieved by homologous dependent recombination (HDR): by introducing a 500-3000bp homologous arm on each of two sides of the exogenous gene, precise site-specific integration of the exogenous gene can be achieved, but its efficiency is very low, only about 0.01%. By using artificially constructed nucleases such as ZFN (zinc-finger nucleases), TALEN (transcription activator-like effector nucleases) or CRISPR/Cas9 (clustered regularly interspaced short palindromic repeats/CRISPR-associated protein-9 nucleases), a targeted site of the genome can be cut to generate a DNA double strand fragment (DSB), which can promote the site-directed knock-in of an exogenous gene mediated by homologous recombination. However, since most mammalian cells mainly rely on NHEJ (non-homologous end joining) for DSB repair, the efficiency of site-directed knock-in based on nucleases and homologous recombination is still very low, generally around 1%. In addition, since homologous recombination only occurs in the S/G2 phase of the cell cycle, the site-directed integration of exogenous genes cannot be achieved by the above method for most somatic cells at the terminal differentiation stage.

The site-directed integration of exogenous DNA fragments can also be achieved using linear single-stranded DNA as a donor. Both ends of the single-stranded DNA donor have a 30-50nt homology arm, and after a specific site in the genome is cut by using nuclease, the single-strand is integrated into the DSB site by means of SDSA (synthesis dependent strand annealing), thereby realizing integration at the specific site of the genome. Integration based on a linear single-stranded DNA is more efficient than HDR but less precise: extra base insertions and deletions often occur at the junction at the 5' end of single-stranded DNA. In addition, chemical synthesis of long linear DNA single strands is expensive and it is difficult to obtain long linear DNA single strands. Therefore, this method is not suitable for site-directed knock-in of exogenous genes with large fragments (greater than 1Kb). In addition, when the inserted fragment exceeds 1Kb, its integration efficiency will also be significantly reduced.

NHEJ-based site-directed knock-in, such as HITI (Homology-independent target integration) technology, does not rely on the homology arms at both ends of the exogenous gene, in which the nuclease cuts the specific site on the genome and also cuts the donor vector, subsequently, linearized exogenous gene DNA fragment is inserted into the fragmentation site of the genome through the NHEJ DNA repair pathway. NHEJ-based site-directed knock-in is not directional, and the position of the linker is often imprecise, which is prone to extra base insertions or deletions. The MMEJ-based site-directed knock-in method is based on NHEJ, which introduces micro-homology arms at both ends of the exogenous gene, but the efficiency is still very low.

Prime Editing is a novel gene editing method. This method uses a fusion protein composed of spCas9 (nCas9) with H840A mutation and MLV-RT (Murine Leukemia Virus-Reverse Transcriptase), and a PegRNA (Prime editing guide RNA) modified from gRNA (guide RNA), thereby realizing any single base conversion/transversion or deletion, addition and substitution of small DNA fragments. The PegRNA is generated by introducing a PBS (Prime binding site) sequence and a template sequence at the 3' end of the gRNA, wherein the template sequence comprises an editing sequence and a homologous sequence of genomic DSB site. In this method, the complex formed by nCas9 and PegRNA binds to the genomic targeting site and cleaves the PAM strand, and then the PBS sequence on the PegRNA is paired with the free 3' end of the PAM strand, and then MLV-RT uses the template sequence of PegRNA as a template, the editing sequence and the homologous sequence are introduced by reverse transcription at the 3' end of the PAM strand nick. Subsequently, through processes such as DNA single-strand replacement and mismatch repair, the repair can be completed at the nick and the editing sequence can be integrated into the targeted site. Since nCas9 with H840A only cuts one strand of double-stranded DNA (that is, the PAM strand), it does not generate DSB to trigger NHEJ. Therefore, this method is not prone to introduce additional base deletions or insertions, and the editing accuracy is high. However, since the length of the template sequence on PegRNA limits the length of editable sequence, Prime Editing is only suitable for deletion or knock-in of nucleotide sequences less than 100 bp.

Therefore, establishment of a method that can efficiently perform site-directed knock-in and replacement of a gene, especially a method that can efficiently perform insertion and replacement of exogenous genes of large fragments (greater than 1Kbp), is critically important for expanding the application of gene editing technology in production and medicine.

### Contents of the present invention

In the present invention, unless otherwise specified, the scientific and technical terms used herein have the meanings commonly understood by those skilled in the art. In addition, the nucleic acid chemistry laboratory operation steps used herein are all routine steps widely used in the corresponding fields. Meanwhile, for a better understanding of the present invention, definitions and explanations of related terms are provided below.

The term "Cas protein" or "Cas nuclease" is an RNA-guided nuclease. Cas protein is also known as casn1 nuclease or CRISPR-associated nuclease. CRISPR (Clustered Regularly Interspaced Short Palindromic Repeat) is an adaptive immune system that provides protection against mobile genetic elements (viruses, transposable elements and conjugative plasmids). CRISPR clusters comprise repeats and spacers, in which spacers are sequences complementary to mobile genetic elements, which can target invasive nucleic acids. CRISPR clusters are transcribed and processed into CRISPR RNA (crRNA). In type II CRISPR systems, the correct processing of pre-crRNA also requires the participation of a trans-encoded small RNA (tracrRNA). Thus, in nature, the cleavage of DNA by the type II CRISPR system requires a Cas protein and two RNAs. However, crRNA and tracrRNA can be incorporated into a single guide RNA ("sgRNA" or "gRNA" for short) by engineering. See, for example, Jinek M., Chylinski K., Fonfara I., Hauer M., Doudna J. A., Charpentier E. Science 337: 816-821 (2012), the entire contents of which are incorporated herein by reference.

As used herein, the term "complementary" means that two nucleic acid sequences are capable of forming hydrogen bonds with each other according to the principles of base pairing (Waston-Crick principle), and thereby forming duplexes. In the present application, the term "complementary" includes "substantially complementary" and "completely complementary". As used herein, the term "completely complementary" means that every base in one nucleic acid sequence is capable of pairing with bases in another nucleic acid strand without mismatch or gap. As used herein, the term "substantially complementary" means that a majority of bases in one nucleic acid sequence are capable of pairing with bases in the other nucleic acid strand, which allows for mismatches or gaps (e.g., mismatches or gaps of one or several nucleotides). Typically, two nucleic acid sequences that are "complementary" (e.g., substantially complementary or fully complementary) will selectively/specifically hybridize or anneal under conditions that allow nucleic acid hybridization, annealing, or amplification, and form a duplex.

As used herein, the term "DNA polymerase" refers to an enzyme capable of using one nucleic acid strand (e.g., a DNA strand or RNA strand) as a template to synthesize another nucleic acid strand (e.g., a DNA strand). In the present application, a DNA polymerase may be a DNA-dependent DNA polymerase (i.e., an enzyme capable of synthesizing a complementary DNA strand using a DNA strand as a template), or an RNA-dependent DNA polymerase (i.e., an enzyme capable of synthesizing a complementary DNA strand using an RNA strand as a template). In certain embodiments, the DNA polymerase used herein is an RNA-dependent DNA polymerase, such as reverse transcriptase.

As used herein, the term "reverse transcriptase (RT)" refers to an enzyme capable of synthesizing a complementary DNA strand using an RNA strand as a template. The reverse transcriptases in the present application include, but are not limited to, reverse transcriptases from retroviruses or other viruses or bacteria, and DNA polymerases with reverse transcription activity, such as TTH DNA polymerase, Taq DNA polymerase, TNE DNA polymerase, TMA DNA polymerase, etc. The reverse transcriptases from retroviruses include, but are not limited to, reverse transcriptases from Moloney murine leukemia virus (M-MLV), human immunodeficiency virus (HIV), avian sarcoma-leukemia virus (ASLV), Rous sarcoma virus (RSV), avian myeloblastosis virus (AMV), avian erythroblastosis virus helper virus, avian granulocytosis virus MC29 helper virus, avian reticuloendotheliosis virus helper virus, avian sarcoma virus UR2 helper virus, avian sarcoma virus Y73 helper virus, Rous-associated virus and myeloblastosis-associated virus (MAV). Specific examples of reverse transcriptases can also be found in, for example, US Patent Application 2002/0198944 (hereby incorporated by reference in its entirety). In addition, the reverse transcriptases of the present application include, but are not limited to any form, for example, a naturally occurring reverse transcriptase, a naturally occurring mutant reverse transcriptase, an engineered mutant reverse transcriptase, or other variants (e.g., truncated variants retaining reverse transcription activity thereof).

As used herein, the terms "hybridization" and "annealing" refers to a process by which complementary single-stranded nucleic acid molecules form a double-stranded nucleic acid. In the present application, "hybridization" and "annealing" have the same meaning and are used interchangeably. Typically, two nucleic acid sequences that are completely complementary or substantially complementary can hybridize or anneal. The complementarity required for hybridization or annealing of two nucleic acid sequences depends on the hybridization conditions used, in particular the temperature.

As used herein, "conditions that allow nucleic acid hybridization" have the meaning commonly understood by those skilled in the art, and can be determined by conventional methods. For example, two nucleic acid molecules having complementary sequences can hybridize under suitable hybridization conditions. Such hybridization conditions may involve factors such as temperature, pH, composition and ionic strength of the hybridization buffer, etc., and may be determined based on the length and GC content of the two nucleic acid molecules that are complementary. For example, low stringency hybridization conditions can be used when the lengths of the two complementary nucleic acid molecules are relatively short and/or the GC content is relatively low. High stringency hybridization conditions can be used when the lengths of the two complementary nucleic acid molecules are relatively long and/or the GC content is relatively high. Such hybridization conditions are well known to those skilled in the art, and can be found in, for example, Joseph Sambrook, et al., Molecular Cloning, A Laboratory Manual, Cold Spring Harbor Laboratory Press, Cold Spring Harbor, N.Y. (2001); and M.L.M. Anderson, Nucleic Acid Hybridization, Springer-Verlag New York Inc. N.Y. (1999). In the present application, "hybridization" and "annealing" have the same meaning and are used interchangeably. Accordingly, the expressions "conditions allowing nucleic acid hybridization" and "conditions allowing nucleic acid annealing" also have the same meaning and are used interchangeably.

As used herein, the term "upstream" is used to describe the relative positional relationship of two nucleic acid sequences (or two nucleic acid molecules) and has the meaning commonly understood by those skilled in the art. For example, the expression "a nucleic acid sequence is located upstream of another nucleic acid sequence" means that when arranged in a 5' to 3' direction, the former is located more forward (i.e., closer to the 5' end) as compared with the latter. As used herein, the term "downstream" has the opposite meaning to "upstream."

As used herein, the term "linker" refers to a chemical entity used to join two physical elements (e.g., two nucleic acids or two polypeptides). For example, a linker used to join two polypeptides can be a peptide linker (e.g., a linker comprising multiple amino acid residues); a linker used to join two nucleic acids can be a nucleic acid linker (e.g., a linker comprising multiple nucleotides).

As used herein, the term "guide sequence" is a targeting sequence comprised by a guide RNA. In certain instances, a guide sequence is a polynucleotide sequence that is sufficiently complementary to a target sequence, and thus capable of hybridizing to the target sequence and guiding the CRISPR/Cas complex to specifically bind to the target sequence. In certain embodiments, the degree of complementarity between the guide sequence and its corresponding target sequence is at least 50%, at least 60%, at least 70%, at least 80%, at least 90%, at least 95%, or at least 99%. Methods for determining the complementarity of two nucleic acid sequences are within the purview of those skilled in the art. For example, there are published and commercially available alignment algorithms and programs such as, but not limited to, ClustalW, Smith-Waterman in matlab, Bowtie, Geneious, Biopython, and SeqMan.

As used herein, the term "scaffold sequence" refers to a sequence comprised by a guide RNA that is recognized by and bound to a Cas protein. In some cases, the scaffold sequence may comprise or consist of repeats of CRISPR.

As used herein, the term "functional complex" refers to a complex formed by binding a guide RNA (or gRNA) to a Cas protein, which is capable of recognizing and cleaving a polynucleotide complementary to the guide RNA.

As used herein, the term "target nucleic acid" or "target sequence" is a polynucleotide that is targeted by a guide sequence, for example, a sequence that is complementary to the guide sequence. Complete complementarity of the guide sequence to the target sequence is not required, so long as there is sufficient complementarity to cause their hybridization and facilitate the binding of CRISPR/Cas complex. The target sequence can comprise any polynucleotide, such as DNA or RNA. In certain instances, the target sequence is located at the nucleus or cytoplasm of the cell. In certain instances, the target sequence may be located at an organelle such as the mitochondria or chloroplast of the eukaryotic cell.

In the present invention, the expression "target sequence" or "target nucleic acid" can be any endogenous or exogenous polynucleotide with respect to a cell (e.g., an eukaryotic cell). For example, the target nucleic acid can be a polynucleotide (e.g., genomic DNA) present in the nucleus of an eukaryotic cell, or a polynucleotide (e.g., vector DNA) exogenously introduced in the cell. For example, a target nucleic acid can be a sequence encoding a gene product (e.g., a protein) or a non-coding sequence (e.g., a regulatory polynucleotide or unwanted DNA). In certain instances, the target nucleic acid or target sequence comprises or is adjacent to a protospacer adjacent motif (PAM). The exact sequence and length requirements for the PAM depend on the Cas protein used. Typically, a PAM is a 2-5 base pair sequence adjacent to the protospacer sequence in a CRISPR cluster. One of skill in the art is able to identify PAM sequences for use with a given Cas protein.

As used herein, the term "vector" refers to a nucleic acid delivery vehicle into which a polynucleotide can be inserted. When the vector can express the protein encoded by the inserted polynucleotide, the vector is called an expression vector. The vector can be introduced into a host cell by transformation, transduction or transfection, so that the genetic material elements carried thereby can be expressed in the host cell. Vectors are well known to those skilled in the art and include, but are not limited to: plasmid; phagemid; cosmid; nanoliposome particle; exosome; artificial chromosome such as yeast artificial chromosome (YAC), bacterial artificial chromosome (BAC) or P1-derived artificial chromosome (PAC); phage such as λ phage or M13 phage and animal virus. Animal viruses that can be used as vectors include, but are not limited to, retrovirus (including lentivirus), adenovirus, adeno-associated virus, herpesvirus (e.g., herpes simplex virus), poxvirus, baculovirus, papillomavirus, papovavirus (e.g. SV40). A vector may comprise a variety of elements that control expression, including, but not limited to, promoter sequence, transcription initiation sequence, enhancer sequence, selection element, and reporter gene. In addition, the vector may also comprise an origin of replication site. Those skilled in the art will appreciate that the design of expression vector may depend on factors such as the choice of host cell to be transformed, the desired level of expression, and the like. When the vector carries the exogenous DNA to be integrated into the host genome and the non-protein expression elements related to the integration of the exogenous DNA, the vector is called a donor vector. Exogenous DNA includes, but is not limited to, complete gene or gene fragment, promoter sequence, transcription initiation sequence, enhancer sequence, selection element, and protein-coding sequence. Non-protein expression elements related to the integration of exogenous DNA include, but are not limited to, homologous sequences of the proposed insertion site, cleavage sequences targeted by tool enzymes, and the like. Adeno-associated virus vectors include but are not limited to AAV1, AAV2, AAV3, AAV4, AAV5, AAV6, AAV7, AAV8, AAV9, AAV-DJ and other adeno-associated viruses of different serotypes and adeno-associated viruses of other modified serotypes.

In the present invention, the "intein" refers to a type of internal protein element that can mediate the splicing of the translated protein. The intein is located within the polypeptide sequence, which is excised after processing, and catalyzes the connection of the protein exteins at both ends into a mature protein molecule. The "intein splitting system" is a system for efficient splitting and splicing of larger protein molecules using inteins. Inteins can be split into N-terminal and C-terminal fragments. The target protein is split into two parts, the N-terminal fragment and the C-terminal fragment, which are respectively connected with the N-terminal fragment and the C-terminal fragment of the intein to form fusion proteins. Only when the N-terminal part and the C-terminal part of the two fusion proteins meet, the intein in the split precursor protein is removed by protein splicing, and the N-terminal fragment and C-terminal fragment of the target protein are spliced to form a functional protein of the interest. Inteins suitable for use in the present invention are derived from, but not limited to, DnaE DNA polymerases from Synechocystis sp. PCC6803 and Nostoc punctiforme PCC73102 (Npu).

As used herein, the term "host cell" refers to a cell that can be used to introduce a vector, including, but not limited to, prokaryotic cell such as E. *coli* or *Bacillus subtilis,* fungal cell such as yeast cell or *Aspergillus,* etc., insect cell such as *S2 Drosophila* cell or Sf9, or animal cell such as fibroblast, CHO cell, COS cell, NSO cell, HeLa cell, BHK cell, HEK 293 cell or human cell.

In a first aspect, the present application provides a system or kit, which comprises the following four components:
(1) a first Cas protein or a nucleic acid molecule A1 comprising a nucleotide sequence encoding the first Cas protein, wherein the first Cas protein is capable of cleaving or fragmenting a first double-stranded target nucleic acid;
(2) a first DNA polymerase that is template-dependent or a nucleic acid molecule B1 comprising a nucleotide sequence encoding the first DNA polymerase;
(3) a first gRNA or a nucleic acid molecule C1 comprising a nucleotide sequence encoding the first gRNA, wherein the first gRNA can bind to the first Cas protein and form a first functional complex; the first functional complex can fragment the two strands of the first double-stranded target nucleic acid to form target nucleic acid fragments;
(4) a first tag primer or a nucleic acid molecule D1 comprising a nucleotide sequence encoding the first tag primer, wherein the first tag primer comprises a first tag sequence and a first target-binding sequence, the first tag sequence is located at the upstream or 5' end of the first target-binding sequence; and, under conditions that allow hybridization or annealing of nucleic acids, the first target-binding sequence is capable of being hybridized or annealed to 3' end of one nucleic acid chain of the target nucleic acid fragments to form a double-stranded structure, and the first tag sequence does not bind to the target nucleic acid fragment and is in a free single-stranded state.

In certain embodiments, the first Cas protein is selected from, but not limited to, Cas9 protein, Cas12a protein, cas12b protein, cas12c protein, cas12d protein, cas12e protein, cas12f protein, cas12g protein, cas12h protein, cas12i protein, cas14 protein, Cas13a protein, Cas1 protein, Cas1B protein, Cas2 protein, Cas3 protein, Cas4 protein, Cas5 protein, Cas6 protein, Cas7 protein, Cas8 protein, Cas10 protein, Csy1 protein, Csy2 protein, Csy3 protein, Cse1 protein, Cse2 protein, Csc1 protein, Csc2 protein, Csa5 protein, Csn2 protein, Csm2 protein, Csm3 protein, Csm4 protein, Csm5 protein, Csm6 protein, Cmr1 protein, Cmr3 protein, Cmr4 protein, Cmr5 protein, Cmr6 protein, Csb1 protein, Csb2 protein, Csb3 protein, Csx17 protein, Csx14 protein, Csx10 protein, Csx16 protein, CsaX protein, Csx3 protein, Csx1 protein, Csx15 protein, Csf1 protein, Csf2 protein, Csf3 protein, Csf4 protein and homologs or modified forms thereof.

In certain embodiments, the first Cas protein is capable of cleaving the first double-stranded target nucleic acid and producing a sticky end or blunt end.

In certain embodiments, the first Cas protein is a Cas9 protein, such as a Cas9 protein of *Streptococcus pyogenes* (*S*. *pyogenes*) (spCas9).

In certain embodiments, the first Cas protein has the amino acid sequence set forth in SEQ ID NO: 1.

The sequences and structures of various Cas proteins are well known to those skilled in the art. Currently, various Cas9 proteins and their homologues have been reported in various species, including but not limited to *Streptococcus pyogenes* and *Streptococcus thermophilus.* Based on the present disclosure, other suitable Cas9 proteins will be apparent to those skilled in the art, for example, those Cas9 proteins disclosed in Chylinski, Rhun, and Charpentier, The tracrRNA and Cas9 families of type II CRISPR-Cas immunity systems. (2013) RNA Biology 10:5, 726-737, which is hereby incorporated by reference in its entirety.

In some embodiments, the Cas9 protein is a Cas9 protein from the following species: *Corynebacterium ulcerans* (NCBI Refs: NC_015683.1, NC_017317.1); *Corynebacterium diphtheriae* (NCBI Refs: NC_016782.1, NC_016786.1); *Spiroplasma syrphidicola* (Ref: NC_021284.1); *Prevotella intermedia* (NCBI Ref: NC_017861.1); *Spiroplasma taiwanense* (NCBI Ref: NC_021846.1); *Streptococcus iniae* (NCBI Ref: NC_021314.1); *Belliella baltica* (NCBI Ref: NC_018010.1); *Psychrof lexus torq uisl* (NCBI Ref: NC_018721.1); *Streptococcus thermophilus* (NCBI Ref:̅ YP_820832.1); *Listeria innocuous* (NCBI Ref: NP_472073.1); *Streptococcus pyogenes* (NCBI Ref: NC_017053.1).

In certain embodiments, the first DNA polymerase is selected from, but is not limited to, DNA-dependent DNA polymerase and RNA-dependent DNA polymerase.

In certain embodiments, the first DNA polymerase is an RNA-dependent DNA polymerase.

In certain embodiments, the first DNA polymerase is a reverse transcriptase, such as the reverse transcriptase listed above, for example, the reverse transcriptase of Moloney murine leukemia virus.

In certain embodiments, the first DNA polymerase has the amino acid sequence set forth in SEQ ID NO:4.

In certain embodiments, the first Cas protein is linked to the first DNA polymerase.

In certain embodiments, the first Cas protein is covalently linked to the first DNA polymerase with or without a linker.

In certain embodiments, the linker is a peptide linker, for example, a flexible peptide linker; for example, the linker has the amino acid sequence set forth in SEQ ID NO:51.

In certain embodiments, the first Cas protein is fused to the first DNA polymerase with or without a peptide linker to form a first fusion protein.

In certain embodiments, the first Cas protein is linked or fused to the N-terminal of the first DNA polymerase optionally through a linker; or, the first Cas protein is linked or fused to the C-terminal of the first DNA polymerase optionally through a linker.

In certain embodiments, the first fusion protein has the amino acid sequence set forth in SEQ ID NO:52.

In some embodiments, the linker is a peptide linker. In some embodiments, the peptide linker has a length of 5-200 amino acids, for example, 5, 6, 7, 8, 9, 10, 15, 20, 25, 30, 30-40, 40-50, 50- 60, 60-70, 70-80, 80-90, 90-100, 100-150 or 150-200 amino acids.

In certain embodiments, the first fusion protein or the first cas protein can be split into two parts by an intein splitting system. It is easy to understand that the intein splitting system can split the first fusion protein or the first cas protein at any amino acid position. For example, in certain embodiments, the intein splitting system performs splitting within the first cas protein. Thus, in certain embodiments, the first cas protein is split into an N-terminal fragment and a C-terminal fragment. For example, the N-terminal fragment and the C-terminal fragment of the first cas protein can be fused to the N-terminal fragment and the C-terminal fragment of the intein, respectively (or to the C-terminal fragment and the N-terminal fragment of the intein, respectively), and the two can be reconstituted into the active first cas protein in a cell. In certain embodiments, the N-terminal fragment and the C-terminal fragment of the first cas protein are each inactive in isolation state, but are capable of being reconstituted into the active first cas protein in a cell. Accordingly, in certain embodiments, the nucleic acid molecule A1 can be split into two parts, which comprise nucleotide sequences encoding the N-terminal fragment and the C-terminal fragment of the first cas protein, respectively. In addition, it is easy to understand that in the first fusion protein, the first DNA polymerase can be fused to the N-terminal fragment or the C-terminal fragment of the first cas protein. In certain embodiments, the first DNA polymerase is fused to the C-terminal fragment of the first cas protein.

In certain embodiments, the first gRNA comprises a first guide sequence, and, under conditions that allow hybridization or annealing of nucleic acids, the first guide sequence is capable of being hybridized or annealed to one nucleic acid strand of the first double-stranded target nucleic acid.

In certain embodiments, the first guide sequence has a length of at least 5nt, for example, 5-10nt, 10-15nt, 15-20nt, 20-25nt, 25-30nt, 30-40nt, 40-50nt, 50-100nt, 100-200nt, or longer.

In certain embodiments, the first gRNA further comprises a first scaffold sequence capable of being recognized by and bound to the first Cas protein to form a first functional complex.

In certain embodiments, the first scaffold sequence has a length of at least 20nt, such as 20-30nt, 30-40nt, 40-50nt, 50-100nt, 100-200nt, or longer.

In certain embodiments, the first guide sequence is located at the upstream or 5' end of the first scaffold sequence.

In certain embodiments, the first functional complex is capable of fragmenting two strands of the first double-stranded target nucleic acid after the first guide sequence binds to the first double-stranded target nucleic acid.

In certain embodiments, the first target-binding sequence is capable of being hybridized or annealed to the 3' end of one nucleic acid strand of the target nucleic acid fragment under conditions that allow hybridization or annealing of nucleic acids, and the 3' end is formed by fragmenting the first double-stranded target nucleic acid by the first functional complex.

In certain embodiments, the first target-binding sequence has a length of at least 5 nt, for example, 5-10 nt, 10-15 nt, 15-20 nt, 20-25 nt, 25-30 nt, 30-40 nt, 40-50 nt, 50-100nt, 100-200nt, or longer.

In certain embodiments, the first tag sequence has a length of at least 4nt, for example, 4-10nt, 10-15nt, 15-20nt, 20-25nt, 25-30nt, 30-40nt, 40-50nt, 50- 100nt, 100-200nt, or longer.

In certain embodiments, after the first target-binding sequence is hybridized or annealed to the 3' end of one nucleic acid strand of the target nucleic acid fragment, the first DNA polymerase is capable of extending the 3' end of the nucleic acid strand by using the first tag primer as a template. In certain embodiments, the extension forms a first overhang.

In certain embodiments, the first tag primer is a single-stranded deoxyribonucleic acid or a single-stranded ribonucleic acid.

In certain embodiments, the first tag primer is a single-stranded ribonucleic acid, and the first DNA polymerase is an RNA-dependent DNA polymerase; or, the first tag primer is a single-stranded deoxyribonucleic acid, and the first DNA polymerase is a DNA-dependent DNA polymerase.

In certain embodiments, the nucleic acid strand bound to the first guide sequence is different from the nucleic acid strand bound to the first target-binding sequence. In certain embodiments, the nucleic acid strand bound to the first guide sequence is an opposite strand of the nucleic acid strand bound to the first target-binding sequence.

In certain embodiments, the first tag primer is linked to the first gRNA.

In certain embodiments, the first tag primer is covalently linked to the first gRNA with or without a linker.

In certain embodiments, the first tag primer is linked to the 3' end of the first gRNA, optionally through a linker.

In certain embodiments, the linker is a nucleic acid linker (e.g., a ribonucleic acid linker or a deoxyribonucleic acid linker).

In certain embodiments, the first tag primer is a single-stranded ribonucleic acid, and it is linked to the 3' end of the first gRNA through a ribonucleic acid linker or not through a ribonucleic acid linker to form a first PegRNA.

In certain embodiments, the nucleic acid molecule A1 is capable of expressing the first Cas protein in a cell. In certain embodiments, the nucleic acid molecule B1 is capable of expressing the first DNA polymerase in a cell. In certain embodiments, the nucleic acid molecule C1 is capable of transcribing the first gRNA in a cell. In certain embodiments, the nucleic acid molecule D1 is capable of transcribing the first tag primer in a cell.

In certain embodiments, the nucleic acid molecule A1 is comprised in an expression vector (e.g., an eukaryotic expression vector), or, the nucleic acid molecule A1 is an expression vector (e.g., an eukaryotic expression vector) comprising a nucleotide sequence encoding the first Cas protein.

In certain embodiments, the nucleic acid molecule B1 is comprised in an expression vector (e.g., an eukaryotic expression vector), or, the nucleic acid molecule B 1 is an expression vector (e.g., an eukaryotic expression vector) comprising a nucleotide sequence encoding the first DNA polymerase.

In certain embodiments, the nucleic acid molecule C1 is comprised in an expression vector (e.g., an eukaryotic expression vector), or, the nucleic acid molecule C1 is an expression vector (e.g., an eukaryotic expression vector) comprising a nucleotide sequence encoding the first gRNA.

In certain embodiments, the nucleic acid molecule D1 is comprised in an expression vector (e.g., an eukaryotic expression vector), or, the nucleic acid molecule D1 is an expression vector (e.g., an eukaryotic expression vector) comprising a nucleotide sequence encoding the first tag primer.

In certain embodiments, the nucleic acid molecule A1 and the nucleic acid molecule B1 are comprised in the same or different expression vectors (e.g., eukaryotic expression vectors). In certain embodiments, the nucleic acid molecule A1 and the nucleic acid molecule B1 are capable of expressing the isolated first Cas protein and the first DNA polymerase in a cell, or capable of expressing a first fusion protein comprising the first Cas protein and the first DNA polymerase in a cell.

In certain embodiments, the nucleic acid molecule C1 and the nucleic acid molecule D1 are comprised in the same expression vector (e.g., an eukaryotic expression vector); in certain embodiments, the nucleic acid molecule C1 and the nucleic acid molecule D1 are capable of transcribing a first PegRNA comprising the first gRNA and the first tag primer in a cell.

In certain embodiments, two, three, or four of the nucleic acid molecules A1, B1, C1 and D1 are comprised in the same expression vector (e.g., an eukaryotic expression vector).

In certain embodiments, the system or kit comprises:
(M1-1) a first fusion protein comprising the first Cas protein and the first DNA polymerase, or, a nucleic acid molecule comprising a nucleotide sequence encoding the first fusion protein; or, (M1-2) the first Cas protein and the first DNA polymerase in isolation state, or, a nucleic acid molecule capable of expressing the first Cas protein and the first DNA polymerase in isolation state; and,
(M2) a first PegRNA comprising the first gRNA and the first tag primer, or a nucleic acid molecule comprising a nucleotide sequence encoding the first PegRNA.

In certain embodiments, the system or kit further comprises:
(5) a second gRNA or a nucleic acid molecule C2 comprising a nucleotide sequence encoding the second gRNA, wherein the second gRNA is capable of binding to a second Cas protein and form a second functional complex; the second functional complex is capable of fragmenting two strands of a second double-stranded target nucleic acid to form target nucleic acid fragments.

In certain embodiments, the second Cas protein is the same as or different from the first Cas protein. In certain embodiments, the second Cas protein is the same as the first Cas protein.

In certain embodiments, the second gRNA comprises a second guide sequence and, under conditions that allow hybridization or annealing of nucleic acids, the second guide sequence is capable of being hybridized or annealed to one nucleic acid strand of the second double-stranded target nucleic acid.

In certain embodiments, the second functional complex fragments two strands of the second double-stranded target nucleic acid upon binding of the second guide sequence to the second double-stranded target nucleic acid.

In certain embodiments, the second guide sequence is different from the first guide sequence.

In certain embodiments, the second double-stranded target nucleic acid is the same as or different from the first double-stranded target nucleic acid.

In certain embodiments, the second double-stranded target nucleic acid is the same as the first double-stranded target nucleic acid, and the second functional complex and the first functional complex fragment the same double-stranded target nucleic acid at different positions.

In certain embodiments, the second functional complex and the first functional complex fragment the same double-stranded target nucleic acid, and the nucleic acid strand bound to the first guide sequence is different from the nucleic acid strand bound to the second guide sequence; in certain embodiments, the nucleic acid strand bound to the first guide sequence is an opposite strand of the nucleic acid strand bound to the second guide sequence.

In certain embodiments, the second guide sequence has a length of at least 5nt, for example, 5-10nt, 10-15nt, 15-20nt, 20-25nt, 25-30nt, 30-40nt, 40-50nt, 50-100nt, 100-200nt, or longer.

In certain embodiments, the second gRNA further comprises a second scaffold sequence capable of being recognized by and bound to the second Cas protein to form a second functional complex.

In certain embodiments, the second scaffold sequence has a length of at least 20nt, for example, 20-30nt, 30-40nt, 40-50nt, 50-100nt, 100-200nt, or longer.

In certain embodiments, the second scaffold sequence is the same as or different from the first scaffold sequence; in certain embodiments, the second scaffold sequence is the same as the first scaffold sequence.

In certain embodiments, the second guide sequence is located at the upstream or 5' end of the second scaffold sequence.

In certain embodiments, the nucleic acid molecule C2 is capable of transcribing the second gRNA in a cell.

In certain embodiments, the nucleic acid molecule C2 is comprised in an expression vector (e.g., an eukaryotic expression vector), or, the nucleic acid molecule C2 is an expression vector (e.g. eukaryotic expression vector) comprising a nucleotide sequence encoding the second gRNA.

In certain embodiments, the second Cas protein is different from the first Cas protein; and, the system or kit further comprises:
(6) the second Cas protein or a nucleic acid molecule A2 comprising a nucleotide sequence encoding the second Cas protein, wherein the second Cas protein is capable of cleaving or fragmenting the second double-stranded target nucleic acid.

In certain embodiments, the second Cas protein is capable of fragmenting the second double-stranded target nucleic acid and producing a sticky end or blunt end.

In certain embodiments, the second Cas protein is selected from, but not limited to, Cas9 protein, Cas12a protein, cas12b protein, cas12c protein, cas12d protein, cas12e protein, cas12f protein, cas12g protein, cas12h protein, cas12i protein, cas14 protein, Cas13a protein, Cas1 protein, Cas1B protein, Cas2 protein, Cas3 protein, Cas4 protein, Cas5 protein, Cas6 protein, Cas7 protein, Cas8 protein, Cas10 protein, Csy1 protein, Csy2 protein, Csy3 protein, Cse1 protein, Cse2 protein, Csc1 protein, Csc2 protein, Csa5 protein, Csn2 protein, Csm2 protein, Csm3 protein, Csm4 protein, Csm5 protein, Csm6 protein, Cmr1 protein, Cmr3 protein, Cmr4 protein, Cmr5 protein, Cmr6 protein, Csb 1 protein, Csb2 protein, Csb3 protein, Csx17 protein, Csx14 protein, Csx10 protein, Csx16 protein, CsaX protein, Csx3 protein, Csx1 protein, Csx15 protein, Csf1 protein, Csf2 protein, Csf3 protein, Csf4 protein, and homologs or modified forms thereof.

In certain embodiments, the second Cas protein is a Cas9 protein, such as the Cas9 protein of *Streptococcus pyogenes* (*S. pyogenes*) (spCas9).

In certain embodiments, the second Cas protein has the amino acid sequence set forth in SEQ ID NO: 1.

In certain embodiments, the nucleic acid molecule A2 is capable of expressing the second Cas protein in a cell.

In certain embodiments, the nucleic acid molecule A2 is comprised in an expression vector (e.g., an eukaryotic expression vector), or, the nucleic acid molecule A2 is an expression vector (e.g., an eukaryotic expression vector) comprising a nucleotide sequence encoding the second Cas protein.

In certain embodiments, the system or kit further comprises:
(7) a second tag primer or a nucleic acid molecule D2 comprising a nucleotide sequence encoding the second tag primer, wherein the second tag primer comprises a second tag sequence and a second target-binding sequence, the second tag sequence is located at the upstream or 5' end of the second target-binding sequence; and, under conditions that allow hybridization or annealing of nucleic acids, the second target-binding sequence is capable of being hybridized or annealed to the 3' end of one nucleic acid strand of the target nucleic acid fragment to form a double-stranded structure, and, the second tag sequence does not bind to the target nucleic acid fragment, and is in a free single-stranded state.

In certain embodiments, the second target-binding sequence is capable of being hybridized or annealed to the 3' end of one nucleic acid strand of the target nucleic acid fragment under conditions that allow hybridization or annealing of nucleic acids, and the 3' end is formed by fragmenting the second double-stranded target nucleic acid by the second functional complex.

In certain embodiments, the second target-binding sequence has a length of at least 5nt, for example, 5-10nt, 10-15nt, 15-20nt, 20-25nt, 25-30nt, 30-40nt, 40-50nt, 50-100nt, 100-200nt, or longer.

In certain embodiments, the second target-binding sequence is different from the first target-binding sequence. In certain embodiments, the nucleic acid strand bound to the second target-binding sequence is different from the nucleic acid strand bound to the first target-binding sequence. In certain embodiments, the nucleic acid strand bound to the second target-binding sequence is an opposite strand of the nucleic acid strand bound to the first target-binding sequence.

In certain embodiments, the second tag sequence has a length of at least 4nt, for example, 4-10nt, 10-15nt, 15-20nt, 20-25nt, 25-30nt, 30-40nt, 40-50nt, 50-100nt, 100-200nt, or longer.

In certain embodiments, the second tag sequence is the same as or different from the first tag sequence. In certain embodiments, the second tag sequence is different from the first tag sequence.

In certain embodiments, after the second target-binding sequence is hybridized or annealed to the 3' end of one nucleic acid strand of the target nucleic acid fragment, a second DNA polymerase is capable of extending the 3' end of the nucleic acid strand by using the second tag primer as a template. In certain embodiments, the extension forms a second overhang.

In certain embodiments, the second DNA polymerase is the same as or different from the first DNA polymerase. In certain embodiments, the second DNA polymerase is the same as the first DNA polymerase.

In certain embodiments, the second tag primer is a single-stranded deoxyribonucleic acid or a single-stranded ribonucleic acid.

In certain embodiments, the second tag primer is a single-stranded ribonucleic acid, and the second DNA polymerase is an RNA-dependent DNA polymerase; or, the second tag primer is a single-stranded deoxyribonucleic acid, and the second DNA polymerase is a DNA-dependent DNA polymerase.

In certain embodiments, the nucleic acid strand bound to the second guide sequence is different from the nucleic acid strand bound to the second target-binding sequence. In certain embodiments, the nucleic acid strand bound to the second guide sequence is an opposite strand of the nucleic acid strand bound to the second target-binding sequence.

In certain embodiments, the second guide sequence and the first target-binding sequence bind to the same nucleic acid strand, and the binding position of the second guide sequence is located at the upstream or 5' end of the binding position of the first target-binding sequence.

In certain embodiments, the first guide sequence and the second target-binding sequence bind to the same nucleic acid strand, and the binding position of the first guide sequence is located at the upstream or 5' end of the binding position of the second target-binding sequence.

In certain embodiments, the first overhang and the second overhang are comprised in the same target nucleic acid fragment, and are located at nucleic acid strands opposite to each other.

In certain embodiments, the nucleic acid molecule D2 is capable of transcribing the second tag primer in a cell.

In certain embodiments, the nucleic acid molecule D2 is comprised in an expression vector (e.g., an eukaryotic expression vector), or, the nucleic acid molecule D2 is an expression vector (e.g., an eukaryotic expression vector) comprising a nucleotide sequence encoding the second tag primer.

In certain embodiments, the second DNA polymerase is different from the first DNA polymerase; and, the system or kit further comprises:
(8) the second DNA polymerase or a nucleic acid molecule B2 comprising a nucleotide sequence encoding the second DNA polymerase.

In certain embodiments, the second DNA polymerase is selected from, but is not limited to, DNA-dependent DNA polymerase and RNA-dependent DNA polymerase.

In certain embodiments, the second DNA polymerase is an RNA-dependent DNA polymerase.

In certain embodiments, the second DNA polymerase is a reverse transcriptase, such as the reverse transcriptase listed above, for example, the reverse transcriptase of Moloney murine leukemia virus.

In certain embodiments, the second DNA polymerase has the amino acid sequence set forth in SEQ ID NO:4.

In certain embodiments, the nucleic acid molecule B2 is capable of expressing the second DNA polymerase in a cell.

In certain embodiments, the nucleic acid molecule B2 is comprised in an expression vector (e.g., an eukaryotic expression vector), or, the nucleic acid molecule B2 is an expression vector (e.g., an eukaryotic expression vector) comprising a nucleotide sequence encoding the second DNA polymerase.

In certain embodiments, wherein the second tag primer is linked to the second gRNA.

In certain embodiments, the second tag primer is covalently linked to the second gRNA with or without a linker.

In certain embodiments, the second tag primer is linked to the 3' end of the second gRNA, optionally through a linker.

In certain embodiments, the linker is a nucleic acid linker (e.g., a ribonucleic acid linker or a deoxyribonucleic acid linker).

In certain embodiments, the second tag primer is a single-stranded ribonucleic acid, and is linked to the 3' end of the second gRNA through a ribonucleic acid linker or not through a ribonucleic acid linker to form a second PegRNA.

In certain embodiments, the nucleic acid molecule C2 and the nucleic acid molecule D2 are comprised in the same expression vector (e.g., an eukaryotic expression vector); in certain embodiments, in a cell, the nucleic acid molecule C2 and the nucleic acid molecule D2 are capable of transcribing a second PegRNA comprising the second gRNA and the second tag primer.

In certain embodiments, the system or kit comprises: a second PegRNA comprising the second gRNA and the second tag primer, or, a nucleic acid molecule comprising a nucleotide sequence encoding the second PegRNA.

In certain embodiments, the second Cas protein is isolated from or linked to the second DNA polymerase.

In certain embodiments, the second Cas protein is covalently linked to the second DNA polymerase with or without a linker.

In certain embodiments, the linker is a peptide linker, for example, a flexible peptide linker; for example, the linker has the amino acid sequence set forth in SEQ ID NO: 51.

In certain embodiments, the second Cas protein is fused to the second DNA polymerase with or without a peptide linker to form a second fusion protein.

In certain embodiments, the second Cas protein is linked or fused to the N-terminal of the second DNA polymerase optionally through a linker; or, the second Cas protein is linked or fused to the C-terminal of the second DNA polymerase optionally through a linker.

In certain embodiments, the second fusion protein has the amino acid sequence set forth in SEQ ID NO:52.

In certain embodiments, the second fusion protein or the second cas protein can be split into two parts by an intein splitting system. It is easy to understand that the intein splitting system can split the second fusion protein or the second cas protein at any amino acid position. For example, in certain embodiments, the intein splitting system performs splitting within the second cas protein. Thus, in certain embodiments, the second cas protein is split into an N-terminal fragment and a C-terminal fragment. For example, the N-terminal fragment and the C-terminal fragment of the second cas protein can be fused to the N-terminal fragment and the C-terminal fragment of the intein, respectively (or to the C-terminal fragment and the N-terminal fragment of the intein, respectively), and the two can be reconstituted to form the active second cas protein in a cell. In certain embodiments, the N-terminal fragment and the C-terminal fragment of the second cas protein each in isolation state is inactive, but is capable of being reconstituted to form the active second cas protein in a cell. Accordingly, in certain embodiments, the nucleic acid molecule A2 can be split into two parts, which comprise nucleotide sequences encoding the N-terminal fragment and the C-terminal fragment of the second cas protein, respectively. In addition, it is easy to understand that in the second fusion protein, the second DNA polymerase can be fused to the N-terminal fragment or the C-terminal fragment of the second cas protein. In certain embodiments, the second DNA polymerase is fused to the C-terminal fragment of the second cas protein.

In certain embodiments, the nucleic acid molecule A2 and the nucleic acid molecule B2 are comprised in the same or different expression vectors (e.g., eukaryotic expression vectors). In certain embodiments, the nucleic acid molecule A2 and the nucleic acid molecule B2 are capable of expressing the second Cas protein and the second DNA polymerase in isolation state in a cell, or are capable of expressing a second fusion protein comprising the second Cas protein and the second DNA polymerase in a cell.

In certain embodiments, the system or kit comprises a second fusion protein comprising the second Cas protein and the second DNA polymerase, or, comprises a nucleic acid molecule comprising a nucleotide sequence encoding the second fusion protein, or, the second Cas protein and the second DNA polymerase in isolation state, or, a nucleic acid molecule capable of expressing the second Cas protein and the second DNA polymerase in isolation state.

In certain embodiments, the first and second Cas proteins are the same Cas protein, the first and second DNA polymerases are the same DNA polymerase; and, the system or kit comprises:
(M1-1) a first fusion protein comprising the first Cas protein and the first DNA polymerase, or, a nucleic acid molecule comprising a nucleotide sequence encoding the first fusion protein; or, (M1-2) the first Cas protein and the first DNA polymerase in isolation state, or a nucleic acid molecule capable of expressing the first Cas protein and the first DNA polymerase in isolation state;
(M2) a first PegRNA comprising the first gRNA and the first tag primer, or a nucleic acid molecule comprising a nucleotide sequence encoding the first PegRNA;
(M3) a second PegRNA comprising the second gRNA and the second tag primer, or, a nucleic acid molecule comprising a nucleotide sequence encoding the second PegRNA.

In certain embodiments, the system or kit further comprises a nucleic acid vector.

In certain embodiments, the nucleic acid vector is double-stranded.

In certain embodiments, the nucleic acid vector is a circular double-stranded vector.

In certain embodiments, the nucleic acid vector comprises a first guide binding sequence (e.g., a complementary sequence to the first guide sequence) capable of being hybridized or annealed to the first guide sequence, and/or, a second guide binding sequence (e.g., a complementary sequence to the second guide sequence) capable of being hybridized or annealed to the second guide sequence. In certain embodiments, the nucleic acid vector further comprises a restriction enzyme cleavage site between the first guide binding sequence and the second guide binding sequence.

In certain embodiments, the first guide binding sequence and the second guide binding sequence are located at an opposite strand of the nucleic acid vector.

In certain embodiments, the nucleic acid vector further comprises a first PAM sequence recognized by the first Cas protein, and/or a second PAM sequence recognized by the second Cas protein.

In certain embodiments, the first functional complex is capable of binding and fragmenting the nucleic acid vector through the first guide binding sequence and the first PAM sequence; and/or, the second functional complex is capable of binding and fragmenting the nucleic acid vector through the second guide binding sequence and the second PAM sequence.

In certain embodiments, the nucleic acid vector further comprises a gene of interest.

In certain embodiments, the gene of interest is located between the first guide binding sequence and the second guide binding sequence.

In certain embodiments, the first functional complex and the second functional complex cleave the nucleic acid vector, resulting in the production of a nucleic acid fragment comprising the gene of interest.

In certain embodiments, under conditions that allow hybridization or annealing of nucleic acids, the first tag primer is capable of being hybridized or annealed to the 3' end of one nucleic acid strand of the nucleic acid fragment via the first target-binding sequence to form a double-stranded structure, and the first tag sequence of the first tag primer is in a free state; in certain embodiments, the nucleic acid strand to which the first target-binding sequence is hybridized or annealed is an opposite strand of a nucleic acid strand comprising the first guide binding sequence.

In certain embodiments, under conditions that allow hybridization or annealing of nucleic acids, the second tag primer is capable of being hybridized or annealed to the 3' end of one nucleic acid strand of the nucleic acid fragment via the second target-binding sequence to form a double-stranded structure, and the second tag sequence of the second tag primer is in a free state; in certain embodiments, the nucleic acid strand to which the second target-binding sequence is hybridized or annealed is an opposite strand of a nucleic acid strand comprising the second guide binding sequence.

In certain embodiments, the nucleic acid strand to which the first target-binding sequence is hybridized or annealed is an opposite strand of the nucleic acid strand to which the second target-binding sequence is hybridized or annealed.

In certain embodiments, the nucleic acid vector further comprises a first target sequence; wherein, under conditions that allow hybridization or annealing of nucleic acids, the first tag primer is capable of being hybridized or annealed to the first target sequence through the first target-binding sequence to form a double-stranded structure, and the first tag sequence of the first tag primer is in a free state. In certain embodiments, the first target sequence is located between the first guide binding sequence and the second guide binding sequence. In certain embodiments, the first target sequence is located on an opposite strand of the first guide binding sequence. In certain embodiments, after the nucleic acid vector is cleaved by the first functional complex, the nucleic acid strand comprising the first target sequence can be extended by using the first tag primer annealed to the first target sequence as a template (in certain embodiments, to form a first overhang). In certain embodiments, the first functional complex cleaves the nucleic acid vector at a site located at the 3' end or 3' portion of the first target sequence. In certain embodiments, the first target sequence is located at the 3' end of one nucleic acid strand of the nucleic acid fragment comprising the gene of interest;
and/or,
the nucleic acid vector further comprises a second target sequence; wherein, under conditions that allow hybridization or annealing of nucleic acids, the second tag primer can hybridize or anneal to the second target sequence through the second target-binding sequence to form a double-stranded structure, and the second tag sequence of the second tag primer is in a free state; in certain embodiments, the second target sequence is located between the first guide binding sequence and the second guide binding sequence. In certain embodiments, the second target sequence is located at an opposite strand of the second guide binding sequence. In certain embodiments, after cleavage of the nucleic acid vector by the second functional complex, the nucleic acid strand comprising the second target sequence can be extended using the second tag primer annealed to the second target sequence as a template (in certain embodiments, to form a second overhang). In certain embodiments, the second functional complex cleaves the nucleic acid vector at a site located at the 3' end or 3' portion of the second target sequence; in certain embodiments, the second target sequence is located at the 3' end of one nucleic acid strand of the nucleic acid fragment comprising the gene of interest.

In certain embodiments, the nucleic acid strand comprising the first target sequence is located at an opposite strand of the nucleic acid strand comprising the second target sequence.

In certain embodiments, the nucleic acid vector further comprises a restriction enzyme cleavage site between the first target sequence and the second target sequence.

In certain embodiments, the nucleic acid vector further comprises a gene of interest between the first target sequence and the second target sequence.

In certain embodiments, the system or kit further comprises:
(9) a third gRNA or a nucleic acid molecule C3 comprising a nucleotide sequence encoding the third gRNA, wherein the third gRNA is capable of binding to a third Cas protein to form a third functional complex; the third functional complex is capable of fragmenting two strands of the third double-stranded target nucleic acid to form fragmented nucleotide fragments a1 and a2.

In certain embodiments, the third Cas protein is the same as or different from the first Cas protein or the second Cas protein; in certain embodiments, the first, second and third Cas proteins are the same Cas protein.

In certain embodiments, the third gRNA comprises a third guide sequence, and, under conditions that allow hybridization or annealing of nucleic acids, the third guide sequence is capable of being hybridized or annealed to one nucleic acid strand of the third double-stranded target nucleic acid.

In certain embodiments, the third functional complex fragments two strands of the third double-stranded target nucleic acid after the third guide sequence binds to the third double-stranded target nucleic acid.

In certain embodiments, the third guide sequence is the same as or different from the first guide sequence or the second guide sequence. In certain embodiments, the first, second and third priming sequences are different from each other.

In certain embodiments, the third double-stranded target nucleic acid is the same as or different from the first double-stranded target nucleic acid or the second double-stranded target nucleic acid. In certain embodiments, the second double-stranded target nucleic acid is the same as the first double-stranded target nucleic acid, and the third double-stranded target nucleic acid is different from the first and second double-stranded target nucleic acids. In certain embodiments, the third double-stranded target nucleic acid is a genomic DNA.

In certain embodiments, the third guide sequence has a length of at least 5nt, for example, 5-10nt, 10-15nt, 15-20nt, 20-25nt, 25-30nt, 30-40nt, 40-50nt, 50-100nt, 100-200nt, or longer.

In certain embodiments, the third gRNA further comprises a third scaffold sequence capable of being recognized by and bound to the third Cas protein, thereby forming a third functional complex.

In certain embodiments, the third scaffold sequence has a length of at least 20nt, for example, 20-30nt, 30-40nt, 40-50nt, 50-100nt, 100-200nt, or longer.

In certain embodiments, the third scaffold sequence is the same as or different from the first scaffold sequence or the second scaffold sequence. In certain embodiments, the first, second and third scaffold sequences are the same.

In certain embodiments, the third guide sequence is located at the upstream or 5' end of the third scaffold sequence.

In certain embodiments, the nucleic acid molecule C3 is capable of transcribing the third gRNA in a cell.

In certain embodiments, the nucleic acid molecule C3 is comprised in an expression vector (e.g., an eukaryotic expression vector), or, the nucleic acid molecule C3 is an expression vector (e.g. an eukaryotic expression vector) comprising a nucleotide sequence encoding the third gRNA.

In certain embodiments, the third functional complex is capable of fragmenting two strands of the third double-stranded target nucleic acid to form nucleotide fragments a1 and a2.

In certain embodiments, under conditions that allow hybridization or annealing of nucleic acids, the first tag sequence or complementary sequence thereof or the first overhang is capable of being hybridized or annealed to the fragmented nucleotide fragment a1. In certain embodiments, the first tag sequence or complementary sequence thereof or the first overhang is capable of being hybridized or annealed to the nucleotide fragment a1 at the end formed by fragmenting the third double-stranded target nucleic acid by the third functional complex. In certain embodiments, the complementary sequence of the first tag sequence or the first overhang is capable of being hybridized or annealed to the 3' end or 3' portion of one nucleic acid strand of the fragmented nucleotide fragment a1, and the 3' end or 3' portion is formed by fragmenting the third double-stranded target nucleic acid by the third functional complex.

In certain embodiments, the complementary sequence of the first tag sequence or the first overhang is capable of being hybridized or annealed to the 3' portion of one nucleic acid strand of the nucleotide fragment a1, and there is a first spacer region between the 3' portion of the fragment a1 and the cleaved end formed by the third double-stranded target nucleic acid.

In certain embodiments, the first spacer region has a length of 1 nt to 200 nt, for example, 1-10 nt, 10-20 nt, 20-30 nt, 30-40 nt, 40-50 nt, 50-100 nt, or 100-200 nt.

In certain embodiments, under conditions that allow hybridization or annealing of nucleic acids, the second tag sequence or complementary sequence thereof or the second overhang is capable of being hybridized or annealed to the nucleotide fragment a2. In certain embodiments, the second tag sequence or complementary sequence thereof or the second overhang is capable of being hybridized or annealed to the nucleotide fragment a2 formed by fragmenting the third double-stranded target nucleic acid by the third functional complex. In certain embodiments, the complementary sequence of the second tag sequence or the second overhang is capable of being hybridized or annealed to the 3' end or 3' portion of one nucleic acid strand of the nucleotide fragment a2, and the 3' end or 3' portion is formed by fragmenting the third double-stranded target nucleic acid by the third functional complex.

In certain embodiments, the complementary sequence of the second tag sequence or the second overhang is capable of being hybridized or annealed to the 3' portion of one nucleic acid strand of the nucleotide fragment a2, and there is a second spacer region between the 3' portion of the nucleotide fragment a2 and the cleaved end formed by the third double-stranded target nucleic acid.

In certain embodiments, the second spacer region has a length of 1 nt to 200 nt, for example, 1-10 nt, 10-20 nt, 20-30 nt, 30-40 nt, 40-50 nt, 50-100 nt, or 100-200 nt.

In certain embodiments, the third Cas protein is different from the first Cas protein or the second Cas protein; and, the system or kit further comprises:
(10) the third Cas protein or a nucleic acid molecule A3 comprising a nucleotide sequence encoding the third Cas protein, wherein the third Cas protein is capable of cleaving or fragmenting the third double-stranded target nucleic acid.

In certain embodiments, the third Cas protein is selected from, but not limited to, Cas9 protein, Cas12a protein, cas12b protein, cas12c protein, cas12d protein, cas12e protein, cas12f protein, cas12g protein, cas12h protein, cas12i protein, cas14 protein, Cas13a protein, Cas1 protein, Cas1B protein, Cas2 protein, Cas3 protein, Cas4 protein, Cas5 protein, Cas6 protein, Cas7 protein, Cas8 protein, Cas10 protein, Csy1 protein, Csy2 protein, Csy3 protein, Cse1 protein, Cse2 protein, Csc1 protein, Csc2 protein, Csa5 protein, Csn2 protein, Csm2 protein, Csm3 protein, Csm4 protein, Csm5 protein, Csm6 protein, Cmr1 protein, Cmr3 protein, Cmr4 protein, Cmr5 protein, Cmr6 protein, Csb1 protein, Csb2 protein, Csb3 protein, Csx17 protein, Csx14 protein, Csx10 protein, Csx16 protein, CsaX protein, Csx3 protein, Csx1 protein, Csx15 protein, Csf1 protein, Csf2 protein, Csf3 protein, Csf4 protein and homologs or modified forms thereof.

In certain embodiments, the third Cas protein is capable of fragmenting the third double-stranded target nucleic acid and producing a sticky end or blunt end.

In certain embodiments, the third Cas protein is a Cas9 protein, such as a Cas9 protein (spCas9) of *Streptococcus pyogenes (S. pyogenes).*

In certain embodiments, the third Cas protein has the amino acid sequence set forth in SEQ ID NO: 1.

In certain embodiments, the nucleic acid molecule A3 is capable of expressing the third Cas protein in a cell.

In certain embodiments, the nucleic acid molecule A3 is comprised in an expression vector (e.g., an eukaryotic expression vector), or, the nucleic acid molecule A3 is an expression vector (e.g., an eukaryotic expression vector) comprising a nucleotide sequence encoding the third Cas protein.

In certain embodiments, the first, second and third Cas proteins are the same Cas protein, the first and second DNA polymerases are the same DNA polymerase; and, the system or kit comprises:
(M1-1) a first fusion protein comprising the first Cas protein and the first DNA polymerase, or, a nucleic acid molecule comprising a nucleotide sequence encoding the first fusion protein; or, (M1-2) the first Cas protein and the first DNA polymerase in isolation state, or a nucleic acid molecule capable of expressing the first Cas protein and the first DNA polymerase in isolation state;
(M2) a first PegRNA comprising the first gRNA and the first tag primer, or a nucleic acid molecule comprising a nucleotide sequence encoding the first PegRNA;
(M3) a second PegRNA comprising the second gRNA and the second tag primer, or a nucleic acid molecule comprising a nucleotide sequence encoding the second PegRNA;
(M4) the third gRNA or a nucleic acid molecule comprising a nucleotide sequence encoding the third gRNA.

In certain embodiments, the system or kit further comprises: a nucleic acid vector as defined above.

In certain embodiments, the system or kit further comprises:
(11) a third tag primer or a nucleic acid molecule D3 comprising a nucleotide sequence encoding the third tag primer, wherein the third tag primer comprises a third tag sequence and a third target-binding sequence, the third tag sequence is located at the upstream or 5' end of the third target-binding sequence; and, under conditions that allow hybridization or annealing of nucleic acids, the third target-binding sequence is capable of being hybridized or annealed to the 3' end of one nucleic acid strand of the nucleotide fragment a1 or a2 to form a double-stranded structure, and the third tag sequence does not bind to the nucleotide fragment a1 or a2, and is in a free single-stranded state.

In certain embodiments, under conditions that allow hybridization or annealing of nucleic acids, the third target-binding sequence is capable of being hybridized or annealed to the 3' end of one nucleic acid strand of the nucleotide fragment a1 or a2, and the 3' end is formed by fragmenting the third double-stranded target nucleic acid by the third functional complex.

In certain embodiments, the nucleic acid strand bound to the third target-binding sequence is different from the nucleic acid strand bound to the third guide sequence; in certain embodiments, the nucleic acid strand bound to the third target-binding sequence is an opposite strand of the nucleic acid strand bound to the third guide sequence.

In certain embodiments, the third target-binding sequence has a length of at least 5nt, for example, 5-10nt, 10-15nt, 15-20nt, 20-25nt, 25-30nt, 30-40nt, 40-50nt, 50-100nt, 100-200nt, or longer.

In certain embodiments, the third target-binding sequence is different from the first or second target-binding sequence.

In certain embodiments, after the third target-binding sequence is hybridized or annealed to the 3' end of one nucleic acid strand of the nucleotide fragment a1 or a2, the third DNA polymerase is capable of extending the 3' end of the nucleic acid strand by using the third tag primer as a template; in certain embodiments, the extension forms a third overhang.

In certain embodiments, the third tag sequence has a length of at least 4nt, for example, 4-10nt, 10-15nt, 15-20nt, 20-25nt, 25-30nt, 30-40nt, 40-50nt, 50-100nt, 100-200nt, or longer.

In certain embodiments, the third tag sequence is the same as or different from the first or second tag sequence. In certain embodiments, the third tag sequence is different from the first or second tag sequence.

In certain embodiments, under conditions that allow hybridization or annealing of nucleic acids, the complementary sequence of the third tag sequence or the third overhang is capable of being hybridized or annealed to a nucleic acid strand comprising the first overhang or the second overhang; in certain embodiments, the complementary sequence of the third tag sequence or the third overhang is hybridized or annealed to the first overhang or the second overhang or a nucleotide sequence at the upstream thereof.

In certain embodiments, the third DNA polymerase is the same as or different from the first or second DNA polymerase; in certain embodiments, the first, second and third DNA polymerases are the same DNA polymerase.

In certain embodiments, the third tag primer is a single-stranded deoxyribonucleic acid or a single-stranded ribonucleic acid.

In certain embodiments, the third tag primer is a single-stranded ribonucleic acid, and the third DNA polymerase is an RNA-dependent DNA polymerase; or, the third tag primer is a single-stranded deoxyribonucleic acid, and the third DNA polymerase is a DNA-dependent DNA polymerase.

In certain embodiments, the nucleic acid molecule D3 is capable of transcribing the third tag primer in a cell.

In certain embodiments, the nucleic acid molecule D3 is comprised in an expression vector (e.g., an eukaryotic expression vector), or, the nucleic acid molecule D3 is an expression vector (e.g., eukaryotic expression vector) comprising a nucleotide sequence encoding the third tag primer.

In certain embodiments, the third DNA polymerase is different from the first or second DNA polymerase; and, the system or kit further comprises:
(12) the third DNA polymerase or a nucleic acid molecule B3 comprising a nucleotide sequence encoding the third DNA polymerase.

In certain embodiments, the third DNA polymerase is selected from, but is not limited to, DNA-dependent DNA polymerase and RNA-dependent DNA polymerase.

In certain embodiments, the third DNA polymerase is an RNA-dependent DNA polymerase.

In certain embodiments, the third DNA polymerase is a reverse transcriptase, such as the reverse transcriptase listed above, for example, a reverse transcriptase of Moloney murine leukemia virus.

In certain embodiments, the third DNA polymerase has the amino acid sequence set forth in SEQ ID NO:4.

In certain embodiments, the nucleic acid molecule B3 is capable of expressing the third DNA polymerase in a cell.

In certain embodiments, the nucleic acid molecule B3 is comprised in an expression vector (e.g., an eukaryotic expression vector), or, the nucleic acid molecule B3 is an expression vector (e.g., an eukaryotic expression vector) comprising a nucleotide sequence encoding the third DNA polymerase.

In certain embodiments, the third tag primer is linked to the third gRNA.

In certain embodiments, the third tag primer is covalently linked to the third gRNA with or without a linker.

In certain embodiments, the third tag primer is linked to the 3' end of the third gRNA, optionally through a linker.

In certain embodiments, the linker is a nucleic acid linker (e.g., a ribonucleic acid linker or a deoxyribonucleic acid linker).

In certain embodiments, the third tag primer is a single-stranded ribonucleic acid, and it is linked to the 3' end of the third gRNA through a ribonucleic acid linker or not through a ribonucleic acid linker to form a third PegRNA.

In certain embodiments, the nucleic acid molecule C3 and the nucleic acid molecule D3 are comprised in the same expression vector (e.g., an eukaryotic expression vector). In certain embodiments, the nucleic acid molecule C3 and the nucleic acid molecule D3 are capable of transcribing the third PegRNA comprising the third gRNA and the third tag primer in a cell.

In certain embodiments, the system or kit comprises: a third PegRNA comprising the third gRNA and the third tag primer, or, a nucleic acid molecule comprising a nucleotide sequence encoding the third PegRNA.

In certain embodiments, the third Cas protein is isolated or linked to the third DNA polymerase.

In certain embodiments, the third Cas protein is covalently linked to the third DNA polymerase with or without a linker.

In certain embodiments, the linker is a peptide linker, for example, a flexible peptide linker; for example, the linker has the amino acid sequence set forth in SEQ ID NO:51.

In certain embodiments, the third Cas protein is fused to the third DNA polymerase with or without a peptide linker to form a third fusion protein.

In certain embodiments, the third Cas protein is linked or fused to the N-terminal of the third DNA polymerase optionally through a linker; or, the third Cas protein is linked or fused to the C-terminal of the third DNA polymerase optionally through a linker.

In certain embodiments, the third fusion protein has the amino acid sequence set forth in SEQ ID NO:52.

In certain embodiments, the third fusion protein or the third cas protein can be split into two parts by an intein splitting system. It is easy to understand that the intein splitting system can split the third fusion protein or the third cas protein at any amino acid position. For example, in certain embodiments, the intein splitting system performs splitting within the third cas protein. Thus, in certain embodiments, the third cas protein is split into an N-terminal fragment and a C-terminal fragment. For example, the N-terminal fragment and the C-terminal fragment of the third cas protein can be fused to the N-terminal fragment and the C-terminal fragment of the intein, respectively (or to the C-terminal fragment and the N-terminal fragment of the intein, respectively), and the two can be reconstituted to form the active third cas protein in a cell. In certain embodiments, the N-terminal fragment and the C-terminal fragment of the third cas protein are each inactive in isolation state, but are capable of being reconstituted to form the active third cas protein in a cell. Accordingly, in certain embodiments, the nucleic acid molecule A3 can be split into two parts, which comprise nucleotide sequences encoding the N-terminal fragment and the C-terminal fragment of the third cas protein, respectively. In addition, it is easy to understand that in the third fusion protein, the third DNA polymerase can be fused to the N-terminal fragment or the C-terminal fragment of the third cas protein. In certain embodiments, the third DNA polymerase is fused to the C-terminal fragment of the third cas protein.

In certain embodiments, the nucleic acid molecule A3 and the nucleic acid molecule B3 are comprised in the same or different expression vectors (e.g., eukaryotic expression vectors). In certain embodiments, the nucleic acid molecule A3 and the nucleic acid molecule B3 are capable of expressing the third Cas protein and the third DNA polymerase in isolation state in a cell, or are capable of expressing a third fusion protein comprising the third Cas protein and the third DNA polymerase in a cell.

In certain embodiments, the system or kit comprises a third fusion protein comprising the third Cas protein and the third DNA polymerase, or, a nucleic acid molecule comprising a nucleotide sequence encoding the third fusion protein, or, the third Cas protein and the third DNA polymerase in isolation state, or, a nucleic acid molecule capable of expressing the third Cas protein and the third DNA polymerase in isolation state.

In certain embodiments, the first, second and third Cas proteins are the same Cas protein, the first, second and third DNA polymerases are the same DNA polymerase; and, the system or kit comprises:
(M1-1) a first fusion protein comprising the first Cas protein and the first DNA polymerase, or, a nucleic acid molecule comprising a nucleotide sequence encoding the first fusion protein; or, (M1-2) the first Cas protein and the first DNA polymerase in isolation state, or a nucleic acid molecule capable of expressing the first Cas protein and the first DNA polymerase in isolation state;
(M2) a first PegRNA comprising the first gRNA and the first tag primer, or a nucleic acid molecule comprising a nucleotide sequence encoding the first PegRNA;
(M3) a second PegRNA comprising the second gRNA and the second tag primer, or a nucleic acid molecule comprising a nucleotide sequence encoding the second PegRNA;
(M4) a third PegRNA comprising the third gRNA and the third tag primer, or, a nucleic acid molecule comprising a nucleotide sequence encoding the third PegRNA.

In certain embodiments, the system or kit further comprises: a nucleic acid vector as defined above.

In certain embodiments, the system or kit further comprises:
(13) a fourth gRNA or a nucleic acid molecule C4 comprising a nucleotide sequence encoding the fourth gRNA, wherein the fourth gRNA is capable of binding to the fourth Cas protein and forming a fourth functional complex; the fourth functional complex is capable of fragmenting two strands of the fourth double-stranded target nucleic acid to form target nucleic acid fragments b1 and b2.

In certain embodiments, the fourth Cas protein is the same as or different from the first, second, or third Cas protein; in certain embodiments, the first, second, third, and fourth Cas proteins are the same Cas protein.

In certain embodiments, the fourth gRNA comprises a fourth guide sequence, and, under conditions that allow hybridization or annealing of nucleic acids, the fourth guide sequence is capable of being hybridized or annealed to one nucleic acid strand of the fourth double-stranded target nucleic acid.

In certain embodiments, the fourth functional complex fragments two strands of the fourth double-stranded target nucleic acid after the fourth guide sequence binds to the fourth double-stranded target nucleic acid.

In certain embodiments, the fourth guide sequence is the same as or different from the first, second or third guide sequence. In certain embodiments, the first, second, third and fourth guide sequences are different from each other.

In certain embodiments, the fourth double-stranded target nucleic acid is the same as or different from the first, second or third double-stranded target nucleic acid. In certain embodiments, the second double-stranded target nucleic acid is the same as the first double-stranded target nucleic acid, and the fourth double-stranded target nucleic acid is the same as the third double-stranded target nucleic acid, but different from the first or second double-stranded target nucleic acid. In certain embodiments, the fourth functional complex and the third functional complex fragment the same double-stranded target nucleic acid at different positions.

In certain embodiments, the fourth functional complex and the third functional complex fragment the same double-stranded target nucleic acid, and the nucleic acid strand bound to the fourth guide sequence is different from the nucleic acid strand bound to the third guide sequence. In certain embodiments, the nucleic acid strand bound to the fourth guide sequence is an opposite strand of the nucleic acid strand bound to the third guide sequence.

In certain embodiments, the fourth double-stranded target nucleic acid is a genomic DNA.

In certain embodiments, the fourth guide sequence has a length of at least 5nt, for example 5-10nt, 10-15nt, 15-20nt, 20-25nt, 25-30nt, 30-40nt, 40-50nt, 50-100nt, 100-200nt, or longer.

In certain embodiments, the fourth gRNA further comprises a fourth scaffold sequence capable of being recognized by and bound to the fourth Cas protein to form a fourth functional complex.

In certain embodiments, the fourth scaffold sequence has a length of at least 20nt, for example, 20-30nt, 30-40nt, 40-50nt, 50-100nt, 100-200nt, or longer.

In certain embodiments, the fourth scaffold sequence is the same as or different from the first, second or third scaffold sequence. In certain embodiments, the first, second, third and fourth scaffold sequences are the same.

In certain embodiments, the fourth guide sequence is located at the upstream or 5' end of the fourth scaffold sequence.

In certain embodiments, the nucleic acid molecule C4 is capable of transcribing the fourth gRNA in a cell.

In certain embodiments, the nucleic acid molecule C4 is comprised in an expression vector (e.g., an eukaryotic expression vector), or, the nucleic acid molecule C4 is an expression vector (e.g. eukaryotic expression vector) comprising a nucleotide sequence encoding the fourth gRNA.

In certain embodiments, the fourth double-stranded target nucleic acid is the same as the third double-stranded target nucleic acid, and the third and fourth functional complexes fragment the same double-stranded target nucleic acid at different positions, thereby forming fragmented nucleotide fragments a1, a2, and a3; wherein, in the same double-stranded target nucleic acid, prior to the fragmentation, the nucleotide fragments a1, a2, and a3 are sequentially arranged (i.e., the nucleotide fragment a1 is linked to the nucleotide fragment a3 through the nucleotide fragment a2); in certain embodiments, the third and fourth functional complexes result in the separation of nucleotide fragments a1 and a2, and the separation of nucleotide fragments a2 and a3, respectively.

In certain embodiments, under conditions that allow hybridization or annealing of nucleic acids, the first tag sequence or complementary sequence thereof or the first overhang is capable of being hybridized or annealed to the nucleotide fragment a1; in certain embodiments, the first tag sequence or complementary sequence thereof or the first overhang is capable of being hybridized or annealed to the nucleotide fragment a1 at the end formed by the fragmentation of the third double-stranded target nucleic acid by the third functional complex. In certain embodiments, the complementary sequence of the first tag sequence or the first overhang is capable of being hybridized or annealed to the 3' end or 3' portion of one nucleic acid strand of the nucleotide fragment a1, and the 3' end or 3' portion is formed by fragmenting the third double-stranded target nucleic acid by the third functional complex.

In certain embodiments, under conditions that allow hybridization or annealing of nucleic acids, the second tag sequence or complementary sequence thereof or the second overhang is capable of being hybridized or annealed to the nucleotide fragment a3. In certain embodiments, the second tag sequence or complementary sequence thereof or the second overhang is capable of being hybridized or annealed to the nucleotide fragment a3 at the end formed by fragmenting the third double-stranded target nucleic acid by the fourth functional complex. In certain embodiments, the complementary sequence of the second tag sequence or the second overhang is capable of being hybridized or annealed to the 3' end or 3' portion of one nucleic acid strand of the nucleotide fragment a3, and the 3' end or 3' portion is formed by fragmenting the third double-stranded target nucleic acid by the fourth functional complex.

In certain embodiments, the fourth Cas protein is different from the first, second or third Cas protein; and, the system or kit further comprises:
(14) the fourth Cas protein or a nucleic acid molecule A4 comprising a nucleotide sequence encoding the fourth Cas protein, wherein the fourth Cas protein is capable of cleaving or fragmenting the fourth double-stranded target nucleic acid.

In certain embodiments, the fourth Cas protein is selected from, but not limited to, Cas9 protein, Cas12a protein, cas12b protein, cas12c protein, cas12d protein, cas12e protein, cas12f protein, cas12g protein, cas12h protein, cas12i protein, cas14 protein, Cas13a protein, Cas1 protein, Cas1B protein, Cas2 protein, Cas3 protein, Cas4 protein, Cas5 protein, Cas6 protein, Cas7 protein, Cas8 protein, Cas 10 protein, Csy1 protein, Csy2 protein, Csy3 protein, Cse1 protein, Cse2 protein, Csc1 protein, Csc2 protein, Csa5 protein, Csn2 protein, Csm2 protein, Csm3 protein, Csm4 protein, Csm5 protein, Csm6 protein, Cmr1 protein, Cmr3 protein, Cmr4 protein, Cmr5 protein, Cmr6 protein, Csb1 protein, Csb2 protein, Csb3 protein, Csx17 protein, Csx14 protein, Csx10 protein, Csx16 protein, CsaX protein, Csx3 protein, Csx1 protein, Csx15 protein, Csf1 protein, Csf2 protein, Csf3 protein, Csf4 protein and homologs or modified forms thereof.

In certain embodiments, the fourth Cas protein is capable of fragmenting the fourth double-stranded target nucleic acid and producing a sticky end or blunt end.

In certain embodiments, the fourth Cas protein is a Cas9 protein, such as a Cas9 protein (spCas9) of *Streptococcus pyogenes (S. pyogenes).*

In certain embodiments, the fourth Cas protein has the amino acid sequence set forth in SEQ ID NO: 1.

In certain embodiments, the nucleic acid molecule A4 is capable of expressing the fourth Cas protein in a cell.

In certain embodiments, the nucleic acid molecule A4 is comprised in an expression vector (e.g., an eukaryotic expression vector), or, the nucleic acid molecule A4 is an expression vector (e.g., an eukaryotic expression vector) comprising a nucleotide sequence encoding the fourth Cas protein.

In certain embodiments, the first, second, third and fourth Cas proteins are the same Cas protein, and the first and second DNA polymerases (and optionally the third DNA polymerase) are the same DNA polymerase; and, the system or kit comprises:
(M1-1) a first fusion protein comprising the first Cas protein and the first DNA polymerase, or, a nucleic acid molecule comprising a nucleotide sequence encoding the first fusion protein; or, (M1-2) the first Cas protein and the first DNA polymerase in isolation state, or a nucleic acid molecule capable of expressing the first Cas protein and the first DNA polymerase in isolation state;
(M2) a first PegRNA comprising the first gRNA and the first tag primer, or a nucleic acid molecule comprising a nucleotide sequence encoding the first PegRNA;
(M3) a second PegRNA comprising the second gRNA and the second tag primer, or a nucleic acid molecule comprising a nucleotide sequence encoding the second PegRNA;
(M4) the third gRNA or a nucleic acid molecule comprising a nucleotide sequence encoding the third gRNA; or, a third PegRNA comprising the third gRNA and a third tag primer, or, a nucleic acid molecule comprising a nucleotide sequence encoding the third PegRNA;
(M5) the fourth gRNA or a nucleic acid molecule comprising a nucleotide sequence encoding the fourth gRNA.

In certain embodiments, the system or kit further comprises: a nucleic acid vector as defined above.

In certain embodiments, the system or kit further comprises:
(15) a fourth tag primer or a nucleic acid molecule D4 comprising a nucleotide sequence encoding the fourth tag primer, wherein the fourth tag primer comprises a fourth tag sequence and a fourth target-binding sequence, the fourth tag sequence is located at the upstream or 5' end of the fourth target-binding sequence; and, under conditions that allow hybridization or annealing of nucleic acids, the fourth target-binding sequence is capable of being hybridized or annealed to the 3' end of one nucleic acid strand of the target nucleic acid fragment b 1 or b2 to form a double-stranded structure, and the fourth tag sequence does not bind to the target nucleic acid fragment b 1 or b2, and is in a free single-stranded state.

In certain embodiments, under conditions that allow hybridization or annealing of nucleic acids, the fourth target-binding sequence is capable of being hybridized or annealed to the 3' end of one nucleic acid strand of the target nucleic acid fragment b 1 or b2, and the 3' end is formed by fragmenting the fourth double-stranded target nucleic acid by the fourth functional complex.

In certain embodiments, the nucleic acid strand bound to the fourth target-binding sequence is different from the nucleic acid strand bound to the fourth guide sequence. In certain embodiments, the nucleic acid strand bound to the fourth target-binding sequence is an opposite strand of the nucleic acid strand bound to the fourth guide sequence.

In certain embodiments, the fourth target-binding sequence has a length of at least 5nt, for example, 5-10nt, 10-15nt, 15-20nt, 20-25nt, 25-30nt, 30-40nt, 40-50nt, 50-100nt, 100-200nt, or longer.

In certain embodiments, the fourth target-binding sequence is different from the first, second or third target-binding sequence. In certain embodiments, the nucleic acid strand bound to the fourth target-binding sequence is different from the nucleic acid strand bound to the third target-binding sequence. In certain embodiments, the nucleic acid strand bound to the fourth target-binding sequence is an opposite strand of the nucleic acid strand bound to the third target-binding sequence.

In certain embodiments, after the fourth target-binding sequence is hybridized or annealed to the 3' end of one nucleic acid strand of the target nucleic acid fragment b 1 or b2, the fourth DNA polymerase is capable of extending the 3' end of the nucleic acid strand by using the fourth tag primer as a template. In certain embodiments, the extension forms a fourth overhang.

In certain embodiments, the fourth tag sequence has a length of at least 4nt, for example, 4-10nt, 10-15nt, 15-20nt, 20-25nt, 25-30nt, 30-40nt, 40-50nt, 50-100nt, 100-200nt, or longer.

In certain embodiments, the fourth tag sequence is the same as or different from the first, second or third tag sequence. In certain embodiments, the fourth tag sequence is different from the first, second or third tag sequence.

In certain embodiments, the fourth DNA polymerase is the same as or different from the first, second or third DNA polymerase. In certain embodiments, the first, second, third and fourth DNA polymerases are the same DNA polymerase.

In certain embodiments, the fourth tag primer is a single-stranded deoxyribonucleic acid or a single-stranded ribonucleic acid.

In certain embodiments, the fourth tag primer is a single-stranded ribonucleic acid, and the fourth DNA polymerase is an RNA-dependent DNA polymerase. or, the fourth tag primer is a single-stranded deoxyribonucleic acid, and the fourth DNA polymerase is a DNA-dependent DNA polymerase.

In certain embodiments, the fourth guide sequence and the third target-binding sequence bind to the same nucleic acid strand, and the binding position of the third target-binding sequence is located at the upstream or 5' end of the binding position of the fourth guide sequence.

In certain embodiments, the third guide sequence and the fourth target-binding sequence bind to the same nucleic acid strand, and the binding position of the fourth target-binding sequence is located at the upstream or 5' end of the binding position of the third guide sequence.

In certain embodiments, the third and fourth overhangs are comprised in different target nucleic acid fragments, and in certain embodiments, are located at nucleic acid strands opposite to each other.

In certain embodiments, under conditions that allow hybridization or annealing of nucleic acids, the complementary sequence of the fourth tag sequence or the fourth overhang is capable of being hybridized or annealed to a nucleic acid strand comprising the first overhang or the second overhang. In certain embodiments, the complementary sequence of the fourth tag sequence or the fourth overhang is capable of being hybridized or annealed to the first overhang or the second overhang or a nucleotide sequence at the upstream thereof.

In certain embodiments, under conditions that allow hybridization or annealing of nucleic acids, the complementary sequence of the third tag sequence or the third overhang is capable of being hybridized or annealed to the first overhang or a nucleotide sequence at the upstream thereof, and the complementary sequence of the fourth tag sequence or the fourth overhang is capable of being hybridized or annealed to the second overhang or a nucleotide sequence at the upstream thereof; or, under conditions that allow hybridization or annealing of nucleic acids, the complementary sequence of the third tag sequence or the third overhang is capable of being hybridized or annealed to the second overhang or a nucleotide sequence at the upstream thereof, and the complementary sequence of the fourth tag sequence or the fourth overhang is capable of being hybridized or annealed to the first overhang or a nucleotide sequence at the upstream thereof.

In certain embodiments, the nucleic acid molecule D4 is capable of transcribing the fourth tag primer in a cell.

In certain embodiments, the nucleic acid molecule D4 is comprised in an expression vector (e.g., an eukaryotic expression vector), or, the nucleic acid molecule D4 is an expression vector (e.g., an eukaryotic expression vector) comprising a nucleotide sequence encoding the fourth tag primer.

In certain embodiments, the fourth DNA polymerase is different from the first, second, or third DNA polymerase; and, the system or kit further comprises:
(16) the fourth DNA polymerase or a nucleic acid molecule B4 comprising a nucleotide sequence encoding the fourth DNA polymerase.

In certain embodiments, the fourth DNA polymerase is selected from, but is not limited to, DNA-dependent DNA polymerase and RNA-dependent DNA polymerase.

In certain embodiments, the fourth DNA polymerase is an RNA-dependent DNA polymerase.

In certain embodiments, the fourth DNA polymerase is a reverse transcriptase, such as the reverse transcriptase listed above, for example, a reverse transcriptase of Moloney murine leukemia virus.

In certain embodiments, the fourth DNA polymerase has the amino acid sequence set forth in SEQ ID NO:4.

In certain embodiments, the nucleic acid molecule B4 is capable of expressing the fourth DNA polymerase in a cell;

In certain embodiments, the nucleic acid molecule B4 is comprised in an expression vector (e.g., an eukaryotic expression vector), or, the nucleic acid molecule B4 is an expression vector (e.g., eukaryotic expression vector) comprising a nucleotide sequence encoding the fourth DNA polymerase.

In certain embodiments, the fourth tag primer is linked to the fourth gRNA.

In certain embodiments, the fourth tag primer is covalently linked to the fourth gRNA with or without a linker.

In certain embodiments, the fourth tag primer is linked to the 3' end of the fourth gRNA, optionally through a linker.

In certain embodiments, the linker is a nucleic acid linker (e.g., a ribonucleic acid linker or a deoxyribonucleic acid linker).

In certain embodiments, the fourth tag primer is a single-stranded ribonucleic acid, and it is linked to the 3' end of the fourth gRNA through a ribonucleic acid linker or not through a ribonucleic acid linker to form a fourth PegRNA.

In certain embodiments, the nucleic acid molecule C4 and the nucleic acid molecule D4 are comprised in the same expression vector (e.g., an eukaryotic expression vector). In certain embodiments, the nucleic acid molecule C4 and the nucleic acid molecule D4 are capable of transcribing a fourth PegRNA comprising the fourth gRNA and the fourth tag primer in a cell.

In certain embodiments, the system or kit comprises: a fourth PegRNA comprising the fourth gRNA and the fourth tag primer, or, a nucleic acid molecule comprising a nucleotide sequence encoding the fourth PegRNA.

In certain embodiments, the fourth Cas protein is isolated or linked to the fourth DNA polymerase.

In certain embodiments, the fourth Cas protein is covalently linked to the fourth DNA polymerase with or without a linker.

In certain embodiments, the linker is a peptide linker, for example, a flexible peptide linker; for example, the linker has the amino acid sequence set forth in SEQ ID NO:51.

In certain embodiments, the fourth Cas protein is fused to the fourth DNA polymerase with or without a peptide linker to form a fourth fusion protein.

In certain embodiments, the fourth Cas protein is linked or fused to the N-terminal of the fourth DNA polymerase optionally through a linker; or, the fourth Cas protein is linked or fused to the C-terminal of the fourth DNA polymerase optionally through a linker.

In certain embodiments, the fourth fusion protein has the amino acid sequence set forth in SEQ ID NO:52.

In certain embodiments, the fourth fusion protein or the fourth cas protein can be split into two parts by an intein splitting system. It is easy to understand that the intein splitting system can split the fourth fusion protein or the fourth cas protein at any amino acid position. For example, in certain embodiments, the intein splitting system performs splitting within the fourth cas protein. Thus, in certain embodiments, the fourth cas protein is split into an N-terminal fragment and a C-terminal fragment. For example, the N-terminal fragment and the C-terminal fragment of the fourth cas protein can be fused to the N-terminal fragment and the C-terminal fragment of the intein, respectively (or to the C-terminal fragment and the N-terminal fragment of the intein, respectively), and the two can be reconstituted to form the active fourth cas protein in a cell. In certain embodiments, the N-terminal fragment and the C-terminal fragment of the fourth cas protein are each inactive in isolated state, but are capable of being reconstituted to form the active fourth cas protein in a cell. Correspondingly, in certain embodiments, the nucleic acid molecule A4 can be split into two parts, which respectively comprise nucleotide sequences encoding the N-terminal fragment and the C-terminal fragment of the fourth cas protein. In addition, it is easy to understand that in the fourth fusion protein, the fourth DNA polymerase can be fused to the N-terminal fragment or the C-terminal fragment of the fourth cas protein. In certain embodiments, the fourth DNA polymerase is fused to the C-terminal fragment of the fourth cas protein.

In certain embodiments, the nucleic acid molecule A4 and the nucleic acid molecule B4 are comprised in the same or different expression vectors (e.g., eukaryotic expression vectors). In certain embodiments, the nucleic acid molecule A4 and the nucleic acid molecule B4 are capable of expressing the fourth Cas protein and the fourth DNA polymerase in isolation state in a cell, or are capable of expressing a fourth fusion protein comprising the fourth Cas protein and the fourth DNA polymerase in a cell.

In certain embodiments, the system or kit comprises a fourth fusion protein comprising the fourth Cas protein and the fourth DNA polymerase, or, comprising a nucleic acid molecule comprising a nucleotide sequence encoding the fourth fusion protein, or, the fourth Cas protein and the fourth DNA polymerase in isolation state, or, a nucleic acid molecule capable of expressing the fourth Cas protein and the fourth DNA polymerase in isolation state.

In certain embodiments, the first, second, third and fourth Cas proteins are the same Cas protein, the first, second, third and fourth DNA polymerases are the same DNA polymerase; and, the system or kit comprises:
(M1-1) a first fusion protein comprising the first Cas protein and the first DNA polymerase, or, a nucleic acid molecule comprising a nucleotide sequence encoding the first fusion protein; or, (M1-2) the first Cas protein and the first DNA polymerase in isolation state, or a nucleic acid molecule capable of expressing the first Cas protein and the first DNA polymerase in isolation state;
(M2) a first PegRNA comprising the first gRNA and the first tag primer, or a nucleic acid molecule comprising a nucleotide sequence encoding the first PegRNA;
(M3) a second PegRNA comprising the second gRNA and the second tag primer, or a nucleic acid molecule comprising a nucleotide sequence encoding the second PegRNA;
(M4) a third PegRNA comprising the third gRNA and the third tag primer, or a nucleic acid molecule comprising a nucleotide sequence encoding the third PegRNA;
(M5) a fourth PegRNA comprising the fourth gRNA and the fourth tag primer, or a nucleic acid molecule comprising a nucleotide sequence encoding the fourth PegRNA.

In certain embodiments, the system or kit further comprises: a nucleic acid vector as defined above.

In certain embodiments, the fourth double-stranded target nucleic acid is the same as the third double-stranded target nucleic acid, and the third and fourth functional complexes fragment the same double-stranded target nucleic acid at different positions to form nucleotide fragments a1, a2 and a3. Wherein, in the same double-stranded target nucleic acid, the nucleotide fragment a1 is linked to the nucleotide fragment a3 through the nucleotide fragment a2.

In certain embodiments, the third and fourth functional complexes result in the separation of nucleotide fragments a1 and a2 and the separation of nucleotide fragments a2 and a3, respectively.

In certain embodiments, the nucleotide fragment a1 has a third overhang formed by extension using the third tag primer as a template; and the nucleotide fragment a3 has a fourth overhang formed by extension using the fourth tag primer as a template.

In certain embodiments, under conditions that allow hybridization or annealing of nucleic acids, the first tag sequence or complementary sequence thereof or the first overhang is capable of being hybridized or annealed to the nucleotide fragment a1. In certain embodiments, the first tag sequence or complementary sequence thereof or the first overhang is capable of being hybridized or annealed to the nucleotide fragment a1 at the end formed by fragmenting the third double-stranded target nucleic acid by the third functional complex. In certain embodiments, the complementary sequence of the first tag sequence or the first overhang is capable of being hybridized or annealed to the 3' end or 3' portion of one nucleic acid strand of the nucleotide fragment a1, and the 3' end or 3' portion is formed by fragmenting the third double-stranded target nucleic acid by the third functional complex. In certain embodiments, the first overhang is capable of being hybridized or annealed to the third overhang of the nucleotide fragment a1 or an upstream nucleotide sequence thereof.

In certain embodiments, the complementary sequence of the third tag sequence or the third overhang is capable of being hybridized or annealed to the first overhang or a nucleotide sequence at the upstream thereof under conditions that allow hybridization or annealing of nucleic acids.

In certain embodiments, under conditions that allow hybridization or annealing of nucleic acids, the second tag sequence or complementary sequence thereof or the second overhang is capable of being hybridized or annealed to the nucleotide fragment a3. In certain embodiments, the second tag sequence or complementary sequence thereof or the second overhang is capable of being hybridized or annealed to the nucleotide fragment a3 at the end formed by fragmenting the third double-stranded target nucleic acid by the fourth functional complex. In certain embodiments, the complementary sequence of the second tag sequence or the second overhang is capable of being hybridized or annealed to the 3' end or 3' portion of one nucleic acid strand of the nucleotide fragment a3, and the 3' end or 3' portion is formed by fragmenting the third double-stranded target nucleic acid by the fourth functional complex. In certain embodiments, the second overhang is capable of being hybridized or annealed to the fourth overhang of the nucleotide fragment a3 or an upstream nucleotide sequence thereof.

In certain embodiments, under conditions that allow hybridization or annealing of nucleic acids, the complementary sequence of the fourth tag sequence or the fourth overhang is capable of being hybridized or annealed to the second overhang or an upstream nucleotide sequence thereof.

In certain embodiments, the kit further comprises an additional component.

In certain embodiments, the additional component comprises one or more selected from the group consisting of:
(1) one or more (e.g., 2, 3, 4, 5, 10, 15, 20, or more) additional gRNAs or a nucleic acid molecule comprising a nucleotide sequence encoding the additional gRNA, wherein the additional gRNA is capable of binding to a Cas protein and forming a functional complex. In certain embodiments, the functional complex is capable of fragmenting two strands of a double-stranded target nucleic acid to form a target nucleic acid fragment.
(2) one or more (e.g., 2, 3, 4, 5, 10, 15, 20, or more) additional Cas proteins or a nucleic acid molecule comprising a nucleotide sequence encoding the additional Cas protein. In certain embodiments, the Cas protein is capable of cleaving or fragmenting a double-stranded target nucleic acid.
(3) one or more (e.g., 2, 3, 4, 5, 10, 15, 20, or more) additional tag primers or a nucleic acid comprising a nucleotide sequence encoding the additional tag primer, wherein the additional tag primer comprises a tag sequence and a target-binding sequence, the tag sequence is located at the upstream or 5' end of the target-binding sequence. In certain embodiments, under conditions that allow hybridization or annealing of nucleic acids, the target-binding sequence is capable of being hybridized or annealed to the 3' end of one nucleic acid strand of the target nucleic acid fragment, to form a double-stranded structure, and, the tag sequence does not bind to the target nucleic acid fragment and is in a free single-stranded state.
(4) one or more (e.g., 2, 3, 4, 5, 10, 15, 20, or more) additional DNA polymerases or a nucleic acid comprising a nucleotide sequence encoding the additional DNA polymerase. In certain embodiments, the additional DNA polymerase is selected from DNA-dependent DNA polymerase and RNA-dependent DNA polymerase. In certain embodiments, the additional DNA polymerase is an RNA-dependent DNA polymerase, such as reverse transcriptase.

In a second aspect, the present application provides a fusion protein, which comprises a Cas protein and a template-dependent DNA polymerase, wherein the Cas protein is capable of fragmenting a double-stranded target nucleic acid.

In certain embodiments, the Cas protein is capable of fragmenting a double-stranded target nucleic acid and producing a sticky end or blunt end.

In certain embodiments, the Cas protein is selected from, but not limited to, Cas9 protein, Cas12a protein, cas12b protein, cas12c protein, cas12d protein, cas12e protein, cas12f protein, cas12g protein, cas12h protein, cas12i protein, cas14 protein, Cas13a protein, Cas1 protein, Cas1B protein, Cas2 protein, Cas3 protein, Cas4 protein, Cas5 protein, Cas6 protein, Cas7 protein, Cas8 protein, Cas10 protein, Csy1 protein, Csy2 protein, Csy3 protein, Cse1 protein, Cse2 protein, Csc1 protein, Csc2 protein, Csa5 protein, Csn2 protein, Csm2 protein, Csm3 protein, Csm4 protein, Csm5 protein, Csm6 protein, Cmr1 protein, Cmr3 protein, Cmr4 protein, Cmr5 protein, Cmr6 protein, Csb1 protein, Csb2 protein, Csb3 protein, Csx17 protein, Csx14 protein, Csx10 protein, Csx16 protein, CsaX protein, Csx3 protein, Csx1 protein, Csx15 protein, Csf1 protein, Csf2 protein, Csf3 protein, Csf4 protein and their homologues or modified forms thereof.

In certain embodiments, the Cas protein is a Cas9 protein, such as a Cas9 protein of *Streptococcus pyogenes* (*S. pyogenes*) (spCas9).

In certain embodiments, the Cas protein has the amino acid sequence set forth in SEQ ID NO: 1.

In certain embodiments, the DNA polymerase is selected from, but is not limited to, DNA-dependent DNA polymerase and RNA-dependent DNA polymerase.

In certain embodiments, the DNA polymerase is an RNA-dependent DNA polymerase.

In certain embodiments, the DNA polymerase is a reverse transcriptase, such as the reverse transcriptases listed above, for example, a reverse transcriptase of Moloney murine leukemia virus.

In certain embodiments, the DNA polymerase has the amino acid sequence set forth in SEQ ID NO:4.

In certain embodiments, the Cas protein is covalently linked to the DNA polymerase with or without a linker.

In certain embodiments, the linker is a peptide linker, for example, a flexible peptide linker; for example, the linker has the amino acid sequence set forth in SEQ ID NO:51.

In certain embodiments, the Cas protein is linked or fused to the N-terminal of the DNA polymerase optionally through a linker; or, the Cas protein is linked or fused to the C-terminal of the DNA polymerase optionally through a linker.

In certain embodiments, the fusion protein has the amino acid sequence set forth in SEQ ID NO:52.

In a third aspect, the present application provides a nucleic acid molecule, which comprises a polynucleotide encoding the fusion protein as described above.

In a fourth aspect, the present application provides a vector, which comprises the nucleic acid molecule as previously described.

In certain embodiments, the vector is an expression vector.

In certain embodiments, the vector is an eukaryotic expression vector.

In a fifth aspect, the present application provides a host cell, which comprises the aforementioned nucleic acid molecule or the aforementioned vector.

In certain embodiments, the host cell is a prokaryotic cell, for example, E. *coli* cell; or the host cell is an eukaryotic cell, for example, yeast cell, fungal cell, plant cell, animal cell.

In certain embodiments, the host cell is a mammalian cell, for example, a human cell.

In a fifth aspect, the present application provides a method for preparing the aforementioned fusion protein, comprising, (1) culturing the aforementioned host cell under conditions that allow the expression of the protein; and (2) isolating the fusion protein expressed by the host cell.

In a sixth aspect, the present application provides a complex, which comprises a first Cas protein and a first DNA polymerase that is template-dependent, wherein the first Cas protein has the ability to fragment a double-stranded target nucleic acid, and, the first Cas protein is complexed with the first DNA polymerase in a covalent or non-covalent manner.

In certain embodiments, the first Cas protein is capable of fragmenting a double-stranded target nucleic acid and producing a sticky end or blunt end.

In certain embodiments, the first Cas protein is selected from, but not limited to, Cas9 protein, Cas 12a protein, cas12b protein, cas12c protein, cas12d protein, cas12e protein, cas12f protein, cas12g protein, cas12h protein, cas12i protein, cas14 protein, Cas13a protein, Cas1 protein, Cas1B protein, Cas2 protein, Cas3 protein, Cas4 protein, Cas5 protein, Cas6 protein, Cas7 protein, Cas8 protein, Cas10 protein, Csy1 protein, Csy2 protein, Csy3 protein, Cse1 protein, Cse2 protein, Csc1 protein, Csc2 protein, Csa5 protein, Csn2 protein, Csm2 protein, Csm3 protein, Csm4 protein, Csm5 protein, Csm6 protein, Cmr1 protein, Cmr3 protein, Cmr4 protein, Cmr5 protein, Cmr6 protein, Csb1 protein, Csb2 protein, Csb3 protein, Csx17 protein, Csx14 protein, Csx10 protein, Csx16 protein, CsaX protein, Csx3 protein, Csx1 protein, Csx15 protein, Csf1 protein, Csf2 protein, Csf3 protein, Csf4 protein and homologs or modified forms thereof.

In certain embodiments, the first Cas protein is a Cas9 protein, such as a Cas9 protein of *Streptococcus pyogenes* (*S. pyogenes*) (spCas9).

In certain embodiments, the first Cas protein has the amino acid sequence set forth in SEQ ID NO: 1.

In certain embodiments, the first DNA polymerase is selected from, but is not limited to, DNA-dependent DNA polymerase and RNA-dependent DNA polymerase.

In certain embodiments, the first DNA polymerase is an RNA-dependent DNA polymerase.

In certain embodiments, the first DNA polymerase is a reverse transcriptase, such as the reverse transcriptase listed above, for example, a reverse transcriptase of Moloney murine leukemia virus.

In certain embodiments, the first DNA polymerase has the amino acid sequence set forth in SEQ ID NO:4.

In certain embodiments, the first Cas protein is covalently linked to the first DNA polymerase with or without a linker.

In certain embodiments, the linker is a peptide linker, for example, a flexible peptide linker; for example, the linker has the amino acid sequence set forth in SEQ ID NO:51.

In certain embodiments, the first Cas protein is fused to the first DNA polymerase with or without a peptide linker to form a first fusion protein.

In certain embodiments, the first Cas protein is linked or fused to the N-terminal of the first DNA polymerase optionally through a linker; or, the first Cas protein is linked or fused to the C-terminal of the first DNA polymerase optionally through a linker.

In certain embodiments, the first fusion protein has the amino acid sequence set forth in SEQ ID NO:52.

In certain embodiments, the complex further comprises a first gRNA.

In certain embodiments, the first gRNA is capable of binding to the first Cas protein and forming a first functional unit; the first functional unit is capable of binding to a double-stranded target nucleic acid and fragmenting two strands thereof to form target nucleic acid fragments.

In certain embodiments, the first gRNA comprises a first guide sequence, and, under conditions that allow hybridization or annealing of nucleic acids, the first guide sequence is capable of being hybridized or annealed to one nucleic acid strand of a double-stranded target nucleic acid.

In certain embodiments, the first guide sequence has a length of at least 5nt, for example 5-10nt, 10-15nt, 15-20nt, 20-25nt, 25-30nt, 30-40nt, 40-50nt, 50-100nt, 100-200nt, or longer.

In certain embodiments, the first gRNA further comprises a first scaffold sequence, which is capable of being recognized by and bound to the first Cas protein to form a first functional unit.

In certain embodiments, the first scaffold sequence has a length of at least 20nt, for example, 20-30nt, 30-40nt, 40-50nt, 50-100nt, 100-200nt, or longer.

In certain embodiments, the first guide sequence is located at the upstream or 5' end of the first scaffold sequence.

In certain embodiments, after the first guide sequence binds to the double-stranded target nucleic acid, the complex or the first functional unit is capable of fragmenting two strands of the double-stranded target nucleic acid to form a target nucleic acid fragment.

In certain embodiments, the complex further comprises a double-stranded target nucleic acid.

In certain embodiments, the double-stranded target nucleic acid comprises a first PAM sequence recognized by the first Cas protein and a first guide binding sequence capable of being hybridized or annealed to the first guide sequence, whereby the first functional unit binds to the double-stranded target nucleic acid through the first guide binding sequence and the first PAM sequence.

In certain embodiments, the complex further comprises a first tag primer that is hybridized or annealed to the double-stranded target nucleic acid; wherein the first tag primer comprises a first target-binding sequence, which is capable of hybridizing to annealing to the double-stranded target nucleic acid.

In certain embodiments, the tag primer comprises a first tag sequence and a first target-binding sequence, the first tag sequence is located at the upstream or 5' end of the first target-binding sequence; and, under conditions allowing hybridization or annealing of nucleic acids, the first target-binding sequence is capable of being hybridized or annealed to the double-stranded target nucleic acid. In certain embodiments, the first target-binding sequence is capable of being hybridized or annealed to the position where the double-stranded target nucleic acid is fragmented by the first functional unit; in certain embodiments, the first target-binding sequence is capable of being hybridized or annealed to the 3' end of one nucleic acid strand of the target nucleic acid fragment to form a double-stranded structure. In certain embodiments, the 3' end is formed by fragmenting the double-stranded target nucleic acid by the first functional unit; in certain embodiments, the first tag sequence does not bind to the target nucleic acid fragment and is in a free single-stranded state.

In certain embodiments, the first target-binding sequence has a length of at least 5 nt, for example, 5-10 nt, 10-15 nt, 15-20 nt, 20-25 nt, 25-30 nt, 30-40 nt, 40-50 nt, 50-100nt, 100-200nt, or longer.

In certain embodiments, the first tag sequence has a length of at least 4nt, for example, 4-10nt, 10-15nt, 15-20nt, 20-25nt, 25-30nt, 30-40nt, 40-50nt, 50-100nt, 100-200nt, or longer.

In certain embodiments, the first tag primer binds to the target nucleic acid fragment through the first target-binding sequence; in certain embodiments, the first DNA polymerase binds to the target nucleic acid fragment and the first tag primer.

In certain embodiments, the first tag primer is a single-stranded deoxyribonucleic acid or a single-stranded ribonucleic acid.

In certain embodiments, the first tag primer is a single-stranded ribonucleic acid, and the first DNA polymerase is an RNA-dependent DNA polymerase; or, the first tag primer is a single-stranded deoxyribonucleic acid, and the first DNA polymerase is a DNA-dependent DNA polymerase.

In certain embodiments, the target nucleic acid fragment is extended by the first DNA polymerase using the first tag primer as a template to form a first overhang.

In certain embodiments, the nucleic acid strand to which the first gRNA binds is different from the nucleic acid strand to which the first tag primer binds. In certain embodiments, the nucleic acid strand to which the first gRNA binds is an opposite strand of the nucleic acid strand to which the first tag primer binds.

In certain embodiments, the first tag primer is linked to the first gRNA.

In certain embodiments, the first tag primer is covalently linked to the first gRNA with or without a linker.

In certain embodiments, the first tag primer is linked to the 3' end of the first gRNA, optionally through a linker.

In certain embodiments, the linker is a nucleic acid linker (e.g., a ribonucleic acid linker or a deoxyribonucleic acid linker).

In certain embodiments, the first tag primer is a single-stranded ribonucleic acid, and it is linked to the 3' end of the first gRNA through a ribonucleic acid linker or not through a ribonucleic acid linker to form a first PegRNA.

In certain embodiments, the complex further comprises a second Cas protein and a second gRNA, wherein the second Cas protein has the ability to fragment a double-stranded target nucleic acid, the second gRNA is capable of binding to the second Cas protein and forming a second functional unit; the second functional unit is capable of binding to a double-stranded target nucleic acid and fragmenting two strands thereof to form a target nucleic acid fragment.

In certain embodiments, the second Cas protein is the same as or different from the first Cas protein. In certain embodiments, the second Cas protein is the same as the first Cas protein.

In certain embodiments, the second Cas protein is capable of fragmenting the double-stranded target nucleic acid and producing a sticky end or blunt end.

In certain embodiments, the second Cas protein is selected from, but not limited to, Cas9 protein, Cas12a protein, cas12b protein, cas12c protein, Cas 12d protein, Cas12e protein, Cas12f protein, Cas12g protein, Cas12h protein, Cas12i protein, Cas14 protein, Cas13a protein, Cas1 protein, Cas1B protein, Cas2 protein, Cas3 protein, Cas4 protein, Cas5 protein, Cas6 protein, Cas7 protein, Cas8 protein, Cas10 protein, Csy1 protein, Csy2 protein, Csy3 protein, Cse1 protein, Cse2 protein, Csc1 protein, Csc2 protein, Csa5 protein, Csn2 protein, Csm2 protein, Csm3 protein, Csm4 protein, Csm5 protein, Csm6 protein, Cmr1 protein, Cmr3 protein, Cmr4 protein, Cmr5 protein, Cmr6 protein, Csb1 protein, Csb2 protein, Csb3 protein, Csx17 protein, Csx14 protein, Csx10 protein, Csx16 protein, CsaX protein, Csx3 protein, Csx1 protein, Csx15 protein, Csf1 protein, Csf2 protein, Csf3 protein, Csf4 protein and homologues or modified forms thereof.

In certain embodiments, the second Cas protein is a Cas9 protein, such as a Cas9 protein of *Streptococcus pyogenes* (*S. pyogenes*) (spCas9).

In certain embodiments, the second Cas protein has the amino acid sequence set forth in SEQ ID NO: 1.

In certain embodiments, the second gRNA comprises a second guide sequence, and under conditions that allow hybridization or annealing of nucleic acids, the second guide sequence is capable of being hybridized or annealed to one nucleic acid strand of the double-stranded target nucleic acid.

In certain embodiments, the second guide sequence is different from the first guide sequence; in certain embodiments, the nucleic acid strand to which the first guide sequence binds is different from the nucleic acid strand to which the second guide sequence binds. In certain embodiments, the nucleic acid strand to which the first guide sequence binds is an opposite strand of the nucleic acid strand to which the second guide sequence binds.

In certain embodiments, the second guide sequence has a length of at least 5nt, for example, 5-10nt, 10-15nt, 15-20nt, 20-25nt, 25-30nt, 30-40nt, 40-50nt, 50-100nt, 100-200nt, or longer.

In certain embodiments, the second gRNA further comprises a second scaffold sequence, which is capable of being recognized by and bound to the second Cas protein to form a second functional unit.

In certain embodiments, the second scaffold sequence is the same as or different from the first scaffold sequence. In certain embodiments, the second scaffold sequence is the same as the first scaffold sequence.

In certain embodiments, the second scaffold sequence has a length of at least 20nt, for example, 20-30nt, 30-40nt, 40-50nt, 50-100nt, 100-200nt, or longer.

In certain embodiments, the second guide sequence is located at the upstream or 5' end of the second scaffold sequence.

In certain embodiments, the double-stranded target nucleic acid comprises a second PAM sequence recognized by the second Cas protein and a second guide binding sequence capable of being hybridized or annealed to the second guide sequence, whereby the second functional unit binds to the double-stranded target nucleic acid through the second guide binding sequence and the second PAM sequence.

In certain embodiments, the complex further comprises a second template-dependent DNA polymerase, the second DNA polymerase is complexed with the second Cas protein in covalent or non-covalent manner.

In certain embodiments, the second DNA polymerase is selected from, but is not limited to, DNA-dependent DNA polymerase and RNA-dependent DNA polymerase.

In certain embodiments, the second DNA polymerase is an RNA-dependent DNA polymerase.

In certain embodiments, the second DNA polymerase is a reverse transcriptase, such as the reverse transcriptase listed above, for example, a reverse transcriptase of Moloney murine leukemia virus.

In certain embodiments, the second DNA polymerase has the amino acid sequence set forth in SEQ ID NO:4.

In certain embodiments, the second DNA polymerase is the same as or different from the first DNA polymerase. In certain embodiments, the second DNA polymerase is the same as the first DNA polymerase.

In certain embodiments, the second Cas protein is covalently linked to the second DNA polymerase with or without a linker.

In certain embodiments, the linker is a peptide linker, for example, a flexible peptide linker; for example, the linker has the amino acid sequence set forth in SEQ ID NO:51.

In certain embodiments, the second Cas protein is fused to the second DNA polymerase with or without a peptide linker to form a fusion second protein.

In certain embodiments, the second Cas protein is linked or fused to the N-terminal of the second DNA polymerase optionally through a linker; or, the second Cas protein is linked or fused to the C-terminal of the second DNA polymerase optionally through a linker.

In certain embodiments, the second fusion protein has the amino acid sequence set forth in SEQ ID NO:52.

In certain embodiments, the complex further comprises a second tag primer that is hybridized or annealed to the double-stranded target nucleic acid; wherein the second tag primer comprises a second target-binding sequence capable of being hybridized or annealed to the double-stranded target nucleic acid.

In certain embodiments, the tag primer comprises a second tag sequence and a second target-binding sequence, the second tag sequence is located at the upstream or 5' end of the second target-binding sequence; and, under conditions allowing hybridization or annealing of nucleic acid, the second target-binding sequence is capable of being hybridized or annealed to the double-stranded target nucleic acid. In certain embodiments, the second target-binding sequence is capable of being hybridized or annealed to the position where the double-stranded target nucleic acid is fragmented by the second functional unit; in certain embodiments, the second target binding sequence is capable of being hybridized or annealed to the 3' end of one nucleic acid strand of the target nucleic acid fragment to form a double-stranded structure. In certain embodiments, the 3' end is formed by fragmenting the double-stranded target nucleic acid by the second functional unit. In certain embodiments, the second tag sequence does not bind to the target nucleic acid fragment and is in a free single-stranded state.

In certain embodiments, the second target-binding sequence has a length of at least 5nt, for example, 5-10nt, 10-15nt, 15-20nt, 20-25nt, 25-30nt, 30-40nt, 40-50nt, 50-100nt, 100-200nt, or longer.

In certain embodiments, the second target-binding sequence is different from the first target-binding sequence. In certain embodiments, the nucleic acid strand bound to the second target-binding sequence is different from the nucleic acid strand bound to the first target-binding sequence. In certain embodiments, the nucleic acid strand bound to the second target-binding sequence is an opposite strand of the nucleic acid strand bound to the first target-binding sequence.

In certain embodiments, the second tag sequence has a length of at least 4nt, for example, 4-10nt, 10-15nt, 15-20nt, 20-25nt, 25-30nt, 30-40nt, 40-50nt, 50-100nt, 100-200nt, or longer.

In certain embodiments, the second tag sequence is the same as or different from the first tag sequence. In certain embodiments, the second tag sequence is different from the first tag sequence.

In certain embodiments, the second tag primer binds to the target nucleic acid fragment through the second target-binding sequence. In certain embodiments, the second DNA polymerase binds to the target nucleic acid fragment and the second tag primer.

In certain embodiments, the second tag primer is a single-stranded deoxyribonucleic acid or a single-stranded ribonucleic acid.

In certain embodiments, the second tag primer is a single-stranded ribonucleic acid, and the second DNA polymerase is an RNA-dependent DNA polymerase; or, the second tag primer is a single-stranded deoxyribonucleic acid, and the second DNA polymerase is a DNA-dependent DNA polymerase.

In certain embodiments, the target nucleic acid fragment is extended by the second DNA polymerase using the second tag primer as a template to form a second overhang.

In certain embodiments, the nucleic acid strand to which the second gRNA binds is different from the nucleic acid strand to which the second tag primer binds; in certain embodiments, the nucleic acid strand to which the second gRNA binds is an opposite strand of the nucleic acid strand to which the second tag primer binds.

In certain embodiments, the second tag primer is linked to the second gRNA.

In certain embodiments, the second tag primer is covalently linked to the second gRNA with or without a linker.

In certain embodiments, the second tag primer is linked to the 3' end of the second gRNA, optionally through a linker.

In certain embodiments, the linker is a nucleic acid linker (e.g., a ribonucleic acid linker or a deoxyribonucleic acid linker).

In certain embodiments, the second tag primer is a single-stranded ribonucleic acid, and is linked to the 3' end of the second gRNA through a ribonucleic acid linker or not through a ribonucleic acid linker to form a second PegRNA.

In certain embodiments, the first and second functional units bind a double-stranded target nucleic acid in a predetermined positional relationship.

In certain embodiments, the second guide sequence and the first target-binding sequence bind to the same nucleic acid strand; and/or, the first guide sequence and the second target-binding sequence bind the same nucleic acid strand.

In certain embodiments, the binding position of the second guide sequence is located at the upstream or 5' end of the binding position of the first target-binding sequence; and/or, the binding position of the first guide sequence is located at the upstream or 5' end of the binding site of the second target-binding sequence.

In certain embodiments, the binding position of the second guide sequence is located at downstream or 3' end of the binding position of the first target-binding sequence; and/or, the binding position of the first guide sequence is located at the downstream or 3' end of the binding site of the second target-binding sequence.

In certain embodiments, the double-stranded target nucleic acid is selected from, but not limited to, genomic DNA and nucleic acid vector DNA.

In a seventh aspect, the present application provides a method for fragmenting a double-stranded target nucleic acid and adding an overhang at its 3' end, wherein the method comprises using the system or kit as previously described.

In certain embodiments, the method comprises the steps of:
i. providing a double-stranded target nucleic acid; and
   providing the first Cas protein, the first gRNA, the first DNA polymerase and the first tag primer;
ii. contacting the double-stranded target nucleic acid with the first Cas protein, the first gRNA, the first DNA polymerase and the first tag primer.

In certain embodiments, in step ii:
the first Cas protein binds to the first gRNA to form a first functional complex, and the first functional complex binds to and fragments the double-stranded target nucleic acid to form a target nucleic acid fragment; and,
the first tag primer is hybridized or annealed to the 3' end of one nucleic acid strand of the target nucleic acid fragment via the first target-binding sequence; and,
the first DNA polymerase extends the target nucleic acid fragment to form a first overhang by using the first tag primer annealed to the target nucleic acid fragment as a template.

In certain embodiments, the method is performed in a cell.

In certain embodiments, in step i, the first Cas protein or nucleic acid molecule A1, the first DNA polymerase or nucleic acid molecule B 1, the first gRNA or nucleic acid molecule C1 and the first tag primer or nucleic acid molecule D 1 is delivered into a cell, to provide the first Cas protein, the first gRNA, the first DNA polymerase and the first tag primer in the cell.

In certain embodiments, in step i, the nucleic acid molecule A1, the nucleic acid molecule B1, the first gRNA or nucleic acid molecule C 1 and the first tag primer or nucleic acid molecule D 1 are delivered into a cell, to provide the first Cas protein, the first gRNA, the first DNA polymerase and the first tag primer in the cell.

In certain embodiments, in step i, the nucleic acid molecules A1, B1, C1 and D1 are delivered into a cell, to provide the first Cas protein, the first gRNA, the first DNA polymerase and the first tag primer in the cell.

In certain embodiments, the nucleic acid molecule A1 and the nucleic acid molecule B1 are comprised in the same or different expression vectors (e.g., eukaryotic expression vectors). In certain embodiments, the nucleic acid molecule A1 and the nucleic acid molecule B1 are capable of expressing the first Cas protein and the first DNA polymerase in isolation state in a cell, or are capable of expressing a first fusion protein comprising the first Cas protein and the first DNA polymerase in a cell. In certain embodiments, in step i, a nucleic acid molecule capable of expressing the first Cas protein and a first DNA polymerase in isolation state or a nucleic acid molecule comprising a nucleotide sequence encoding the first fusion protein is delivered into a cell and expressed in the cell, to provide the first Cas protein and the first DNA polymerase in the cell.

In certain embodiments, the nucleic acid molecule C1 and the nucleic acid molecule D1 are comprised in the same expression vector (e.g., an eukaryotic expression vector). In certain embodiments, the nucleic acid molecule C1 and the nucleic acid molecule D1 are capable of transcribing a first PegRNA comprising the first gRNA and the first tag primer in a cell. In certain embodiments, in step i, the first PegRNA is delivered into a cell to provide the first gRNA and the first tag primer in the cell, or, a nucleic acid molecule comprising a nucleotide sequence encoding the first PegRNA is delivered into a cell, and the first PegRNA is transcribed in the cell, to provide the first gRNA and the first tag primer in the cell.

In certain embodiments, in step i, a nucleic acid molecule capable of expressing the first Cas protein and a first DNA polymerase in isolation state or a nucleic acid molecule comprising a nucleotide sequence encoding the first fusion protein, and a nucleic acid molecule comprising a nucleotide sequence encoding the first PegRNA is delivered into a cell, and is transcribed and expressed in the cell, thereby providing the first Cas protein, the first gRNA, the first DNA polymerase and the first tag primer in the cell.

In certain embodiments, in step i, the double-stranded target nucleic acid or a nucleic acid molecule T comprising the double-stranded target nucleic acid is delivered into a cell, to provide the double-stranded target nucleic acid in the cell.

In certain embodiments, the first Cas protein, the first gRNA, the first DNA polymerase, or the first tag primer are as defined above.

In certain embodiments, the double-stranded target nucleic acid or the nucleic acid molecule T comprises a first PAM sequence recognized by the first Cas protein. In certain embodiments, in step ii, the first functional complex binds to the double-stranded target nucleic acid or the nucleic acid molecule T through the first PAM sequence and the first gRNA, and fragments double-stranded target nucleic acid or nucleic acid molecule T.

In an eighth aspect, the present application provides a method for fragmenting a double-stranded target nucleic acid into target nucleic acid fragments, and adding overhangs to the two 3' ends of the target nucleic acid fragments, wherein the method comprises, using the system or kit as previously described; wherein the first double-stranded target nucleic acid and the second double-stranded target nucleic acid are the same.

In certain embodiments, the method comprises the steps of:
i. providing a double-stranded target nucleic acid; and
   providing the first Cas protein, the first gRNA, the first DNA polymerase, the first tag primer, the second Cas protein, the second gRNA, the second DNA polymerase and the second tag primer;
ii. contacting the double-stranded target nucleic acid with the first Cas protein, the first gRNA, the first DNA polymerase, the first tag primer, the second Cas protein, the second gRNA, the second DNA polymerase, and the second tag primer.

In certain embodiments, in step ii:
the first Cas protein binds to the first gRNA to form a first functional complex, and the second Cas protein binds to the second gRNA to form a second functional complex; and, the first and second functional complexes bind and fragment the double-stranded target nucleic acid, thereby forming a target nucleic acid fragment F1; and,
the first tag primer is hybridized or annealed to the 3' end of one nucleic acid strand of the target nucleic acid fragment F1 through the first target-binding sequence; and, the second tag primer is hybridized or annealed to the 3' end of the other nucleic acid strand of the target nucleic acid fragment F1 through the second target-binding sequence; and,
the first DNA polymerase and the second DNA polymerase extend the target nucleic acid fragment F1 respectively using the first tag primer and the second tag primer annealed to the target nucleic acid fragment F1 as templates to form a target nucleic acid fragment F2 having a first overhang and a second overhang.

In certain embodiments, the method is performed in a cell.

In certain embodiments, in step i, the first Cas protein or nucleic acid molecule A1, the first DNA polymerase or nucleic acid molecule B1, the first gRNA or nucleic acid molecule C1, the first tag primer or nucleic acid molecule D1, the second Cas protein or nucleic acid molecule A2, the second DNA polymerase or nucleic acid molecule B2, the second gRNA or nucleic acid molecule C2, and the second tag primer or nucleic acid molecule D2 is delivered into a cell, to provide the first Cas protein, the first gRNA, the first DNA polymerase, the first tag primer, the second Cas protein, the second gRNA, the second DNA polymerase and the second tag primer in the cell.

In certain embodiments, in step i, the nucleic acid molecule A1, the nucleic acid molecule B1, the first gRNA or nucleic acid molecule C1, the first tag primer or nucleic acid molecule D1, the nucleic acid molecule A2, the nucleic acid molecule B2, the second gRNA or nucleic acid molecule C2, and the second tag primer or nucleic acid molecule D2 is delivered into a cell, to provide the first Cas protein, the first gRNA, the first Cas protein, the first A DNA polymerase, the first tag primer, the second Cas protein, the second gRNA, the second DNA polymerase, and the second tag primer in the cell.

In certain embodiments, in step i, the nucleic acid molecules A1, B1, C1, D1, A2, B2, C2 and D2 are delivered into a cell to provide the first Cas protein, the first gRNA, the first DNA polymerase, the first tag primer, the second Cas protein, the second gRNA, the second DNA polymerase, and the second tag primer in the cell.

In certain embodiments, the nucleic acid molecule A1 and nucleic acid molecule B1 are comprised in the same or different expression vectors (e.g., eukaryotic expression vectors). In certain embodiments, the nucleic acid molecule A1 and the nucleic acid molecule B1 are capable of expressing the first Cas protein and the first DNA polymerase in isolation state in a cell, or are capable of expressing a first fusion protein comprising the first Cas protein and the first DNA polymerase. In certain embodiments, in step i, a nucleic acid molecule capable of expressing the first Cas protein and the first DNA polymerase in isolation state or a nucleic acid molecule comprising a nucleotide sequence encoding the first fusion protein is delivered into a cell, and expressed in the cell, to provide the first Cas protein and the first DNA polymerase in the cell.

In certain embodiments, the nucleic acid molecule A2 and the nucleic acid molecule B2 are comprised in the same or different expression vectors (e.g., eukaryotic expression vectors). In certain embodiments, the nucleic acid molecule A2 and the nucleic acid molecule B2 are capable of expressing the second Cas protein and the second DNA polymerase in isolation state in a cell, or are capable of expressing a second fusion protein comprising the second Cas protein and the second DNA polymerase. In certain embodiments, in step i, a nucleic acid molecule capable of expressing the second Cas protein and the second DNA polymerase in isolation state or a nucleic acid molecule comprising a nucleotide sequence encoding the second fusion protein is delivered into a cell, and expressed in the cell, to provide the second Cas protein and the second DNA polymerase in the cell.

In certain embodiments, the nucleic acid molecule C1 and the nucleic acid molecule D1 are comprised in the same expression vector (e.g., an eukaryotic expression vector). In certain embodiments, the nucleic acid molecule C1 and the nucleic acid molecule D1 are capable of transcribing in a cell a first PegRNA comprising the first gRNA and the first tag primer in a cell. In certain embodiments, in step i, the first PegRNA is delivered into a cell to provide the first gRNA and the first tag primer in the cell, or, a nucleic acid comprising a nucleotide sequence encoding the first PegRNA is delivered into a cell, and the first PegRNA is transcribed in the cell to provide the first gRNA and the first tag primer in the cell.

In certain embodiments, the nucleic acid molecule C2 and the nucleic acid molecule D2 are comprised in the same expression vector (e.g., an eukaryotic expression vector). In certain embodiments, the nucleic acid molecule C2 and the nucleic acid molecule D2 are capable of transcribing in a cell a second PegRNA comprising the second gRNA and the second tag primer in a cell. In certain embodiments, in step i, the second PegRNA is delivered into the cell to provide the second gRNA and the second tag primer in the cell, or, a nucleic acid comprising a nucleotide sequence encoding the second PegRNA is delivered into the cell, and the second PegRNA is transcribed in the cell to provide the second gRNA and the second tag primer in the cell.

In certain embodiments, in step i, the double-stranded target nucleic acid or a nucleic acid molecule T comprising the double-stranded target nucleic acid is delivered into a cell to provide the double-stranded target nucleic acid in the cell.

In certain embodiments, the first Cas protein, the first gRNA, the first DNA polymerase, or the first tag primer are as defined above.

In certain embodiments, the second Cas protein, the second gRNA, the second DNA polymerase, or the second tag primer is as defined above.

In certain embodiments, the double-stranded target nucleic acid or the nucleic acid molecule T comprises a first PAM sequence recognized by the first Cas protein and a second PAM sequence recognized by the second Cas protein. In certain embodiments, in step ii, the first functional complex binds to the double-stranded target nucleic acid or the nucleic acid molecule T through the first PAM sequence and the first gRNA, and fragments it; and, the second functional complex binds to the double-stranded target nucleic acid or the nucleic acid molecule T through the second PAM sequence and the second gRNA, and fragments it.

In certain embodiments, the second Cas protein is the same as the first Cas protein, and the second DNA polymerase is the same as the first DNA polymerase; wherein the first Cas protein combines with the first and second gRNAs to form the first and second functional complexes, respectively, and the first DNA polymerase extends the target nucleic acid fragment F1 using the first tag primer and the second tag primer annealed to the target nucleic acid fragment F1 as templates, respectively, to form a target nucleic acid fragment F2 having a first overhang and a second overhang.

In certain embodiments, in step i, the first Cas protein or nucleic acid molecule A1, the first DNA polymerase or nucleic acid molecule B1, the first gRNA or nucleic acid molecule C1, the first tag primer or nucleic acid molecule D 1, the second gRNA or nucleic acid molecule C2, and the second tag primer or nucleic acid molecule D2 are delivered into a cell, to provide the first Cas protein, the first gRNA, the first DNA polymerase, the first tag primer, the second gRNA and the second tag primer in the cell.

In certain embodiments, in step i, the nucleic acid molecule A1, the nucleic acid molecule B1, the first gRNA or nucleic acid molecule C 1, the first tag primer or nucleic acid molecule D 1, the second gRNA or nucleic acid molecule C2 and the second tag primer or nucleic acid molecule D2 are delivered into a cell, to provide the first Cas protein, the first gRNA, the first DNA polymerase, the first tag primer, the second gRNA and the second tag primer in the cell.

In certain embodiments, in step i, the nucleic acid molecules A1, B1, C1, D1, C2 and D2 are delivered into a cell, to provide the first Cas protein, the first gRNA, the first DNA polymerase, the first tag primer, the second gRNA and the second tag primer in the cell.

In certain embodiments, the nucleic acid molecule A1 and nucleic acid molecule B1 are comprised in the same or different expression vectors (e.g., eukaryotic expression vectors). In certain embodiments, the nucleic acid molecule A1 and the nucleic acid molecule B1 are capable of expressing the first Cas protein and the first DNA polymerase in isolation state in a cell, or are capable of expressing a first fusion protein comprising the first Cas protein and the first DNA polymerase in a cell. In certain embodiments, in step i, a nucleic acid molecule capable of expressing the first Cas protein and the first DNA polymerase in isolation state or a nucleic acid molecule comprising a nucleotide sequence encoding the first fusion protein is delivered into a cell, and expressed in the cell to provide the first Cas protein and the first DNA polymerase in the cell.

In certain embodiments, the nucleic acid molecule C1 and the nucleic acid molecule D 1 are comprised in the same expression vector (e.g., an eukaryotic expression vector). In certain embodiments, the nucleic acid molecule C 1 and the nucleic acid molecule D1 are capable of transcribing a first PegRNA comprising the first gRNA and the first tag primer in a cell. In certain embodiments, in step i, the first PegRNA is delivered into a cell to provide the first gRNA and the first tag primer in the cell, or, a nucleic acid molecule comprising a nucleotide sequence encoding the first PegRNA is delivered into the cell, and the first PegRNA is transcribed in the cell, to provide the first gRNA and the first tag primer in the cell.

In certain embodiments, the nucleic acid molecule C2 and the nucleic acid molecule D2 are comprised in the same expression vector (e.g., an eukaryotic expression vector). In certain embodiments, the nucleic acid molecule C2 and the nucleic acid molecule D2 are capable of transcribing a second PegRNA comprising the second gRNA and the second tag primer in a cell. In certain embodiments, in step i, the second PegRNA is delivered into the cell to provide the second gRNA and the second tag primer in the cell, or, a nucleic acid molecule comprising a nucleotide sequence encoding the second PegRNA is delivered into the cell, and the second PegRNA is transcribed in the cell to provide the second gRNA and the second tag primer in the cell.

In certain embodiments, in step i, a nucleic acid molecule capable of expressing the first Cas protein and the first DNA polymerase in isolation state or a nucleic acid molecule comprising a nucleotide sequence encoding the first fusion protein, a nucleic acid molecule comprising a nucleotide sequence encoding the first PegRNA and a nucleic acid molecule comprising a nucleotide sequence encoding the second PegRNA are delivered into a cell, and are transcribed and expressed in the cell, thereby providing the first Cas protein, the first gRNA, the first DNA polymerase, the first tag primer, the second gRNA and the second tag primer in the cell.

In a ninth aspect, the present application provides a method for inserting a target nucleic acid fragment into a nucleic acid molecule of interest; wherein the method comprises using the aforementioned system or kit; wherein the first double-stranded target nucleic acid is the same as the second double-stranded target nucleic acid for providing the target nucleic acid fragment; and the third double-stranded target nucleic acid is a nucleic acid molecule of interest.

In certain embodiments, the method comprises:
a. by the method described above, the first double-stranded target nucleic acid is fragmented into a target nucleic acid fragment F 1, and the two 3' ends of the target nucleic acid fragment F1 are added with overhangs respectively, thereby forming a target nucleic acid fragment F2 having a first overhang and a second overhang;
b. the nucleic acid molecule of interest is fragmented with the third functional complex to form nucleotide fragments a1 and a2; and,
c. the target nucleic acid fragment F2 is used to link the nucleotide fragments a1 and a2, thereby inserting the target nucleic acid fragment into the nucleic acid molecule of interest.

In certain embodiments, the method comprises the steps of:
i. providing the double-stranded target nucleic acid and the nucleic acid molecule of interest; and providing the first Cas protein, the first gRNA, the first DNA polymerase, the first tag primer, the second Cas protein, the second gRNA, the second DNA polymerase, the second tag primer, the third Cas protein, and the third gRNA;
ii. contacting the double-stranded target nucleic acid with the first Cas protein, the first gRNA, the first DNA polymerase, the first tag primer, the second Cas protein, the second gRNA, the second DNA polymerase, and the second tag primer, and contacting the nucleic acid molecule of interest with the third Cas protein and the third gRNA.

In certain embodiments, in step ii:
the first Cas protein binds to the first gRNA to form a first functional complex, the second Cas protein binds to the second gRNA to form a second functional complex, and the third Cas protein binds to the third gRNAs to form a third functional complex; and,
the first and second functional complexes bind and fragment the double-stranded target nucleic acid to form a target nucleic acid fragment F1, and the third functional complex binds and fragments the nucleic acid molecule of interest to form nucleotide fragments a1 and a2; and,
the first tag primer is hybridized or annealed to the 3' end of one nucleic acid strand of the target nucleic acid fragment F 1 through the first target-binding sequence; and the second tag primer is hybridized or annealed to the 3' end of the other nucleic acid strand of the target nucleic acid fragment F1 through the second target-binding sequence; and,
the first DNA polymerase and the second DNA polymerase respectively extend the target nucleic acid fragment F 1 by using the first tag primer and the second tag primer annealed to the target nucleic acid fragment F 1 as templates, to form a target nucleic acid fragment F2 having a first overhang and a second overhang; wherein, the first overhang and the second overhang are capable of being hybridized or annealed to the nucleotide fragments a1 and a2, respectively; and,
the target nucleic acid fragment F2 is hybridized or annealed with the nucleotide fragments a1 and a2 through the first overhang and the second overhang, respectively, and then inserted or linked between the nucleotide fragments a1 and a2, thereby, the target nucleic acid fragment is inserted into the nucleic acid molecule of interest.

In certain embodiments, the first overhang is capable of being hybridized or annealed to the 3' end or 3' portion of one nucleic acid strand of the nucleotide fragment a1, and the 3' end or 3' portion is formed by fragmenting the nucleic acid molecule of interest by the third functional complex.

In certain embodiments, the complementary sequence of the first tag sequence or the first overhang is capable of being hybridized or annealed to the 3' portion of one nucleic acid strand of the nucleotide fragment a1, and there is a first spacer region between the 3' portion of the nucleotide fragment a1 and the cleaved end formed by the third double-stranded target nucleic acid.

In certain embodiments, the first spacer region has a length of 1 nt to 200 nt, for example, 1-10 nt, 10-20 nt, 20-30 nt, 30-40 nt, 40-50 nt, 50-100 nt, or 100-200 nt.

In certain embodiments, the second overhang is capable of being hybridized or annealed to the 3' end or 3' portion of one nucleic acid strand of the nucleotide fragment a2, and the 3' end or 3' portion is formed by fragmenting the nucleic acid molecule of interest by the third functional complex.

In certain embodiments, the complementary sequence of the second tag sequence or the second overhang is capable of being hybridized or annealed to the 3' portion of one nucleic acid strand of the nucleotide fragment a2, and there is a second spacer region between the 3' portion of the nucleotide fragment a2 and the cleaved end formed by the third double-stranded target nucleic acid.

In certain embodiments, the second spacer region has a length of 1 nt to 200 nt, for example, 1-10 nt, 10-20 nt, 20-30 nt, 30-40 nt, 40-50 nt, 50-100 nt, or 100-200 nt.

In certain embodiments, the method is performed in a cell.

In certain embodiments, in step i, the first Cas protein or nucleic acid molecule A1, the first DNA polymerase or nucleic acid molecule B1, the first gRNA or nucleic acid molecule C1, the first tag primer or nucleic acid molecule D1, the second Cas protein or nucleic acid molecule A2, the second DNA polymerase or nucleic acid molecule B2, the second gRNA or nucleic acid molecule C2, the second tag primer or nucleic acid molecule D2, the third Cas protein or nucleic acid molecule A3 and the third gRNA or nucleic acid molecule C3 are delivered into a cell, to provide the first Cas protein, the first gRNA, the first DNA polymerase, the first tag primer, the second Cas protein, the second gRNA, the second DNA polymerase, the second tag primer, the third Cas protein, and the third gRNA in the cell.

In certain embodiments, in step i, the nucleic acid molecule A1, the nucleic acid molecule B1, the first gRNA or nucleic acid molecule C1, the first tag primer or nucleic acid molecule D1, the nucleic acid molecule A2, the nucleic acid molecule B2, the second gRNA or nucleic acid molecule C2, the second tag primer or nucleic acid molecule D2, the nucleic acid molecule A3 and the third gRNA or nucleic acid molecule C3 are delivered into a cell, to provide the first Cas protein, the first gRNA, the first DNA polymerase, the first tag primer, the second Cas protein, the second gRNA, the second DNA polymerase, the second tag primer, the third Cas protein and the third gRNA in the cell.

In certain embodiments, in step i, the nucleic acid molecules A1, B1, C1, D1, A2, B2, C2, D2, A3 and C3 are delivered into a cell to provide the first Cas protein, the first gRNA, the first DNA polymerase, the first tag primer, the second Cas protein, the second gRNA, the second DNA polymerase, the second tag primer, the third Cas protein, and the third gRNA in the cell.

In certain embodiments, in step i, the double-stranded target nucleic acid or a nucleic acid molecule T comprising the double-stranded target nucleic acid is delivered into a cell to provide the double-stranded target nucleic acid in the cell.

In certain embodiments, the double-stranded target nucleic acid or the nucleic acid molecule T comprises a first PAM sequence recognized by the first Cas protein and a second PAM sequence recognized by the second Cas protein. In certain embodiments, in step ii, the first functional complex binds to the double-stranded target nucleic acid or the nucleic acid molecule T through the first PAM sequence and the first gRNA, and fragments it; and, the second functional complex binds to the double-stranded target nucleic acid or the nucleic acid molecule T through the second PAM sequence and the second gRNA, and fragments it.

In certain embodiments, the nucleic acid molecule of interest comprises a third PAM sequence recognized by the third Cas protein; in certain embodiments, in step ii, the third functional complex binds to the nucleic acid molecule of interest through the third PAM sequence and the third gRNA, and fragments it.

In certain embodiments, the nucleic acid molecule of interest is a genomic DNA of the cell.

In certain embodiments, the first Cas protein, the first gRNA, the first DNA polymerase, or the first tag primer are as defined above.

In certain embodiments, the second Cas protein, the second gRNA, the second DNA polymerase, or the second tag primer is as defined above.

In certain embodiments, the third Cas protein and the third gRNA are as defined above.

In certain embodiments, the first, second and third Cas proteins are the same Cas protein, and the second DNA polymerase is the same as the first DNA polymerase; wherein the first Cas protein combines with the first, second and third gRNAs to form the first, second and third functional complexes, respectively, and the first DNA polymerase extends the target nucleic acid fragment F1 respectively using the first tag primer and the second tag primer annealed to the target nucleic acid fragment F1 as templates to form a target nucleic acid fragment F2 having a first overhang and a second overhang.

In certain embodiments, in step i, the first Cas protein or nucleic acid molecule A1, the first DNA polymerase or nucleic acid molecule B1, the first gRNA or nucleic acid molecule C1, the first tag primer or nucleic acid molecule D1, the second gRNA or nucleic acid molecule C2, the second tag primer or nucleic acid molecule D2 and the third gRNA or nucleic acid molecule C3 are delivered into a cell to provide the first Cas protein, the first gRNA, the first DNA polymerase, the first tag primer, the second gRNA, the second tag primer, and the third gRNA in the cell.

In certain embodiments, in step i, the nucleic acid molecule A1, the nucleic acid molecule B1, the first gRNA or nucleic acid molecule C1, the first tag primer or nucleic acid molecule D1, the second gRNA or nucleic acid molecule C2, the second tag primer or nucleic acid molecule D2 and the third gRNA or nucleic acid molecule C3 are delivered into a cell to provide the first Cas protein, the first gRNA, the first DNA polymerase, the first tag primer, the second gRNA, the second tag primer, and the third gRNA in the cell.

In certain embodiments, in step i, the nucleic acid molecules A1, B1, C1, D1, C2, D2 and C3 are delivered into a cell to provide the first Cas protein, the first gRNA, the first DNA polymerase, the first tag primer, the second gRNA, the second tag primer, and the third gRNA in the cell.

In certain embodiments, the nucleic acid molecule A1 and the nucleic acid molecule B1 are comprised in the same or different expression vectors (e.g., eukaryotic expression vectors). In certain embodiments, the nucleic acid molecule A1 and the nucleic acid molecule B1 are capable of expressing the first Cas protein and the first DNA polymerase in isolation state in a cell, or are capable of expressing a first fusion protein comprising the first Cas protein and the first DNA polymerase in a cell. In certain embodiments, in step i, a nucleic acid molecule capable of expressing the first Cas protein and the first DNA polymerase in isolation state or a nucleic acid molecule comprising a nucleotide sequence encoding the first fusion protein is delivered into a cell, and expressed in the cell to provide the first Cas protein and the first DNA polymerase in the cell.

In certain embodiments, the nucleic acid molecule C1 and the nucleic acid molecule D1 are comprised in the same expression vector (e.g., an eukaryotic expression vector). In certain embodiments, the nucleic acid molecule C1 and the nucleic acid molecule D1 are capable of transcribing a first PegRNA comprising the first gRNA and the first tag primer in a cell. In certain embodiments, in step i, the first PegRNA is delivered into a cell to provide the first gRNA and the first tag primer in the cell, or, a nucleic acid molecule comprising a nucleotide sequence encoding the first PegRNA is delivered into the cell, and the first PegRNA is transcribed in the cell to provide the first gRNA and the first tag primer in the cell.

In certain embodiments, the nucleic acid molecule C2 and the nucleic acid molecule D2 are comprised in the same expression vector (e.g., an eukaryotic expression vector). In certain embodiments, the nucleic acid molecule C2 and the nucleic acid molecule D2 are capable of transcribing a second PegRNA comprising the second gRNA and the second tag primer in a cell. In certain embodiments, in step i, the second PegRNA is delivered into the cell to provide the second gRNA and the second tag primer in the cell, or, a nucleic acid molecule comprising a nucleotide sequence encoding the second PegRNA is delivered into the cell, and the second PegRNA is transcribed in the cell to provide the second gRNA and the second tag primer in the cell;

In certain embodiments, in step i, a nucleic acid molecule capable of expressing the first Cas protein and the first DNA polymerase in isolation state or a nucleic acid molecule comprising a nucleotide sequence encoding the first fusion protein, a nucleic acid molecule comprising a nucleotide sequence encoding the first PegRNA, a nucleic acid molecule comprising a nucleotide sequence encoding the second PegRNA, and a nucleic acid molecule comprising a nucleotide sequence encoding the third gRNA are delivered into a cell, and transcribed and expressed in the cell, thereby providing the first Cas protein, the first gRNA, the first DNA polymerase, the first tag primer, the second gRNA, the second tag primer and the third gRNA in the cell.

In a tenth aspect, the present application provides a method for substituting a nucleic acid fragment in a nucleic acid molecule of interest with a target nucleotide fragment; wherein the method comprises, using the aforementioned system or kit; wherein, the first double-stranded target nucleic acid and the second double-stranded target nucleic acid are the same for providing the target nucleic acid fragment; and the third double-stranded target nucleic acid and the fourth double-stranded target nucleic acid are the same, used as the nucleic acid molecule of interest.

In certain embodiments, the method comprises:
a. by the method as described above, the first double-stranded target nucleic acid is broken into a target nucleic acid fragment F1, and overhangs are added to the two 3' ends of the target nucleic acid fragment F1 to form a target nucleic acid fragment F2 having a first overhang and a second overhang;
b. the nucleic acid molecule of interest is fragmented with the third and fourth functional complexes to form nucleotide fragments a1, a2 and a3; wherein, prior to fragmentation, in the nucleic acid molecule of interest, the nucleotide fragments a1, a2 and a3 are arranged in sequence (i.e., the nucleotide fragment a1 is linked to the nucleotide fragment a3 through the nucleotide fragment a2); and,
c. the nucleotide fragments a1 and a3 are ligated to the target nucleic acid fragment F2, thereby substituting the nucleotide fragment a2 in the nucleic acid molecule of interest with the target nucleic acid fragment.

In certain embodiments, the method comprises the steps of:
i. providing the double-stranded target nucleic acid and a nucleic acid molecule of interest; and providing the first Cas protein, the first gRNA, the first DNA polymerase, the first tag primer, the second Cas protein, the second gRNA, the second DNA polymerase, the second tag primer, the third Cas protein, the third gRNA, the fourth Cas protein and the fourth gRNA;
ii. contacting the double-stranded target nucleic acid with the first Cas protein, the first gRNA, the first DNA polymerase, the first tag primer, the second Cas protein, the second gRNA, the second DNA polymerase, and the second tag primer, and contacting the nucleic acid molecule of interest with the third Cas protein, the third gRNA, the fourth Cas protein, and the fourth gRNA.

In certain embodiments, in step ii:
the first Cas protein binds to the first gRNA to form a first functional complex, the second Cas protein binds to the second gRNA to form a second functional complex, the third Cas protein binds to the third gRNA to form a third functional complex, and the fourth Cas protein binds to the fourth gRNA to form a fourth functional complex; and,
the first and second functional complexes bind and fragment the double-stranded target nucleic acid to form target nucleic acid fragment F1, and the third and fourth functional complexes bind and fragment the nucleic acid molecule of interest to form fragmented nucleotide fragments a1, a2, and a3; and,
the first tag primer is hybridized or annealed to the 3' end of one nucleic acid strand of the target nucleic acid fragment F1 through the first target-binding sequence; and the second tag primer is hybridized or annealed to the 3' end of the other nucleic acid strand of the target nucleic acid fragment F1 through the second target-binding sequence; and,
the first DNA polymerase and the second DNA polymerase extend the target nucleic acid fragment F1 respectively using the first tag primer and the second tag primer annealed to the target nucleic acid fragment F1 as templates to form a target nucleic acid fragment F2 having a first overhang and a second overhang; wherein the first overhang and the second overhang are capable of being hybridized or annealed to the nucleotide fragments a1 and a3, respectively; and,
the target nucleic acid fragment F2 is hybridized or annealed with the nucleotide fragments a1 and a3 through the first overhang and the second overhang, respectively, and is then linked between the nucleotide fragments a1 and a3, thereby, the nucleotide fragment a2 in the nucleic acid molecule of interest is substituted with the target nucleic acid fragment.

In certain embodiments, the first overhang is capable of being hybridized or annealed to the 3' end or 3' portion of one nucleic acid strand of the nucleotide fragment a1, and the 3' end or 3' portion is formed by fragmenting the nucleic acid molecule of interest by the third functional complex;

In certain embodiments, the second overhang is capable of being hybridized or annealed to the 3' end or 3' portion of one nucleic acid strand of the nucleotide fragment a3, and the 3' end or 3' portion is formed by fragmenting the nucleic acid molecule of interest by the fourth functional complex.

In certain embodiments, the method is performed in a cell.

In certain embodiments, in step i, the first Cas protein or nucleic acid molecule A1, the first DNA polymerase or nucleic acid molecule B1, the first gRNA or nucleic acid molecule C1, the first tag primer or nucleic acid molecule D1, the second Cas protein or nucleic acid molecule A2, the second DNA polymerase or nucleic acid molecule B2, the second gRNA or nucleic acid molecule C2, the second tag primer or nucleic acid molecule D2, the third Cas protein or nucleic acid molecule A3, the third gRNA or nucleic acid molecule C3, the fourth Cas protein or nucleic acid molecule A4, the fourth gRNA or nucleic acid molecule C4 are delivered into a cell, to provide the first Cas protein, the first gRNA, the first DNA polymerase, the first tag primer, the second Cas protein, the second gRNA, the second DNA polymerase, the second tag primer, the third Cas protein, the third gRNA, the fourth Cas protein and the fourth gRNA in the cell.

In certain embodiments, in step i, the nucleic acid molecule A1, the nucleic acid molecule B1, the first gRNA or nucleic acid molecule C1, the first tag primer or nucleic acid molecule D1, the nucleic acid molecule A2, the nucleic acid molecule B2, the second gRNA or nucleic acid molecule C2, the second tag primer or nucleic acid molecule D2, the nucleic acid molecule A3, the third gRNA or nucleic acid molecule C3, the nucleic acid molecule A4 and the fourth gRNA or nucleic acid molecule C4 are delivered into a cell, to provide the first Cas protein, the first gRNA, the first DNA polymerase, the first tag primer, the second Cas protein, the second gRNA, the second DNA polymerase, the second tag primer, the third Cas protein, the third gRNA, the fourth Cas protein, and the fourth gRNA in the cell.

In certain embodiments, in step i, the nucleic acid molecules A1, B1, C1, D1, A2, B2, C2, D2, A3, C3, A4, and C4 are delivered into a cell, to provide the first Cas protein, the first gRNA, the first DNA polymerase, the first tag primer, the second Cas protein, the second gRNA, the second DNA polymerase, the second tag primer, the third Cas protein, the third gRNA, the fourth Cas protein and the fourth gRNA in the cell.

In certain embodiments, in step i, the double-stranded target nucleic acid or a nucleic acid molecule T comprising the double-stranded target nucleic acid is delivered into a cell, to provide the double-stranded target nucleic acid in the cell.

In certain embodiments, the double-stranded target nucleic acid or nucleic acid molecule T comprises a first PAM sequence recognized by the first Cas protein and a second PAM sequence recognized by the second Cas protein. In certain embodiments, in step ii, the first functional complex binds to the double-stranded target nucleic acid or nucleic acid molecule T through the first PAM sequence and the first gRNA, and fragments it; and, the second functional complex binds to the double-stranded target nucleic acid or nucleic acid molecule T through the second PAM sequence and the second gRNA, and fragments it.

In certain embodiments, the nucleic acid molecule of interest comprises a third PAM sequence recognized by the third Cas protein and a fourth PAM sequence recognized by the fourth Cas protein. In certain embodiments, in step ii, the third functional complex binds to the nucleic acid molecule of interest through the third PAM sequence and the third gRNA, and fragments it; and, the fourth functional complex binds to the nucleic acid molecule of interest through the fourth PAM sequence and the fourth gRNA, and fragments it.

In certain embodiments, the nucleic acid molecule of interest is a genomic DNA of the cell.

In certain embodiments, the first Cas protein, the first gRNA, the first DNA polymerase, or the first tag primer are as defined above.

In certain embodiments, the second Cas protein, the second gRNA, the second DNA polymerase, or the second tag primer is as defined above.

In certain embodiments, the third Cas protein and the third gRNA are as defined above.

In certain embodiments, the fourth Cas protein and the fourth gRNA are as defined above.

In certain embodiments, the first, second, third and fourth Cas proteins are the same Cas protein, and the second DNA polymerase is the same as the first DNA polymerase; wherein the first Cas protein combines with the first, second, third and fourth gRNAs to form the first, second, third and fourth functional complexes, respectively, and the first DNA polymerase extends the target nucleic acid fragment F1 respectively using the first tag primer and the second tag primer annealed to the target nucleic acid fragment F1 as templates, to form a target nucleic acid fragment F2 having a first overhang and a second overhang.

In certain embodiments, in step i, the first Cas protein or nucleic acid molecule A1, the first DNA polymerase or nucleic acid molecule B1, the first gRNA or nucleic acid molecule C1, the first tag primer or nucleic acid molecule D1, the second gRNA or nucleic acid molecule C2, the second tag primer or nucleic acid molecule D2, the third gRNA or nucleic acid molecule C3 and the fourth gRNA or nucleic acid molecule C4 are delivered into a cell, to provide the first Cas protein, the first gRNA, the first DNA polymerase, the first tag primer, the second gRNA, the second tag primer, the third gRNA, and the fourth gRNA in the cell.

In certain embodiments, in step i, the nucleic acid molecule A1, the nucleic acid molecule B1, the first gRNA or nucleic acid molecule C1, the first tag primer or nucleic acid molecule D1, the second gRNA or nucleic acid molecule C2, the second tag primer or nucleic acid molecule D2, the third gRNA or nucleic acid molecule C3 and the fourth gRNA or nucleic acid molecule C4 are delivered into a cell, to provide the first Cas protein, the first gRNA, the first DNA polymerase, the first tag primer, the second gRNA, the second tag primer, the third gRNA, and the fourth gRNA in the cell.

In certain embodiments, in step i, the nucleic acid molecules A1, B1, C1, D1, C2, D2, C3 and C4 are delivered into a cell, to provide the first Cas protein, the first gRNA, the first DNA polymerase, the first tag primer, the second gRNA, the second tag primer, the third gRNA, and the fourth gRNA in the cell.

In certain embodiments, the nucleic acid molecule A1 and nucleic acid molecule B1 are comprised in the same or different expression vectors (e.g., eukaryotic expression vectors). In certain embodiments, the nucleic acid molecule A1 and the nucleic acid molecule B1 are capable of expressing the first Cas protein and the first DNA polymerase in isolation state in a cell, or are capable of expressing a first fusion protein comprising the first Cas protein and the first DNA polymerase; in certain embodiments, in step i, a nucleic acid molecule capable of expressing the first Cas protein and the first DNA polymerase in isolation state or a nucleic acid molecule comprising a nucleotide sequence encoding the first DNA polymerase is delivered into a cell, and expressed in the cell to provide the first Cas protein and the first DNA polymerase in the cell.

In certain embodiments, the nucleic acid molecule C1 and the nucleic acid molecule D 1 are comprised in the same expression vector (e.g., an eukaryotic expression vector). In certain embodiments, the nucleic acid molecule C 1 and the nucleic acid molecule D1 are capable of transcribing a first PegRNA comprising the first gRNA and the first tag primer in a cell. In certain embodiments, in step i, the first PegRNA is delivered into a cell to provide the first gRNA and the first tag primer in the cell, or, a nucleic acid molecule comprising a nucleotide sequence encoding the first PegRNA is delivered into the cell, and the first PegRNA is transcribed in the cell to provide the first gRNA and the first tag primer in the cell.

In certain embodiments, the nucleic acid molecule C2 and the nucleic acid molecule D2 are comprised in the same expression vector (e.g., an eukaryotic expression vector). In certain embodiments, the nucleic acid molecule C2 and the nucleic acid molecule D2 are capable of transcribing a second PegRNA comprising the second gRNA and the second tag primer in a cell. In certain embodiments, in step i, the second PegRNA is delivered into the cell to provide the second gRNA and the second tag primer in the cell, or, a nucleic acid molecule comprising a nucleotide sequence encoding the second PegRNA is delivered into the cell, and the second PegRNA is transcribed in the cell to provide the second gRNA and the second tag primer in the cell.

In certain embodiments, in step i, a nucleic acid molecule capable of expressing the first Cas protein and the first DNA polymerase in isolation state or a nucleic acid molecule comprising a nucleotide sequence encoding the first fusion protein, a nucleic acid molecule comprising a nucleotide sequence encoding the first PegRNA, a nucleic acid molecule comprising a nucleotide sequence encoding the second PegRNA, a nucleic acid molecule comprising a nucleotide sequence encoding the third gRNA, and a nucleic acid molecule comprising a nucleotide sequence encoding the fourth gRNA are delivered into a cell, and is transcribed and expressed in the cell, thereby providing the first Cas protein, the first gRNA, the first DNA polymerase, the first tag primer, the second gRNA, the second tag primer, the third gRNA, and the fourth gRNA in the cell.

In certain embodiments, the method comprises the steps of:
i. providing the double-stranded target nucleic acid and a nucleic acid molecule of interest; and providing the first, second, third and fourth Cas proteins, the first, second, third and fourth gRNAs, the first, second, third and fourth DNA polymerases, and the first, second, third and fourth tag primers;
ii. contacting the double-stranded target nucleic acid with the first and second Cas proteins, the first and second gRNAs, the first and second DNA polymerases, and the first and second tag primers, and contacting the nucleic acid molecule of interest with the third and fourth Cas proteins, the third and fourth gRNAs, the third and fourth DNA polymerases, and the third and fourth tag primers.

In certain embodiments, in step ii:
the first Cas protein binds to the first gRNA to form a first functional complex, the second Cas protein binds to the second gRNA to form a second functional complex, the third Cas protein binds to the third gRNA to form a third functional complex, and the fourth Cas protein binds to the fourth gRNA to form a fourth functional complex; and,
the first and second functional complexes bind and fragment the double-stranded target nucleic acid to form a target nucleic acid fragment F 1, and the third and fourth functional complexes bind and fragment the nucleic acid molecule of interest to form fragmented nucleotide fragments a1, a2, and a3; and,
the first tag primer is hybridized or annealed to the 3' end of one nucleic acid strand of the target nucleic acid fragment F 1 through the first target-binding sequence; and the second tag primer is hybridized or annealed to the 3' end of the other nucleic acid strand of the target nucleic acid fragment F1 through the second target-binding sequence; and,
the first DNA polymerase and the second DNA polymerase extend the target nucleic acid fragment F 1 respectively using the first tag primer and the second tag primer annealed to the target nucleic acid fragment F1 as templates to form a target nucleic acid fragment F2 having a first overhang and a second overhang; wherein the first overhang and the second overhang are capable of being hybridized or annealed to the nucleotide fragments a1 and a3, respectively; and,
the third tag primer is hybridized or annealed to the 3' end of one nucleic acid strand of the nucleotide fragment a1 through the third target-binding sequence, wherein the 3' end is formed by fragmenting the nucleic acid molecule of interest by the third functional complex; and, the fourth tag primer is hybridized or annealed to the 3' end of the other nucleic acid strand of the nucleotide fragment a3 through the fourth target-binding sequence, wherein the 3' end is formed by fragmenting the nucleic acid molecule of interest by the fourth functional complex; and,
the third DNA polymerase extends the nucleotide fragment a1 using the third tag primer annealed to the nucleotide fragment a1 as a template to form a nucleotide fragment a1 with a third overhang; and, the fourth DNA polymerase extends the nucleotide fragment a3 using the fourth tag primer annealed to the nucleotide fragment a3 as a template to form a nucleotide fragment a3 with a fourth overhang; wherein the third overhang and the fourth overhang are capable of being hybridized or annealed to the target nucleic acid fragment F2, respectively; and,
through the first, second, third and fourth overhangs, the target nucleic acid fragment F2 is hybridized or annealed to the nucleotide fragments a1 and a3, respectively, and then ligated between the nucleotide fragments a1 and a3, thereby the nucleotide fragment a2 in the nucleic acid molecule of interest is substituted with the target nucleic acid fragment.

In certain embodiments, the first overhang is capable of being hybridized or annealed to the 3' end or 3' portion of one nucleic acid strand of the nucleotide fragment a1, and the 3' end or 3' portion is formed by fragmenting the nucleic acid molecule of interest by the third functional complex. In certain embodiments, the first overhang is capable of being hybridized or annealed to the third overhang of the nucleotide fragment a1 or its upstream nucleotide sequence.

In certain embodiments, the second overhang is capable of being hybridized or annealed to the 3' end or 3' portion of one nucleic acid strand of the nucleotide fragment a3, and the 3' end or 3' portion is formed by fragmenting the nucleic acid molecule of interest by the fourth functional complex. In certain embodiments, the second overhang is capable of being hybridized or annealed to the fourth overhang of the nucleotide fragment a3 or its upstream nucleotide sequence.

In certain embodiments, the third overhang is capable of being hybridized or annealed to the first overhang or its upstream nucleotide sequence.

In certain embodiments, the fourth overhang is capable of being hybridized or annealed to the second overhang or its upstream nucleotide sequence.

In certain embodiments, the method is performed in a cell.

In certain embodiments, in step i, the first Cas protein or nucleic acid molecule A1, the first DNA polymerase or nucleic acid molecule B1, the first gRNA or nucleic acid molecule C1, the first tag primer or nucleic acid molecule D1, the second Cas protein or nucleic acid molecule A2, the second DNA polymerase or nucleic acid molecule B2, the second gRNA or nucleic acid molecule C2, the second tag primer or nucleic acid molecule D2, the third Cas protein or nucleic acid molecule A3, the third DNA polymerase or nucleic acid molecule B3, the third gRNA or nucleic acid molecule C3, the third label primer or nucleic acid molecule D3, the fourth Cas Protein or nucleic acid molecule A4, the fourth DNA polymerase or nucleic acid molecule B4, the fourth gRNA or nucleic acid molecule C4 and the fourth tag primer or nucleic acid molecule D4 are delivered into a cell to provide the first, second, third and fourth Cas proteins, the first, the second, third and fourth gRNAs, the first, second, third and fourth DNA polymerases, and the first, second, third and fourth tag primers in the cell.

In certain embodiments, in step i, the nucleic acid molecule A1, the nucleic acid molecule B1, the first gRNA or nucleic acid molecule C1, the first tag primer or nucleic acid molecule D1, the nucleic acid molecule A2, the nucleic acid molecule B2, the second gRNA or nucleic acid molecule C2, the second tag primer or nucleic acid molecule D2, the nucleic acid molecule A3, the nucleic acid molecule B3, the third gRNA or nucleic acid molecule C3, the third tag primer or nucleic acid molecule D3, the nucleic acid molecule A4, the nucleic acid molecule B4, the fourth gRNA or nucleic acid molecule C4 and the fourth tag primer or nucleic acid molecule D4 are delivered into a cell, to provide the first, second, third and fourth Cas proteins, the first, second, third and fourth gRNAs, the first, second, third and fourth DNA polymerases, and the first, second, third and fourth tag primers in the cell.

In certain embodiments, in step i, the nucleic acid molecules A1, B1, C1, D1, A2, B2, C2, D2, A3, B3, C3, D3, A4, B4, C4, D4 are delivered into in a cell to provide the first, second, third and fourth Cas proteins, the first, second, third and fourth gRNAs, the first, second, third and fourth DNA polymerases, and the first, second, third and fourth tag primers in the cell.

In certain embodiments, in step i, the double-stranded target nucleic acid or a nucleic acid molecule T comprising the double-stranded target nucleic acid is delivered into a cell to provide the double-stranded target nucleic acid in the cell.

In certain embodiments, the double-stranded target nucleic acid or the nucleic acid molecule T comprises a first PAM sequence recognized by the first Cas protein and a second PAM sequence recognized by the second Cas protein; in certain embodiments, in step ii, the first functional complex binds to the double-stranded target nucleic acid or nucleic acid molecule T through the first PAM sequence and the first gRNA, and fragments it; and the second functional complex binds to the double-stranded target nucleic acid or nucleic acid molecule T through the second PAM sequence and the second gRNA, and fragments it.

In certain embodiments, the nucleic acid molecule of interest comprises a third PAM sequence recognized by the third Cas protein and a fourth PAM sequence recognized by the fourth Cas protein. In certain embodiments, in step ii, the third functional complex binds to the nucleic acid molecule of interest through the third PAM sequence and the third gRNA, and fragments it. And, the fourth functional complex binds to the nucleic acid molecule of interest through the fourth PAM sequence and the fourth gRNA, and fragments it.

In certain embodiments, the nucleic acid molecule of interest is a genomic DNA of the cell.

In certain embodiments, the first Cas protein, the first gRNA, the first DNA polymerase, or the first tag primer is as defined above.

In certain embodiments, the second Cas protein, the second gRNA, the second DNA polymerase, or the second tag primer is as defined above.

In certain embodiments, the third Cas protein, the third gRNA, the third DNA polymerase, or the third tag primer is as defined above.

In certain embodiments, the fourth Cas protein, the fourth gRNA, the fourth DNA polymerase, or the fourth tag primer is as defined above.

In certain embodiments, the first, second, third and fourth Cas proteins are the same Cas protein, and the first, second, third and fourth DNA polymerases are the same DNA polymerase; wherein, the first Cas protein combines with the first, second, third and fourth gRNAs respectively to form the first, second, third and fourth functional complexes; and, the first DNA polymerase extends the target nucleic acid fragment F1 respectively using the first tag primer and the second tag primer annealed to the target nucleic acid fragment F1 as templates, to form a target nucleic acid fragment F2 having a first overhang and a second overhang; and, the first DNA polymerase extends the nucleotide fragments a1 and a3 using the third tag primer and the fourth tag primer as templates to form third and fourth overhangs.

In certain embodiments, in step i, the first Cas protein or nucleic acid molecule A1, the first DNA polymerase or nucleic acid molecule B1, the first gRNA or nucleic acid molecule C1, the first tag primer or nucleic acid molecule D1, the second gRNA or nucleic acid molecule C2, the second tag primer or nucleic acid molecule D2, the third gRNA or nucleic acid molecule C3, the third tag primer or nucleic acid molecule D3, the fourth gRNA or nucleic acid molecule C4 and the fourth tag primer or nucleic acid molecule D4 are delivered into a cell, to provide the first Cas protein, the first DNA polymerase, the first, second, third and fourth gRNAs, and the first, second, third and fourth tag primers in the cell.

In certain embodiments, in step i, the nucleic acid molecule A1, the nucleic acid molecule B1, the first gRNA or nucleic acid molecule C1, the first tag primer or nucleic acid molecule D1, the second gRNA or nucleic acid molecule C2, the second tag primer or nucleic acid molecule D2, the third gRNA or nucleic acid molecule C3, the third tag primer or nucleic acid molecule D3, the fourth gRNA or nucleic acid molecule C4, and the fourth tag primer or nucleic acid molecule D4 are delivered into a cell, to provide the first Cas protein, the first DNA polymerase, the first, second, third and fourth gRNAs, and the first, second, third and fourth tag primers in the cell.

In certain embodiments, in step i, the nucleic acid molecules A1, B1, C1, D1, C2, D2, C3, D3, C4 and D4 are delivered into a cell to provide the first Cas protein, the first DNA polymerase, the first, second, third and fourth gRNAs, and the first, second, third and fourth tag primers in the cell.

In certain embodiments, the nucleic acid molecule A1 and the nucleic acid molecule B1 are comprised in the same or different expression vectors (e.g., eukaryotic expression vectors). In certain embodiments, the nucleic acid molecule A1 and the nucleic acid molecule B1 are capable of expressing the first Cas protein and the first DNA polymerase in isolation state in a cell, or are capable of expressing a first fusion protein comprising the first Cas protein and the first DNA polymerase. In certain embodiments, in step i, a nucleic acid molecule capable of expressing the first Cas protein and the first DNA polymerase in isolation state or a nucleic acid molecule comprising a nucleotide sequence encoding the first fusion protein is delivered into a cell, and expressed in the cell to provide the first Cas protein and the first DNA polymerase in the cell.

In certain embodiments, the nucleic acid molecule C1 and the nucleic acid molecule D 1 are comprised in the same expression vector (e.g., an eukaryotic expression vector). In certain embodiments, the nucleic acid molecule C 1 and the nucleic acid molecule D1 are capable of transcribing a first PegRNA comprising the first gRNA and the first tag primer in a cell. In certain embodiments, in step i, the first PegRNA is delivered into a cell to provide the first gRNA and the first tag primer in the cell, or, a nucleic acid molecule comprising a nucleotide sequence encoding the first PegRNA is delivered into the cell, and the first PegRNA is transcribed in the cell to provide the first gRNA and the first tag primer in the cell.

In certain embodiments, the nucleic acid molecule C2 and the nucleic acid molecule D2 are comprised in the same expression vector (e.g., an eukaryotic expression vector). In certain embodiments, the nucleic acid molecule C2 and the nucleic acid molecule D2 are capable of transcribing a second PegRNA comprising the second gRNA and the second tag primer in a cell. In certain embodiments, in step i, the second PegRNA is delivered into the cell to provide the second gRNA and the second tag primer in the cell, or, a nucleic acid molecule comprising a nucleotide sequence encoding the second PegRNA is delivered into the cell, and the second PegRNA is transcribed in the cell to provide the second gRNA and the second tag primer in the cell.

In certain embodiments, the nucleic acid molecule C3 and the nucleic acid molecule D3 are comprised in the same expression vector (e.g., an eukaryotic expression vector). In certain embodiments, the nucleic acid molecule C3 and the nucleic acid molecule D3 are capable of transcribing a third PegRNA comprising the third gRNA and the third tag primer in a cell. In certain embodiments, in step i, the third PegRNA is delivered into the cell to provide the third gRNA and the third tag primer in the cell, or, a nucleic acid molecule comprising a nucleotide sequence encoding the third PegRNA is delivered into the cell, and the third PegRNA is transcribed in the cell to provide the third gRNA and the third tag primer in the cell.

In certain embodiments, the nucleic acid molecule C4 and the nucleic acid molecule D4 are comprised in the same expression vector (e.g., an eukaryotic expression vector). In certain embodiments, the nucleic acid molecule C4 and the nucleic acid molecule D4 are capable of transcribing a fourth PegRNA comprising the fourth gRNA and the fourth tag primer in a cell. In certain embodiments, in step i, the fourth PegRNA is delivered into the cell to provide the fourth gRNA and the fourth tag primer in the cell, or, a nucleic acid molecule comprising a nucleotide sequence encoding the fourth PegRNA is delivered into the cell, and the fourth PegRNA is transcribed in the cell to provide the fourth gRNA and the fourth tag primer in the cell.

In certain embodiments, in step i, a nucleic acid molecule capable of expressing the first Cas protein and the first DNA polymerase in isolation state or a nucleic acid molecule comprising a nucleotide sequence encoding the first fusion protein, a nucleic acid molecule comprising a nucleotide sequence encoding the first PegRNA, a nucleic acid molecule comprising a nucleotide sequence encoding the second PegRNA, a nucleic acid molecule comprising a nucleotide sequence encoding the third PegRNA, and a nucleic acid molecule comprising a nucleotide sequence encoding the fourth PegRNA are delivered into a cell, and the first fusion protein is expressed and the first, second, third, and fourth PegRNAs are transcribed in the cell, thereby providing the first Cas protein, the first DNA polymerase, the first, second, third and fourth gRNAs, and the first, second, third and fourth tag primers in the cell.

### Beneficial effects of invention

Compared with the prior art, the nucleic acid editing system, kit and method provided by the present application can fragment a double-stranded nucleic acid and extend/add one or two overhangs of any base sequence at its end (3' end). On this basis, the system, kit and method of the present application can achieve efficient and precise insertion and substitution of exogenous nucleic acids (especially large fragments of exogenous nucleic acids).

The embodiments of the present invention will be described in detail below with reference to the drawings and examples, but those skilled in the art will understand that the following drawings and examples are only used to illustrate the present invention, rather than limit the scope of the present invention. Various objects and advantageous aspects of the present invention will become apparent to those skilled in the art from the accompanying drawings and the following detailed description of the preferred embodiments.

### Brief Description of the Drawings

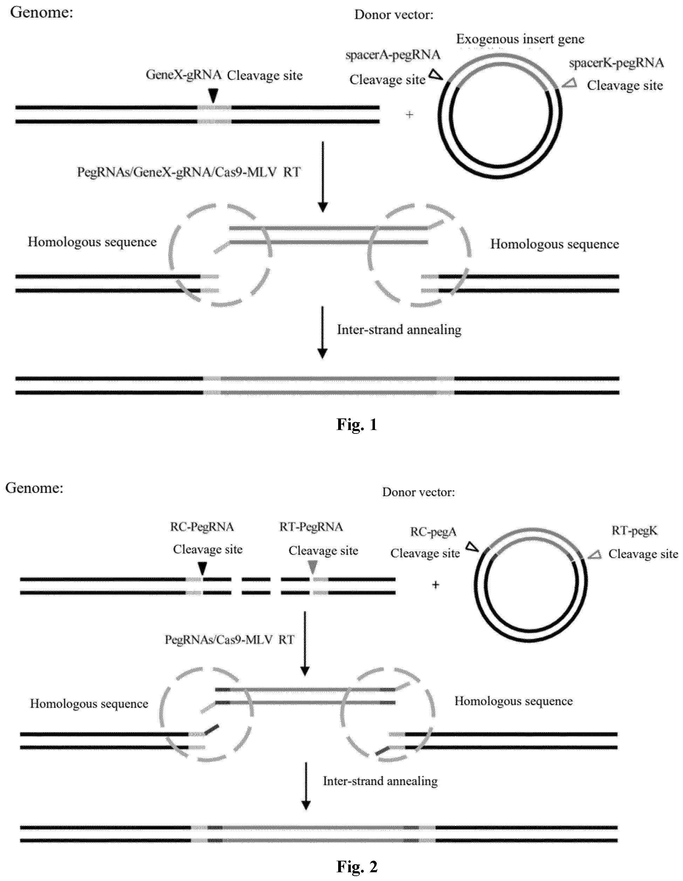

Fig. 1 shows a schematic diagram of the principle by which the method of the present invention mediates the insertion of exogenous genes into the genome. Among them, the black double solid line represents the genome sequence or the backbone sequence of the donor vector; the blue double solid line represents the exogenous gene to be inserted; the orange and green single solid lines in the gray dotted circle represent the extended first overhang and second overhang, the orange and green double solid lines in the gray dotted circle represent the first homologous sequence and second homologous sequence that are complementary to the first overhang and second overhang at the genome cleaved ends, respectively; the black solid triangle indicates the genome specific site spacerX, which can be recognized and cleaved by Cas9-MLV-RT/GeneX-gRNA (a complex formed by Cas9 protein, reverse transcriptase (MLV-RT) and GeneX-gRNA); the black hollow triangle indicates the sapcerA site at the upstream of the exogenous gene on the donor vector, which can be recognized and cleaved by Cas9-MLV-RT/spacerA-pegRNA (a complex formed by Cas9 protein, reverse transcriptase (MLV-RT) and spacerA-pegRNA); the gray hollow triangle indicates the sapcerK site at downstream of the exogenous gene on the donor vector, which can be recognized and cleaved by Cas9-MLV-RT/spacerK-pegRNA (a complex formed by Cas9 protein, reverse transcriptase (MLV-RT) and spacerK-pegRNA); and, spacerA and sapcerK are located at nucleic acid strands opposite to each other.
   The sapcerA site is recognized and cleaved by Cas9-MLV-RT/spacerA-pegRNA, wherein the spacerA-pegRNA comprises a first target-binding sequence and a first tag sequence (which is complementary to one strand of the first homologous sequence), the first target-binding sequence hybridizes to the 3' end of one nucleic acid strand of the cleaved target nucleic acid fragment to form a double-stranded structure, and the first tag sequence does not bind to the target nucleic acid fragment, and is in a free single-stranded state. Thus, the reverse transcriptase (MLV-RT) can use the first tag primer as a template to extend the 3' end of the nucleic acid strand, thereby forming a first overhang (i.e., single solid orange line, which can be, for example, 35nt in length). Similarly, the spacerK site is recognized and cleaved by Cas9-MLV-RT/spacerK-pegRNA, wherein the spacerK-pegRNA comprises a second target-binding sequence and a second tag sequence (which is complementary to one strand of the second homologous sequence), the second target-binding sequence hybridizes to the 3' end of one nucleic acid strand of the cleaved target nucleic acid fragment to form a double-stranded structure, and the second tag sequence does not bind to the target nucleic acid fragment and is in a free single-stranded state. Thus, the reverse transcriptase (MLV-RT) can use the second tag primer as a template to extend the 3' end of the nucleic acid strand, thereby forming a second overhang (i.e., single solid green line, which can be, for example, 35nt in length). By double cleavage, the exogenous gene fragment is cleaved out from the vector, and the first overhang and the second overhang are added at both ends of the exogenous gene fragment.
   In addition, the spacerX site is recognized and cleaved by Cas9-MLV-RT/GeneX-gRNA to form a fragmented genome; and the two ends at the fragmentation site comprise a first homologous sequence (complementary to the first overhang) and a second homologous sequence (complementary to the second overhang), respectively. Thus, the exogenous gene fragment with the first overhang and the second overhang can be integrated into the fragmentation site of the genome through inter-strand annealing, thereby achieving the site-directed insertion of the exogenous gene.
Fig. 2 shows a schematic diagram of the principle by which the method of the present invention mediates the substitution of specific nucleotide fragments of the genome with exogenous genes. Among them, the black double solid line represents the genome sequence or the backbone sequence of the donor vector; the black double dotted line represents the genomic fragment to be substituted; the blue double solid line represents the exogenous insertion gene for the substitution; the solid lines with same color in the gray dotted circle represent homologous sequences that are complementary between each other, the orange single solid line represents the extended first overhang, the green single solid line represents the extended second overhang, the red single solid line represents the extended third overhang, the purple single solid line represents the extended fourth overhang; the black solid triangle indicates the cleavage site RC-PegRNA at the upstream of the fragment to be substituted on the genome, which can be recognized, cleaved and extended by Cas9-MLV-RT/RC-PegRNA (a complex formed with Cas9 protein, reverse transcriptase (MLV-RT)) and RC-PegRNA); the gray solid triangle indicates the cleavage site RT-PegRNA at downstream of the fragment to be substituted on the genome, which can be recognized, cleaved and extended by Cas9-MLV-RT/RT-PegRNA (a complex formed with Cas9 protein, reverse transcriptase (MLV-RT) and RT-PegRNA); the black hollow triangle represents the cleavage site RC-pegA at the upstream of the exogenous insert on the donor vector, which can be recognized, cleaved and extended by Cas9-MLV-RT/RC-pegA; the gray hollow triangle represents the cleavage site RT-pegK at downstream of the exogenous insert on the donor vector, which can be recognized, cleaved and extended by Cas9-MLV-RT/RT-pegK; the RC-pegA and RT-pegK sites are located on nucleic acid strands opposite to each other; and the RC-PegRNA and RT-PegRNA sites are located on nucleic acid strands opposite to each other.
   The site RC-pegA is recognized and cleaved by Cas9-MLV-RT/RC-PegRNA, wherein the RC-PegRNA comprises a first target-binding sequence and a first tag sequence (which is complementary to one strand of the first homologous sequence), the first target-binding sequence hybridizes to the 3' end of one nucleic acid strand of the cleaved target nucleic acid fragment to form a double-stranded structure, and the first tag sequence does not bind to the target nucleic acid fragment and is in a free single-stranded state. Therefore, the reverse transcriptase (MLV-RT) can use the first tag primer as a template to extend the 3' end of the nucleic acid strand to form a first overhang (i.e., the orange single solid line), and the first overhang can be complementary to the nucleotide sequence (the orange double solid line) at the upstream of the RC-PegRNA site.
   Similarly, the site RT-pegK is recognized and cleaved by Cas9-MLV-RT/RT-pegK, wherein RT-pegK comprises a second target-binding sequence and a second tag sequence (which is complementary to one strand of the second homologous sequence), the second target-binding sequence hybridizes to the 3' end of one nucleic acid strand of the cleaved target nucleic acid fragment to form a double-stranded structure, and the second tag sequence does not bind to the target nucleic acid fragment and is in a free single-stranded state. Therefore, the reverse transcriptase (MLV-RT) can use the second tag primer as a template to extend the 3' end of the nucleic acid strand to form a second overhang (i.e., the green single solid line), and the second overhang can be complementary to the nucleotide sequence (the green double solid line) at downstream of the RT-PegRNA site.
   By double cleavage, the exogenous gene fragment is cleaved out from the vector, and added at both ends with a first overhang and a second overhang.
   The site RC-PegRNA is recognized and cleaved by Cas9-MLV-RT/RC-PegRNA, wherein the RC-PegRNA comprises a third target-binding sequence and a third tag sequence (which is complementary to one strand of the third homologous sequence), the third target-binding sequence hybridizes to the 3' end of one nucleic acid strand of the cleaved target nucleic acid fragment to form a double-stranded structure, and the third tag sequence does not bind to the target nucleic acid fragment and is in a free single-stranded state. Therefore, the reverse transcriptase (MLV-RT) can use the third tag primer as a template to extend the 3' end of the nucleic acid strand, thereby forming a third overhang (i.e., the red single solid line), and the third overhang can be complementary to the nucleotide sequence (the red double solid line) at downstream of the RC-pegA site.
   The site RT-PegRNA is recognized and cleaved by Cas9-MLV-RT/RT-PegRNA, wherein the RT-PegRNA comprises a fourth target-binding sequence and a fourth tag sequence (which is complementary to one strand of the fourth homologous sequence), and the fourth target-binding sequence hybridizes to the 3' end of one nucleic acid strand of the cleaved target nucleic acid fragment to form a double-stranded structure, and the fourth tag sequence does not bind to the target nucleic acid fragment and is in a free single-stranded state. Therefore, the reverse transcriptase (MLV-RT) can use the fourth tag primer as a template to extend the 3' end of the nucleic acid strand, thereby forming a fourth overhang (i.e., the purple single solid line), and the fourth overhang can be complementary to the nucleotide sequence (purple double solid line) at the upstream of the RT-pegK site.
   By double cleavage, the fragment to be substituted is cleaved from the genome, and the third and fourth overhangs are added to both ends of the fragmented genome.
   Thus, the exogenous gene fragment with the first and second overhangs can be inserted into the fragmented genome with the third and fourth overhangs through inter-strand annealing, thereby realizing the substitution of specific nucleotide fragment on the genome.
Fig. 3 shows a schematic diagram of the process of site-directed knock-in of exogenous genes (IRES-EGFP) in the GAPDH gene of the human cell genome.

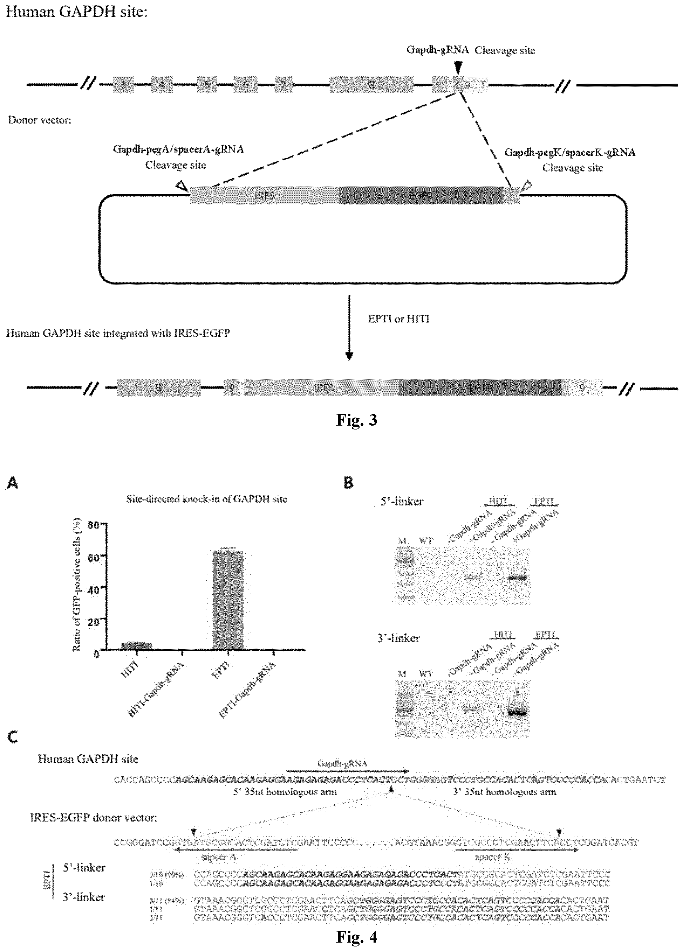

Fig. 4 shows a schematic diagram of the results of site-directed knock-in of exogenous gene (IRES-EGFP) in the GAPDH gene of the human cell genome using the method of the present invention. Fig. 4A shows the ratio of EGFP-positive cells generated by different methods, as determined by flow cytometry fluorescence sorting (FACS). Fig. 4B shows the results of PCR identification of the nucleotide sequence at the junction between the reporter gene IRES-EGFP (5' end and 3' end) and the genomic DNA. Fig. 4C shows the results of Sanger sequencing of the nucleotide sequence at the junction between the reporter gene IRES-EGFP (5' end and 3' end) and the genomic DNA.
Fig. 5 shows the schematic diagram of the site-directed knock-in of exogenous gene (IRES-EGFP) in the ACTB gene of human cell genome.

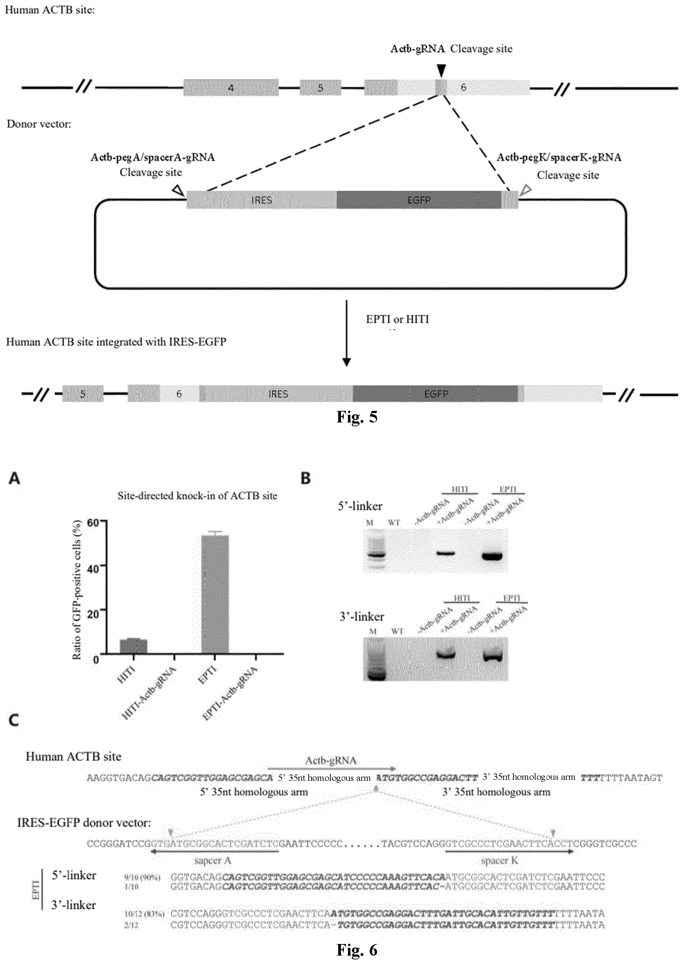

Fig. 6 shows a schematic diagram of the results of site-directed knock-in of an exogenous gene (IRES-EGFP) in the ACTB gene of the human cell genome using the method of the present invention. Fig. 6A shows the ratio of EGFP-positive cells generated by different methods, as determined by flow cytometry fluorescence sorting (FACS). Fig. 6B shows the results of PCR identification of the nucleotide sequence at the junction between the reporter gene IRES-EGFP (5' end and 3' end) and the genomic DNA. Fig. 6C shows the results of Sanger sequencing of the nucleotide sequence at the junction between the reporter gene IRES-EGFP (5' end and 3' end) and the genomic DNA.
Fig. 7 shows a schematic diagram of the process for site-directed substitution of the nucleotide fragment in GAPDH gene of the human cell genome with a target nucleic acid fragment comprising an exogenous gene (T2A-EGFP).
Fig. 8 shows the efficiency of site-directed substitution of the nucleotide fragment in GAPDH gene of the human cell genome with a target nucleic acid fragment comprising an exogenous gene (T2A-EGFP) using the method of the present invention.
Fig. 9 shows a schematic diagram of the results of using the HDR method and the method of the present invention (EPTI) to perform site-directed knock-in of an exogenous gene (T2A-EGFP) before the stop codon of ACTB gene in 293T cell, so that T2A-EGFP and ACTB gene are fused and expressed.
Fig. 9A shows a schematic diagram of HDR and EPTI-mediated site-directed knock-in of an exogenous gene (T2A-EGFP) in front of the stop codon of ACTB gene.
Fig. 9B shows the sequence diagram of EPTI-mediated site-directed knock-in of exogenous gene (T2A-EGFP) in front of the stop codon of ACTB gene;
   wherein, the first row of sequences represents the human ACTB gene sequence; the blue sequence represents the protein-coding sequence of ACTB gene (the blue sequence is also a homologous sequence); the black triangle represents the targeted cleavage site (sgACTB2) in the genome; wherein, TAG represents the stop codon, and the genome-targeted cleavage site is between the "T" base and the "A" base of the stop codon.
   The second row of sequences represents the sequence of the donor vector, wherein the black triangle represents the targeted cleavage site at the upstream of the exogenous gene on the donor vector. As described above, when this site is recognized and cleaved by the complex of Cas9-MLV-RT and pegRNA, the pegRNA will extend the 3' end of the upstream nucleic acid strand of the exogenous gene to form an overhang sequence.
   The third row of sequences represents the sequence after the inter-strand annealing between the overhang sequence at the upstream of the exogenous gene and the homologous sequence of the ACTB gene; due to inter-strand annealing, the spacer sequence (for example, base "T") between the homologous sequence of ACTB gene and the fragmentation site forms a free base;
   wherein, the blue sequence is a homologous sequence of the ACTB gene; the blue sequence in the gray box is an overhang sequence formed at the upstream of the exogenous gene; the red sequence in the gray box is the sequence of the donor vector;
   the fourth row of sequences represents the sequence in which the site-directed knock-in of the exogenous gene (T2A-EGFP) before the stop codon of ACTB gene is completed, wherein the free "T" base is cleaved to achieve the continuity of the open reading frame and the protein fusion expression.

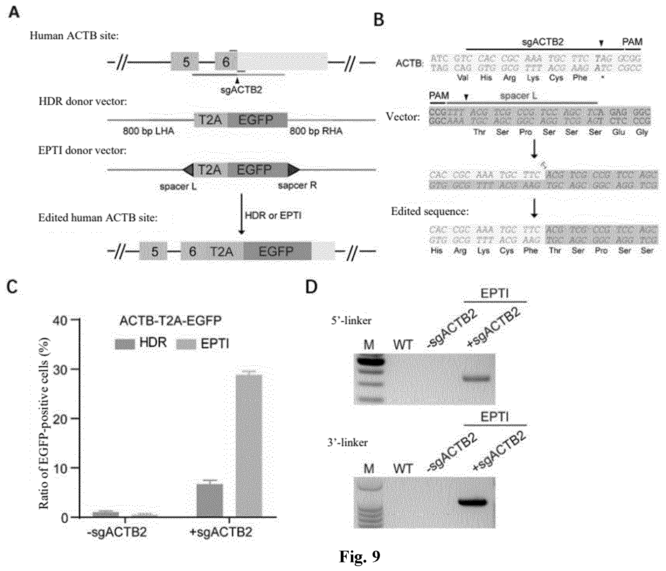

Fig. 9C shows a comparison of efficiency of HDR and EPTI-mediated site-directed knock-in of the exogenous gene (T2A-EGFP) in front of the stop codon of ACTB gene.
Fig. 9D shows the results of PCR identification of the nucleotide sequence at the junction between the reporter gene EGFP (5' end and 3' end) and the genomic DNA.

### Sequence information

Information of partial sequences involved in the present invention is provided in Table 1 below.

**Table 1: Description of sequences**

| SEQ ID NO: | Description | Sequence |
|---|---|---|
| 1 | Cas9 protein | |
| | | |
| 2 | mCherry | MVSKGEEDNMAIIKEFMRFKVHMEGSVNGHEFEIEGEGEGRPYE GTQTAKLKVTKGGPLPFAWDILSPQFMYGSKAYVKHPADIPDYL KLSFPEGFKWERVMNFEDGGVVTVTQDSSLQDGEFIYKVKLRGT NFPSDGPVMQKKTMGWEASSERMYPEDGALKGEIKQRLKLKDG GHYDAEVKTTYKAKKPVQLPGAYNVNIKLDITSHNEDYTIVEQY ERAEGRHSTGGMDELYK |
| 3 | gRNA without guide sequence | GTTTTAGAGCTAGAAATAGCAAGTTAAAATAAGGCTAGTCCG TTATCAACTTGAAAAAGTGGCACCGAGTCGGTGC |
| 4 | MLV RT | MSETPGTSESATPESSGGSSGGSSTLNIEDEYRLHETSKEPDVSLG STWLSDFPQAWAETGGMGLAVRQAPLIIPLKATSTPVSIKQYPM SQEARLGIKPHIQRLLDQGILVPCQSPWNTPLLPVKKPGTNDYRP VQDLREVNKRVEDIHPTVPNPYNLLSGLPPSHQWYTVLDLKDAF FCLRLHPTSQPLFAFEWRDPEMGISGQLTWTRLPQGFKNSPTLFN EALHRDLADFRIQHPDLILLQYVDDLLLAATSELDCQQGTRALL QTLGNLGYRASAKKAQICQKQVKYLGYLLKEGQRWLTEARKET VMGQPTPKTPRQLREFLGKAGFCRLFIPGFAEMAAPLYPLTKPGT LFNWGPDQQKAYQEIKQALLTAPALGLPDLTKPFELFVDEKQGY AKGVLTQKLGPWRRPVAYLSKKLDPVAAGWPPCLRMVAAIAVL TKDAGKLTMGQPLVILAPHAVEALVKQPPDRWLSNARMTHYQA LLLDTDRVQFGPVVALNPATLLPLPEEGLQHNCLDILAEAHGTRP DLTDQPLPDADHTWYTDGSSLLQEGQRKAGAAVTTETEVIWAK ALPAGTSAQRAELIALTQALKMAEGKKLNVYTDSRYAFATAHIH GEIYRRRGWLTSEGKEIKNKDEILALLKALFLPKRLSIIHCPGHQK GHSAEARGNRMADQAARKAAITETPDTSTLLIENSSPSGGSKRT ADGSEFEPKKKRKV |
| 5 | Gapdh-gRNA-F | CCGGAGAGAGAGACCCTCACTGCT |
| 6 | Gapdh-gRNA-R | AAACAGCAGTGAGGGTCTCTCTCT |
| 7 | Gapdh-gRNA | AGAGAGAGACCCTCACTGCTGTTTTAGAGCTAGAAATAGCAA GTTAAAATAAGGCTAGTCCGTTATCAACTTGAAAAAGTGGCA CCGAGTCGGTGC |
| 8 | Actb-gRNA-F | CCGGATCCCCCAAAGTTCACAATG |
| 9 | Actb-gRNA-R | AAACCATTGTGAACTTTGGGGGAT |
| 10 | Actb-gRNA | ATCCCCCAAAGTTCACAATGGTTTTAGAGCTAGAAATAGCAA GTTAAAATAAGGCTAGTCCGTTATCAACTTGAAAAAGTGGCA CCGAGTCGGTGC |
| 11 | GapdhRC- gRNA-F | CCGGTAGCGTTGACCCGACCCCAA |
| 12 | GapdhRC- gRNA-R | AAACTTGGGGTCGGGTCAACGCTA |
| 13 | GapdhRC-gRNA | TAGCGTTGACCCGACCCCAAGTTTTAGAGCTAGAAATAGCAA GTTAAAATAAGGCTAGTCCGTTATCAACTTGAAAAAGTGGCA CCGAGTCGGTGC |
| 14 | GapdhRT- gRNA-F | CCGGGTAAGCACACGTGCAAAGTG |
| 15 | GapdhRT- gRNA-R | AAACCACTTTGCACGTGTGCTTAC |
| 16 | GapdhRT-gRNA | GTAAGCACACGTGCAAAGTGGTTTTAGAGCTAGAAATAGCAA GTTAAAATAAGGCTAGTCCGTTATCAACTTGAAAAAGTGGCA CCGAGTCGGTGC |
| 17 | spacerA-gRNA-F | CCGGGAGATCGAGTGCCGCATCAC |
| 18 | spacerA-gRNA-R | AAAC GTGATGCGGCACTCGATCTC |
| 19 | spacerA-gRNA | GAGATCGAGTGCCGCATCACGTTTTAGAGCTAGAAATAGCAA GTTAAAATAAGGCTAGTCCGTTATCAACTTGAAAAAGTGGCA CCGAGTCGGTGC |
| 20 | spacerK-gRNA-F | CCGG GTCGCCCTCGAACTTCACCT |
| 21 | spacerK-gRNA-R | AAACAGGTGAAGTTCGAGGGCGAC |
| 22 | spacerK-gRNA | GTCGCCCTCGAACTTCACCTGTTTTAGAGCTAGAAATAGCAAG TTAAAATAAGGCTAGTCCGTTATCAACTTGAAAAAGTGGCAC CGAGTCGGTGC |
| 23 | Gapdh-pegA-F | AAAGTGGCACCGAGTCGGTGCAGCAAGAGCACAAGAGGAAG AGAGAGACCCTCACTATGCGGCAC |
| 24 | Gapdh-pegA-R | ACAGCTATGACCATGATTACGCCAAGCTTAAAAAAAATCGAG TGCCGCATAGTGAGGGTCTC |
| 25 | Gapdh-pegA | GAGATCGAGTGCCGCATCACGTTTTAGAGCTAGAAATAGCAA GTTAAAATAAGGCTAGTCCGTTATCAACTTGAAAAAGTGGCA CCGAGTCGGTGCAGCAAGAGCACAAGAGGAAGAGAGAGACC CTCACTATGCGGCACTCGA |
| 26 | Gapdh-pegK-F | AAAGTGGCACCGAGTCGGTGCTGGTGGGGGACTGAGTGTGGC AGGGACTCCCCAGC |
| 27 | Gapdh-pegK-R | GACCATGATTACGCCAAGCTTAAAAAAAACCCTCGAACTTCA GCTGGGGAGTCCCTGCCA |
| 28 | Gapdh-pegK | GTCGCCCTCGAACTTCACCTGTTTTAGAGCTAGAAATAGCAAG TTAAAATAAGGCTAGTCCGTTATCAACTTGAAAAAGTGGCAC CGAGTCGGTGCTGGTGGGGGACTGAGTGTGGCAGGGACTCCC CAGCTGAAGTTCGAGGG |
| 29 | Actb-pegA-F | AAAGTGGCACCGAGTCGGTGCCAGTCGGTTGGAGCGAGCATC CCCCAAAGTTCACAATGCGG |
| 30 | Actb-pegA-R | ACAGCTATGACCATGATTACGCCAAGCTTAAAAAAAATCGAG TGCCGCATTGTGAACTTTGGGGGA |
| 31 | Actb-pegA | GAGATCGAGTGCCGCATCACGTTTTAGAGCTAGAAATAGCAA GTTAAAATAAGGCTAGTCCGTTATCAACTTGAAAAAGTGGCA CCGAGTCGGTGCCAGTCGGTTGGAGCGAGCATCCCCCAAAGT TCACAATGCGGCACTCGA |
| 32 | Actb-pegK-F | AAAGTGGCACCGAGTCGGTGCAAACAACAATGTGCAATCAAA GTCCTCGGCCACATTGAAGTTCGA |
| 33 | Actb-pegK-R | ACAGCTATGACCATGATTACGCCAAGCTTAAAAAAAACCCTC GAACTTCAATGTGGCCGAGGA |
| 34 | Actb-pegK | GTCGCCCTCGAACTTCACCTGTTTTAGAGCTAGAAATAGCAAG TTAAAATAAGGCTAGTCCGTTATCAACTTGAAAAAGTGGCAC CGAGTCGGTGCAAACAACAATGTGCAATCAAAGTCCTCGGCC ACATTGAAGTTCGAGGG |
| 35 | GapdhRC-pegRNA-F | AAGTGGCACCGAGTCGGTGCTTTACAGCCTGGCCTTTGGAGAT CGAGTGCCGCATGGGTC |
| 36 | GapdhRC-pegRNA-R | AACAGCTATGACCATGATTACGCCAAGCTTAAAAAAAATTGA CCCGACCCATGCGGCACTCGATC |
| 37 | GapdhRC-pegRNA | TAGCGTTGACCCGACCCCAAGTTTTAGAGCTAGAAATAGCAA GTTAAAATAAGGCTAGTCCGTTATCAACTTGAAAAAGTGGCA CCGAGTCGGTGCTTTACAGCCTGGCCTTTGGAGATCGAGTGCC GCATGGGTCGGGTCAA |
| 38 | GapdhRT-pegRNA-F | AAGTGGCACCGAGTCGGTGCGTTACTCCCGGGCCTCACGTCG CCCTCGAACTTCATTTGCA |
| 39 | GapdhRT-pegRNA-R | AACAGCTATGACCATGATTACGCCAAGCTTAAAAAAAAGCAC ACGTGCAAATGAAGTTCGAGGGCGAC |
| 40 | GapdhRT-pegRNA | GTAAGCACACGTGCAAAGTGGTTTTAGAGCTAGAAATAGCAA GTTAAAATAAGGCTAGTCCGTTATCAACTTGAAAAAGTGGCA |
| | | CCGAGTCGGTGCGTTACTCCCGGGCCTCACGTCGCCCTCGAAC TTCATTTGCACGTGTGC |
| 41 | GapdhRC-pegA-F | AAGTGGCACCGAGTCGGTGCGCCCTTCCCCTGCCAGCCTAGC GTTGACCCGACCCATGC |
| 42 | GapdhRC-pegA-R | ACAGCTATGACCATGATTACGCCAAGCTTAAAAAAAATCGAG TGCCGCATGGGTCGGGTCAACGCTA |
| 43 | GapdhRC-pegA | GAGATCGAGTGCCGCATCACGTTTTAGAGCTAGAAATAGCAA GTTAAAATAAGGCTAGTCCGTTATCAACTTGAAAAAGTGGCA CCGAGTCGGTGCGCCCTTCCCCTGCCAGCCTAGCGTTGACCCG ACCCATGCGGCACTCGA |
| 44 | GapdhRT-pegK-F | AAGTGGCACCGAGTCGGTGCTACTTTTGTCTCCACTAGGTAAG CACACGTGCAAATGAAG |
| 45 | GapdhRT-pegK-R | AACAGCTATGACCATGATTACGCCAAGCTTAAAAAAAACCCT CGAACTTCATTTGCACGTGTGCTTACCTA |
| 46 | GapdhRT-pegK | GTCGCCCTCGAACTTCACCTGTTTTAGAGCTAGAAATAGCAAG TTAAAATAAGGCTAGTCCGTTATCAACTTGAAAAAGTGGCAC CGAGTCGGTGCTACTTTTGTCTCCACTAGGTAAGCACACGTGC AAATGAAGTTCGAGGG |
| 47 | IRES-EGFP | CCGGTGATGCGGCACTCGATCTCGAATTCCCCCTCTCCCTCCC CCCCCCCTAACGTTACTGGCCGAAGCCGCTTGGAATAAGGCC GGTGTGCGTTTGTCTATATGTTATTTTCCACCATATTGCCGTCT TTTGGCAATGTGAGGGCCCGGAAACCTGGCCCTGTCTTCTTGA CGAGCATTCCTAGGGGTCTTTCCCCTCTCGCCAAAGGAATGCA AGGTCTGTTGAATGTCGTGAAGGAAGCAGTTCCTCTGGAAGC TTCTTGAAGACAAACAACGTCTGTAGCGACCCTTTGCAGGCA GCGGAACCCCCCACCTGGCGACAGGTGCCTCTGCGGCCAAAA GCCACGTGTATAAGATACACCTGCAAAGGCGGCACAACCCCA GTGCCACGTTGTGAGTTGGATAGTTGTGGAAAGAGTCAAATG GCTCTCCTCAAGCGTATTCAACAAGGGGCTGAAGGATGCCCA GAAGGTACCCCATTGTATGGGATCTGATCTGGGGCCTCGGTGC ACATGCTTTACATGTGTTTAGTCGAGGTTAAAAAAACGTCTAG GCCCCCCGAACCACGGGGACGTGGTTTTCCTTTGAAAAACAC GATGATAATATGGCCACAACGCTCGGTTTAAAAAGCTTCTATG CCTGAATAGGTGACCGGAGGTCGGCACCTTTCCTTTGCAATTA CTGACCCTATGAATACAGGATCTATGGTGAGCAAGGGCGAGG AGCTGTTCACCGGGGTGGTGCCCATCCTGGTCGAGCTGGACG GCGACGTAAACGGCCACAAGTTCAGCGTGTCCGGCGAGGGCG AGGGCGATGCCACCTACGGCAAGCTGACCCTGAAGTTCATCT GCACTACGGGGAAACTGCCCGTGCCCTGGCCCACCCTCGTGA CCACCCTGACCTACGGCGTGCAGTGCTTCAGCCGCTACCCCGA CCACATGAAGCAGCACGACTTCTTCAAGTCCGCCATGCCCGA AGGCTACGTCCAGGAGCGCACCATCTTCTTCAAGGACGACGG CAACTACAAGACCCGCGCTGAAGTCAAATTCGAGGGCGACAC CCTGGTGAACCGCATCGAGCTGAAGGGCATCGACTTCAAGGA GGACGGCAACATCCTGGGGCACAAGCTGGAGTACAACTACAA CAGCCACAACGTCTATATCATGGCCGACAAGCAGAAGAACGG CATCAAGGTGAACTTCAAGATCCGCCACAACATCGAGGACGG CAGCGTGCAGCTCGCCGACCACTACCAGCAGAACACCCCCAT CGGCGACGGCCCCGTGCTGCTGCCCGACAACCACTACCTGAG CACCCAGTCCGCCCTGAGCAAAGACCCCAACGAGAAGCGCGA TCACATGGTCCTGCTGGAGTTCGTGACCGCCGCCGGGATCACT CTCGGCATGGACGAGCTGTACAAGTAAGTCGCCCTCGAACTT CACCTCGG |
| 48 | Gene fragment comprising T2A-EGFP | CCGGTGATGCGGCACTCGATCTCCAAAGGCCAGGCTGTAAAT GTCACCGGGAGGATTGGGTGTCTGGGCGCCTCGGGGAACCTG CCCTTCTCCCCATTCCGTCTTCCGGAAACCAGATCTCCCACCG CACCCTGGTCTGAGGTTAAATATAGCTGCTGACCTTTCTGTAG CTGGGGGCCTGGGCTGGGGCTCTCTCCCATCCCTTCTCCCCAC ACACATGCACTTACCTGTGCTCCCACTCCTGATTTCTGGAAAA GAGCTAGGAAGGACAGGCAACTTGGCAAATCAAAGCCCTGGG ACTAGGGGGTTAAAATACAGCTTCCCCTCTTCCCACCCGCCCC AGTCTCTGTCCCTTTTGTAGGAGGGACTTAGAGAAGGGGTGG GCTTGCCCTGTCCAGTTAATTTCTGACCTTTACTCCTGCCCTTT |
| | | GAGTTTGATGATGCTGAGTGTACAAGCGTTTTCTCCCTAAAGG GTGCAGCTGAGCTAGGCAGCAGCAAGCATTCCTGGGGTGGCA TAGTGGGGTGGTGAATACCATGTACAAAGCTTGTGCCCAGAC TGTGGGTGGCAGTGCCCCACATGGCCGCTTCTCCTGGAAGGG CTTCGTATGACTGGGGGTGTTGGGCAGCCCTGGAGCCTTCAGT TGCAGCCATGCCTTAAGCCAGGCCAGCCTGGCAGGGAAGCTC AAGGGAGATAAAATTCAACCTCTTGGGCCCTCCTGGGGGTAA GGAGATGCTGCATTCGCCCTCTTAATGGGGAGGTGGCCTAGG GCTGCTCACATATTCTGGAGGAGCCTCCCCTCCTCATGCCTTC TTGCCTCTTGTCTCTTAGATTTGGTCGTATTGGGCGCCTGGTCA CCAGGGCTGCTTTTAACTCTGGTAAAGTGGATATTGTTGCCAT CAATGACCCCTTCATTGACCTCAACTACATGGTGAGTGCTACA TGGTGAGCCCCAAAGCTGGTGTGGGAGGAGCCACCTGGCTGA TGGGCAGCCCCTTCATACCCTCACGTATTCCCCCAGGTTTACA TGTTCCAATATGATTCCACCCATGGCAAATTCCATGGCACCGT CAAGGCTGAGAACGGGAAGCTTGTCATCAATGGAAATCCCAT CACCATCTTCCAGGAGTGAGTGGAAGACAGAATGGAAGAAAT GTGCTTTGGGGAGGCAACTAGGATGGTGTGGCTCCCTTGGGT ATATGGTAACCTTGTGTCCCTCAATATGGTCCTGTCCCCATCT CCCCCCCACCCCCATAGGCGAGATCCCTCCAAAATCAAGTGG GGCGATGCTGGCGCTGAGTACGTCGTGGAGTCCACTGGCGTC TTCACCACCATGGAGAAGGCTGGGGTGAGTGCAGGAGGGCCC GCGGGAGGGGAAGCTGACTCAGCCCTGCAAAGGCAGGACCC GGGTTCATAACTGTCTGCTTCTCTGCTGTAGGCTCATTTGCAG GGGGGAGCCAAAAGGGTCATCATCTCTGCCCCCTCTGCTGAT GCCCCCATGTTCGTCATGGGTGTGAACCATGAGAAGTATGAC AACAGCCTCAAGATCATCAGGTGAGGAAGGCAGGGCCCGTGG AGAAGCGGCCAGCCTGGCACCCTATGGACACGCTCCCCTGAC TTGCGCCCCGCTCCCTCTTTCTTTGCAGCAATGCCTCCTGCACC ACCAACTGCTTAGCACCCCTGGCCAAGGTCATCCATGACAACT TTGGTATCGTGGAAGGACTCATGGTATGAGAGCTGGGGAATG GGACTGAGGCTCCCACCTTTCTCATCCAAGACTGGCTCCTCCC TGCCGGGGCTGCGTGCAACCCTGGGGTTGGGGGTTCTGGGGA CTGGCTTTCCCATAATTTCCTTTCAAGGTGGGGAGGGAGGTAG AGGGGTGATGTGGGGAGTACGCTGCAGGGCCTCACTCCTTTT GCAGACCACAGTCCATGCCATCACTGCCACCCAGAAGACTGT GGATGGCCCCTCCGGGAAACTGTGGCGTGATGGCCGCGGGGC TCTCCAGAACATCATCCCTGCCTCTACTGGCGCTGCCAAGGCT GTGGGCAAGGTCATCCCTGAGCTGAACGGGAAGCTCACTGGC ATGGCCTTCCGTGTCCCCACTGCCAACGTGTCAGTGGTGGACC TGACCTGCCGTCTAGAAAAACCTGCCAAATATGATGACATCA AGAAGGTGGTGAAGCAGGCGTCGGAGGGCCCCCTCAAGGGC ATCCTGGGCTACACTGAGCACCAGGTGGTCTCCTCTGACTTCA ACAGCGACACCCACTCCTCCACCTTTGACGCTGGGGCTGGCAT TGCCCTCAACGACCACTTTGTCAAGCTCATTTCCTGGTATGTG GCTGGGGCCAGAGACTGGCTCTTAAAAAGTGCAGGGTCTGGC GCCCTCTGGTGGCTGGCTCAGAAAAAGGGCCCTGACAACTCT TTTCATCTTCTAGGTATGACAACGAATTTGGCTACAGCAACAG GGTGGTGGACCTCATGGCCCACATGGCCTCCAAGGAGGAGGG CAGAGGAAGTCTTCTAACATGCGGTGACGTGGAGGAGAATCC CGGCCCAATGGTGAGCAAGGGCGAGGAGCTGTTCACCGGGGT GGTGCCCATCCTGGTCGAACTCGATGGAGATGTGAACGGCCA CAAGTTCAGCGTGTCCGGCGAGGGCGAGGGCGATGCCACCTA CGGCAAGCTGACCCTGAAGTTCATCTGCACTACGGGGAAACT GCCCGTGCCCTGGCCCACCCTCGTGACCACCCTGACCTACGGC GTGCAGTGCTTCAGCCGCTACCCCGACCACATGAAGCAGCAC GACTTCTTCAAGTCCGCCATGCCAGAGGGATATGTGCAAGAG CGCACCATCTTCTTCAAGGACGACGGCAACTACAAGACCCGC GCTGAAGTCAAATTCGAGGGCGACACCCTGGTGAACCGCATC GAGCTGAAGGGCATCGACTTCAAGGAGGACGGCAACATCCTG GGGCACAAGCTGGAGTACAACTACAACAGCCACAACGTCTAT ATCATGGCCGACAAGCAGAAGAACGGCATCAAGGTGAACTTC AAGATCCGCCACAACATCGAGGACGGCAGCGTGCAGCTCGCC GACCACTACCAGCAGAACACCCCCATCGGCGACGGCCCCGTG |
| | | CTGCTGCCCGACAACCACTACCTGAGCACCCAGTCCGCCCTG AGCAAAGACCCCAACGAGAAGCGCGATCACATGGTCCTGCTG GAGTTCGTGACCGCCGCCGGGATCACTCTCGGCATGGACGAG CTGTACAAGTAAGACCCCTGGACCACCAGCCCCAGCAAGAGC ACAAGAGGAAGAGAGAGACCCTCACTGCTGGGGAGTCCCTGC CACACTCAGTCCCCCACCACACTGAATCTCCCCTCCTCACAGT TGCCATGTAGACCCCTTGAAGAGGGGAGGGGCCTAGGGAGCC GCACCTTGTCATGTACCATCAATAAAGTACCCTGTGCTCAACC AGTTACTTGTCCTGTCTTATTCTAGGGTCTGGGGCAGAGGGGA GGGAAGCTGGGCTTGTGTCAAGGTGAGACATTCTTGCTGGGG AGGGACCTGGTATGTTCTCCTCAGACTGAGGGTAGGGCCTCC AAACAGCCTTGCTTGCTTCGAGAACCATTTGCTTCCCGCTCAG ACGTCTTGAGTGCTACAGGAAGCTGGCACCACTACTTCAGAG AACAAGGCCTTTTCCTCTCCTCGCTCCAGTCCTAGGCTATCTG CTGTTGGCCAAACATGGAAGAAGCTATTCTGTGGGCAGCCCC AGGGAGGCTGACAGGTGGAGGAAGTCAGGGCTCGCACTGGG CTCTGACGCTGACTGGTTAGTGGAGCTCAGCCTGGAGCTGAG CTGCAGCGGGCAATTCCAGCTTGGCCTCCGCAGCTGTGAGGTC TTGAGCACGTGCTCTATTGCTTTCTGTGCCCTCGTGTCTTATCT GAGGACATCGTGGCCAGCCCCTAAGGTCTTCAAGCAGGATTC ATCTAGGTAAACCAAGTACCTAAAACCATGCCCAAGGCGGTA AGGACTATATAATGTTTAAAAATCGGTAAAAATGCCCACCTC GCATAGTTTTGAGGAAGATGAACTGAGATGTGTCAGGGTGAC TTATTTCCATCATCGTCCTTAGGGGAACTTGGGTAGGGGCAAG GCGTGTAGCTGGGACCTAGGTCCAGACCCCTGGCTCTGCCACT GAACGGCTCAGTTGCTTTGGGCAGTTACTCCCGGGCCTCACGT CGCCCTCGAACTTCACCTCGG |
| 49 | SpacerA | GAGATCGAGTGCCGCATCACCGG |
| 50 | SpacerK | GTCGCCCTCGAACTTCACCTCGG |
| 51 | Linker | SGGSSGGSSG |
| 52 | Fusion protein | MDKKYSIGLDIGTNSVGWAVITDEYKVPSKKFKVLGNTDRHSIK KNLIGALLFDSGETAEATRLKRTARRRYTRRKNRICYLQEIFSNE MAKVDDSFFHRLEESFLVEEDKKHERHPIFGNIVDEVAYHEKYP TIYHLRKKLVDSTDKADLRLIYLALAHMIKFRGHFLIEGDLNPDN SDVDKLFIQLVQTYNQLFEENPINASGVDAKAILSARLSKSRRLE NLIAQLPGEKKNGLFGNLIALSLGLTPNFKSNFDLAEDAKLQLSK DTYDDDLDNLLAQIGDQYADLFLAAKNLSDAILLSDILRVNTEIT KAPLSASMIKRYDEHHQDLTLLKALVRQQLPEKYKEIFFDQSKN GYAGYIDGGASQEEFYKFIKPILEKMDGTEELLVKLNREDLLRK QRTFDNGSIPHQIHLGELHAILRRQEDFYPFLKDNREKIEKILTFRI PYYVGPLARGNSRFAWMTRKSEETITPWNFEEVVDKGASAQSFI ERMTNFDKNLPNEKVLPKHSLLYEYFTVYNELTKVKYVTEGMR KPAFLSGEQKKAIVDLLFKTNRKVTVKQLKEDYFKKIECFDSVEI SGVEDRFNASLGTYHDLLKIIKDKDFLDNEENEDILEDIVLTLTLF EDREMIEERLKTYAHLFDDKVMKQLKRRRYTGWGRLSRKLINGI RDKQSGKTILDFLKSDGFANRNFMQLIHDDSLTFKEDIQKAQVS GQGDSLHEHIANLAGSPAIKKGILQTVKVVDELVKVMGRHKPEN IVIEMARENQTTQKGQKNSRERMKRIEEGIKELGSQILKEHPVEN TQLQNEKLYLYYLQNGRDMYVDQELDINRLSDYDVDHIVPQSF LKDDSIDNKVLTRSDKNRGKSDNVPSEEVVKKMKNYWRQLLNA KLITQRKFDNLTKAERGGLSELDKAGFIKRQLVETRQITKHVAQI LDSRMNTKYDENDKLIREVKVITLKSKLVSDFRKDFQFYKVREI NNYHHAHDAYLNAVVGTALIKKYPKLESEFVYGDYKVYDVRK MIAKSEQEIGKATAKYFFYSNIMNFFKTEITLANGEIRKRPLIETN GETGEIVWDKGRDFATVRKVLSMPQVNIVKKTEVQTGGFSKESI LPKRNSDKLIARKKDWDPKKYGGFDSPTVAYSVLVVAKVEKGK SKKLKSVKELLGITIMERSSFEKNPIDFLEAKGYKEVKKDLIIKLP KYSLFELENGRKRMLASAGELQKGNELALPSKYVNFLYLASHYE KLKGSPEDNEQKQLFVEQHKHYLDEIIEQISEFSKRVILADANLD KVLSAYNKHRDKPIREQAENIIHLFTLTNLGAPAAFKYFDTTIDR KRYTSTKEVLDATLIHQSITGLYETRIDLSQLGGDSGGSSGGSSGS ETPGTSESATPESSGGSSGGSSTLNIEDEYRLHETSKEPDVSLGST WLSDFPQAWAETGGMGLAVRQAPLIIPLKATSTPVSIKQYPMSQ |
| | | EARLGIKPHIQRLLDQGILVPCQSPWNTPLLPVKKPGTNDYRPVQ DLREVNKRVEDIHPTVPNPYNLLSGLPPSHQWYTVLDLKDAFFC LRLHPTSQPLFAFEWRDPEMGISGQLTWTRLPQGFKNSPTLFNEA LHRDLADFRIQHPDLILLQYVDDLLLAATSELDCQQGTRALLQTL GNLGYRASAKKAQICQKQVKYLGYLLKEGQRWLTEARKETVM GQPTPKTPRQLREFLGKAGFCRLFIPGFAEMAAPLYPLTKPGTLF NWGPDQQKAYQEIKQALLTAPALGLPDLTKPFELFVDEKQGYA KGVLTQKLGPWRRPVAYLSKKLDPVAAGWPPCLRMVAAIAVLT KDAGKLTMGQPLVILAPHAVEALVKQPPDRWLSNARMTHYQAL LLDTDRVQFGPVVALNPATLLPLPEEGLQHNCLDILAEAHGTRP DLTDQPLPDADHTWYTDGSSLLQEGQRKAGAAVTTETEVIWAK ALPAGTSAQRAELIALTQALKMAEGKKLNVYTDSRYAFATAHIH GEIYRRRGWLTSEGKEIKNKDEILALLKALFLPKRLSIIHCPGHQK GHSAEARGNRMADQAARKAAITETPDTSTLLIENSSPSGGSKRT ADGSEFEPKKKRKV |
| 53 | sgACTB2-F | CCGGCCACCGCAAATGCTTCTAGG |
| 54 | sgACTB2-R | AAACCCTAGAAGCATTTGCGGTGG |
| 55 | sgACTB2 | CCACCGCAAATGCTTCTAGGGTTTTAGAGCTAGAAATAGCAA GTTAAAATAAGGCTAGTCCGTTATCAACTTGAAAAAGTGGCA CCGAGTCGGTGC |
| 56 | ACTB2-sgL-F | CCGGGAGCTGGACGGCGACGTAAA |
| 57 | ACTB2-sgL-R | AAACTTTACGTCGCCGTCCAGCTC |
| 58 | ACTB2-sgL | GAGCTGGACGGCGACGTAAAGTTTTAGAGCTAGAAATAGCAA GTTAAAATAAGGCTAGTCCGTTATCAACTTGAAAAAGTGGCA CCGAGTCGGTGC |
| 59 | ACTB2-sgR-F | CCGGCATGCCCGAAGGCTACGTCC |
| 60 | ACTB2-sgR-R | AAACGGACGTAGCCTTCGGGCATG |
| 61 | ACTB2-sgR | CATGCCCGAAGGCTACGTCCGTTTTAGAGCTAGAAATAGCAA GTTAAAATAAGGCTAGTCCGTTATCAACTTGAAAAAGTGGCA CCGAGTCGGTGC |
| 62 | ACTB2-pegL-F | AAAGTGGCACCGAGTCGGTGCGGCCCCTCCATCGTCCACCGC AAATGCTTCACGTCGCCG |
| 63 | ACTB2-pegL-R | AACAGCTATGACCATGATTACGCCAAGCTTAAAAAAAAGACG GCGACGTGAAGCATTTGC |
| 64 | ACTB2-pegL | GAGCTGGACGGCGACGTAAAGTTTTAGAGCTAGAAATAGCAA GTTAAAATAAGGCTAGTCCGTTATCAACTTGAAAAAGTGGCA CCGAGTCGGTGCGGCCCCTCCATCGTCCACCGCAAATGCTTCA CGTCGCCGTC |
| 65 | ACTB2-pegR-F | AAAGTGGCACCGAGTCGGTGCGGTGTAACGCAACTAAGTCAT AGTCCGCCTCGTAGCC |
| 66 | ACTB2-pegR-R | AACAGCTATGACCATGATTACGCCAAGCTTAAAAAAAACGAA GGCTACGAGGCGGACTATGAC |
| 67 | ACTB2-pegR | CATGCCCGAAGGCTACGTCCGTTTTAGAGCTAGAAATAGCAA GTTAAAATAAGGCTAGTCCGTTATCAACTTGAAAAAGTGGCA CCGAGTCGGTGCGGTGTAACGCAACTAAGTCATAGTCCGCCT CGTAGCCTTCG |
| 68 | GAPDH-P1 | AAAAGTGCAGGGTCTGGC |
| 69 | GAPDH-P2 | ACACCGGCCTTATTCCAAGC |
| 70 | GAPDH-P3 | CCGACCACTACCAGCAGAACAC |
| 71 | GAPDH-P4 | CCAGACCCTAGAATAAGACAGGACA |
| 72 | ACTB-P1 | GGGAGCTGTCACATCCAGGGTC |
| 73 | ACTB-P2 | AAGACGGCAATATGGTGGAAAA |
| 74 | ACTB-P3 | CTGCCCGACAACCACTACCT |
| 75 | ACTB-P4 | CTAAGGCTGCTCAATGTCAAGG |
| 76 | ACTB2-P1 | GGGAGCTGTCACATCCAGGGTC |
| 77 | ACTB2-P2 | CATCTCCATCGAGTTCGACCAG |
| 78 | ACTB2-P3 | CTGCCCGACAACCACTACCT |
| 79 | ACTB2-P4 | CTAAGGCTGCTCAATGTCAAGG |
| 80 | spacer L | GAGCTGGACGGCGACGTAAACGG |
| 81 | spacer R | CATGCCCGAAGGCTACGTCCAGG |
| 82 | T2A-EGFP | GAGGGCAGAGGAAGTCTTCTAACATGCGGTGACGTGGAGGAG AATCCCGGCCCAGTGAGCAAGGGCGAGGAGCTGTTCACCGGG |
| | | GTGGTGCCCATCCTGGTCGAACTCGATGGAGATGTGAACGGC CACAAGTTCAGCGTGTCCGGCGAGGGCGAGGGCGATGCCACC TACGGCAAGCTGACCCTGAAGTTCATCTGCACTACGGGGAAA CTGCCCGTGCCCTGGCCCACCCTCGTGACCACCCTGACCTACG GCGTGCAGTGCTTCAGCCGCTACCCCGACCACATGAAGCAGC ACGACTTCTTCAAGTCCGCCATGCCAGAGGGATATGTGCAAG AGCGCACCATCTTCTTCAAGGACGACGGCAACTACAAGACCC GCGCTGAAGTCAAATTCGAGGGCGACACCCTGGTGAACCGCA TCGAGCTGAAGGGCATCGACTTCAAGGAGGACGGCAACATCC TGGGGCACAAGCTGGAGTACAACTACAACAGCCACAACGTCT ATATCATGGCCGACAAGCAGAAGAACGGCATCAAGGTGAACT TCAAGATCCGCCACAACATCGAGGACGGCAGCGTGCAGCTCG CCGACCACTACCAGCAGAACACCCCCATCGGCGACGGCCCCG TGCTGCTGCCCGACAACCACTACCTGAGCACCCAGTCCGCCCT GAGCAAAGACCCCAACGAGAAGCGCGATCACATGGTCCTGCT GGAGTTCGTGACCGCCGCCGGGATCACTCTCGGCATGGACGA GCTGTACAAGTAA |
| 83 | ACTB2 LHA | TGGACCTGGCTGGCCGGGACCTGACTGACTACCTCATGAAGA TCCTCACCGAGCGCGGCTACAGCTTCACCACCACGGCCGAGC GGGAAATCGTGCGTGACATTAAGGAGAAGCTGTGCTACGTCG CCCTGGACTTCGAGCAAGAGATGGCCACGGCTGCTTCCAGCT CCTCCCTGGAGAAGAGCTACGAGCTGCCTGACGGCCAGGTCA TCACCATTGGCAATGAGCGGTTCCGCTGCCCTGAGGCACTCTT CCAGCCTTCCTTCCTGGGTGAGTGGAGACTGTCTCCCGGCTCT GCCTGACATGAGGGTTACCCCTCGGGGCTGTGCTGTGGAAGC TAAGTCCTGCCCTCATTTCCCTCTCAGGCATGGAGTCCTGTGG CATCCACGAAACTACCTTCAACTCCATCATGAAGTGTGACGTG GACATCCGCAAAGACCTGTACGCCAACACAGTGCTGTCTGGC GGCACCACCATGTACCCTGGCATTGCCGACAGGATGCAGAAG GAGATCACTGCCCTGGCACCCAGCACAATGAAGATCAAGGTG GGTGTCTTTCCTGCCTGAGCTGACCTGGGCAGGTCGGCTGTGG GGTCCTGTGGTGTGTGGGGAGCTGTCACATCCAGGGTCCTCAC TGCCTGTCCCCTTCCCTCCTCAGATCATTGCTCCTCCTGAGCGC AAGTACTCCGTGTGGATCGGCGGCTCCATCCTGGCCTCGCTGT CCACCTTCCAGCAGATGTGGATCAGCAAGCAGGAGTATGACG AGTCCGGCCCCTCCATCGTCCACCGCAAATGCTTC |
| 84 | ACTB2 RHA | AGGCGGACTATGACTTAGTTGCGTTACACCCTTTCTTGACAAA ACCTAACTTGCGCAGAAAACAAGATGAGATTGGCATGGCTTT ATTTGTTTTTTTTGTTTTGTTTTGGTTTTTTTTTTTTTTTTGGCTT GACTCAGGATTTAAAAACTGGAACGGTGAAGGTGACAGCAGT CGGTTGGAGCGAGCATCCCCCAAAGTTCACAATGTGGCCGAG GACTTTGATTGCACATTGTTGTTTTTTTAATAGTCATTCCAAAT ATGAGATGCGTTGTTACAGGAAGTCCCTTGCCATCCTAAAAGC CACCCCACTTCTCTCTAAGGAGAATGGCCCAGTCCTCTCCCAA GTCCACACAGGGGAGGTGATAGCATTGCTTTCGTGTAAATTAT GTAATGCAAAATTTTTTTAATCTTCGCCTTAATACTTTTTTATT TTGTTTTATTTTGAATGATGAGCCTTCGTGCCCCCCCTTCCCCC TTTTTTGTCCCCCAACTTGAGATGTATGAAGGCTTTTGGTCTCC CTGGGAGTGGGTGGAGGCAGCCAGGGCTTACCTGTACACTGA CTTGAGACCAGTTGAATAAAAGTGCACACCTTAAAAATGAGG CCAAGTGTGACTTTGTGGTGTGGCTGGGTTGGGGGCAGCAGA GGGTGAACCCTGCAGGAGGGTGAACCCTGCAAAAGGGTGGG GCAGTGGGGGCCAACTTGTCCTTACCCAGAGTGCAGGTGTGT GGAGATCCCTCCTGCCTTGACATTGAGCAGCCTTAGAGGGTG GGGGAGGCTCAGGGGTCAGGTCTCTGTTC |

### Specific Models for Carrying Out the present invention

The present invention will now be described with reference to the following examples, which are intended to illustrate, but not limit, the present invention.

Unless otherwise indicated, the experiments and methods described in the examples were performed essentially according to conventional methods well known in the art and described in various references. For example, conventional techniques of immunology, biochemistry, chemistry, molecular biology, microbiology, cell biology, genomics and recombinant DNA used in the present invention can be found in Sambrook, Fritsch and Maniatis, MOLECULAR CLONING: A LABORATORY MANUAL, 2nd ed. (1989); CURRENT PROTOCOLS IN MOLECULAR BIOLOGY) (F.M. Ausubel et al., (1987)); "METHODS IN ENZYMOLOGY" series (Academic Publishing Company): "PCR 2: A PRACTICAL APPROACH" (edited by M.J. MacPherson, B.D. Hames, and G.R. Taylor (1995)), and ANIMAL CELL CULTURE (edited by R.I. Freshney) (1987)).

In addition, for specific conditions that were not indicated in the examples, they were carried out according to the conventional conditions or the conditions suggested by the manufacturer. The reagents or instruments used without the manufacturer's indication were conventional products that were obtained from the market. Those skilled in the art appreciate that the examples describe the present invention by way of example and are not intended to limit the scope of the present invention as claimed. All publications and other references mentioned herein are incorporated by reference in their entirety.

### Example 1. Site-directed insertion of exogenous genes into human GAPDH gene using EPTI system

In order to verify the effect of EPTI system for site-directed insertion of exogenous gene into the genome, the following experiment was designed in this example: the EPTI system was used to perform site-directed knock-in of the reporter gene IRES-EGFP into the 3' UTR region of the human genome GAPDH, and the HITI system was used as a control. The principle of the EPTI system for site-directed insertion of exogenous gene is shown in Fig. 1, and the specific process for site-directed knock-in of exogenous gene (IRES-EGFP) in GAPDH gene of the human cell genome is shown in Fig. 3.

GAPDH gene is located on chromosome 12, encodes glyceraldehyde-3-phosphate dehydrogenase, is an important housekeeping gene and is highly expressed in 293T cells. After the reporter gene was knocked into the 3' UTR region of GAPDH, it can be transcribed together with the GAPDH gene, and the IRES sequence therein can recruit ribosome, so that EGFP can be expressed. The fluorescence signal of EGFP can be conveniently observed and quantified directly by fluorescence microscopy, and cells expressing EGFP can be captured and quantified by flow cytometry.

In the present example, plasmid pCAG-Cas9-mCherry (which was capable of expressing Cas9 protein (SEQ ID NO: 1) and mCherry protein (SEQ ID NO: 2)) and pUC19-U6-gRNA (which was capable of transcribing gRNA (SEQ ID NO: 3) lacking the guide sequence) used in this example were obtained from Li Wei's research group, Institute of Zoology, Chinese Academy of Sciences.

A nucleotide fragment encoding MLV-TR (SEQ ID NO: 4) was amplified from the pCMV-PE2 (4132775) plasmid of addgene company, and partial nucleotide fragment encoding Cas9 and nucleotide fragment encoding mCherry were amplified from the pCAG-Cas9-mCherry plasmid. The amplified nucleotide fragments were ligated by In-fusion cloning technology to the pCAG-Cas9-mCherry plasmid double digested by AscI/BsrGI to obtain pCAG-Cas9-MLV RT-mCherry plasmid, which could express Cas9 protein, MLV-TR protein and mCherry protein.

Primers Gapdh-gRNA-F (SEQ ID NO: 5) and Gapdh-gRNA-R (SEQ ID NO: 6) were annealed and ligated by T4 ligase to the pUC19-U6-gRNA plasmid vector digested with BsaI to obtain pUC19-U6-Gapdh-gRNA plasmid, which could transcribe Gapdh-gRNA (SEQ ID NO: 7), thereby directing the Cas9 protein to the 3' URT region of the human GAPDH site.

Primers spacerA-gRNA-F (SEQ ID NO: 17) and spacerA-gRNA-R (SEQ ID NO: 18) were annealed and ligated by T4 ligase to the pUC19-U6-gRNA plasmid vector digested with BsaI to obtain pUC19-U6-spacerA-gRNA plasmid, which could transcribe spacerA-gRNA (SEQ ID NO: 19), thereby directing the Cas9 protein to the spacer A sequence (SEQ ID NO: 49).

Primers spacerK-gRNA-F (SEQ ID NO: 20) and spacerK-gRNA-R (SEQ ID NO: 21) were annealed and ligated by T4 ligase to the pUC19-U6-gRNA plasmid vector digested with BsaI to obtain pUC19-U6-spacerK-gRNA plasmid, which could transcribe spacerK-gRNA (SEQ ID NO:22), thereby directing the Cas9 protein to the spacerK sequence (SEQ ID NO:50).

Primers Gapdh-pegA-F (SEQ ID NO: 23) and Gapdh-pegA-R (SEQ ID NO: 24) were subjected to overlap extension PCR, and the obtained fragment was recovered and ligated by In-fusion cloning technology to pUC19-U6-spacerA-gRNA plasmid vector digested by HindIII to obtain pUC19-U6-Gapdh-pegA plasmid, which could transcribe Gapdh-pegA (SEQ ID NO: 25), thereby directing the Cas9 protein to target spacerA sequence (SEQ ID NO: 49).

Primers Gapdh-pegK-F (SEQ ID NO: 26) and Gapdh-pegK-R (SEQ ID NO: 27) were subjected to overlap extension PCR, and the obtained fragment was recovered and ligated by In-fusion cloning technology to pUC19-U6-spacerK-gRNA plasmid vector digested by HindIII, to obtain pUC19-U6-Gapdh-pegK plasmid, which could transcribe Gapdh-pegK (SEQ ID NO: 28), thereby directing the Cas9 protein to target spacerK sequence (SEQ ID NO: 50).

The reporter gene IRES-EGFP (SEQ ID NO: 47) was synthesized by Jerry Company, and was ligated through T4 ligase to pGH vector (provided by Jerry Company) digested by EcoRV, which was used as a donor vector. The reporter gene had the recognition and cleavage sites spacerA and spacerK (their sequences were SEQ ID NO: 49 and SEQ ID NO: 50, respectively) of spacerA-gRNA/Gapdh-pegA and spacerK-gRNA/Gapdh-pegK on both sides.

During the implementation of the EPTI system, pCAG-Cas9-MLV RT-mCherry, Gapdh-gRNA, Gapdh-pegA, Gapdh-pegK together with the donor vector were transfected into 293T cells with Lipofectamine 3000 lipofection reagent of Invitrogen Company. In the negative control group of the EPTI system, pCAG-Cas9-MLV RT-mCherry, Gapdh-pegA, Gapdh-pegK together with the donor vector were transfected into 293T cells. The 293T cell line was obtained from the ATCC cell bank. After 24 hours of transfection, mCherry-positive cells were sorted by flow cytometry, and after the sorted cells were cultured for 5 days, the ratio of EGFP-positive cells was analyzed by flow cytometry. During the implementation of the HITI system, pCAG-Cas9-mCherry, Gapdh-gRNA, spacerA-gRNA, spacerK-gRNA together with the donor vector were transfected into 293T cells with Lipofectamine 3000 lipofection reagent from Invitrogen Company. In the negative control group of the HITI system, pCAG-Cas9-mCherry, spacerA-gRNA, spacerK-gRNA together with the donor vector were transfected into 293T cells. After 24 hours of transfection, mCherry-positive cells were sorted by flow cytometry, and after the sorted cells were cultured for 5 days, the ratio of EGFP-positive cells was analyzed by flow cytometry. The comparison of the ratios of EGFP-positive cells between the two systems could reflect the difference in the efficiency of the two systems in the site-directed knock-in of GAPDH gene. The results of exogenous gene integration efficiency were shown in Fig. 4A. The results showed that the ratio of EGFP-positive cells in the HITI system was below 5%, while in the EPTI system, the ratio of EGFP-positive cells exceeded 60%. In addition, in the negative control group with removal of Gapdh-gRNA component, the HITI system and the EPTI system could not detect EGFP-positive cells, indicating that EGFP-positive cells could reflect the specific integration of exogenous gene IRES-EGFP on the donor vector at the target site of human GAPDH.

The genomic DNA of EGFP-positive cells was extracted, and then primers GAPDH-P1 (SEQ ID NO: 68)/GAPDH-P2 (SEQ ID NO: 69), GAPDH-P3 (SEQ ID NO: 70)/GAPDH-P4 (SEQ ID NO: 71) were used perform PCR identification and sequencing analysis by Sanger method of the nucleotide sequence at the junction between the reporter gene IRES-EGFP (5' end and 3' end) and the genomic DNA.

The PCR identification results were shown in Fig. 4B. The results showed that the used primer pairs GAPDH-P1/GAPDH-P2 and GAPDH-P3/GAPDH-P4 could use the genomic DNA of EGFP-positive cells as a template in the amplification and production of two amplified fragments with expected sizes. The results of Sanger sequencing analysis were shown in Fig. 4C. The results showed that the EPTI method of the present application could efficiently, site-specifically and accurately mediate the ligation of exogenous genes to the cleaved ends of the genomic DNA. In conclusion, the EPTI system described in the present invention could greatly improve the site-directed integration efficiency of exogenous genes.

### Example 2, Site-directed insertion of exogenous gene into human ACTB gene using EPTI system

In order to verify the effect of the EPTI system on the site-directed insertion of exogenous genes into the genome, the following experiments were designed in this example: the EPTI system was used to perform the site-directed knock-in of the reporter gene IRES-EGFP (SEQ ID NO: 47) into the 3'UTR region of the human genome ACTB, and the HITI system was used as a control. The specific process of site-directed knock-in of exogenous gene (IRES-EGFP) in ACTB gene of human cell genome was shown in Fig. 5. The synthesis and cleavage steps of the reporter gene were the same as those in Example 1.

Primers Actb-gRNA-F (SEQ ID NO: 8) and Actb-gRNA-R (SEQ ID NO: 9) were annealed and ligated with T4 ligase to the pUC19-U6-gRNA plasmid digested with BsaI to obtain the pUC19-U6-Actb-gRNA plasmid, which could transcribe Actb-gRNA (SEQ ID NO: 10), thereby directing the Cas9 protein to the 3' URT region of the human ACTB site.

Primers Actb-pegA-F (SEQ ID NO: 29) and Actb-pegA-R (SEQ ID NO: 30) were subjected to overlap extension PCR, and the obtained fragment was recovered and ligated through In-fusion cloning technology to pUC19-U6-spacerA-gRNA plasmid vector digested by HindIII to obtain pUC 19-U6-Actb-pegA plasmid, which could transcribe Actb-pegA (SEQ ID NO: 31), thereby directing the Cas9 protein to target spacer A sequence (SEQ ID NO: 49).

Primers Actb-pegK-F (SEQ ID NO: 32) and Actb-pegK-R (SEQ ID NO: 33) were subjected to overlap extension PCR, and the obtained fragment was recovered and ligated through In-fusion cloning technology to pUC19-U6-spacerK-gRNA plasmid vector digested by HindIII to obtain pUC 19-U6-Actb-pegK plasmid, which could transcribe Actb-pegK (SEQ ID NO: 34), thereby directing the Cas9 protein to target spacer K sequence (SEQ ID NO: 50).

During the implementation of the EPTI system, pCAG-Cas9-MLV RT-mCherry, Actb-gRNA, Actb-pegA, Actb-pegK together with the donor vector were transfected into 293T cells with Lipofectamine 3000 lipofection reagent of Invitrogen Company. In the negative control group of the EPTI system, pCAG-Cas9-MLV RT-mCherry, Actb-pegA, Actb-pegK together with the donor vector were transfected into 293T cells. After 24 hours of transfection, mCherry-positive cells were sorted by flow cytometry, and after the sorted cells were cultured for 5 days, the ratio of EGFP-positive cells was analyzed by flow cytometry. During the implementation of the HITI system, pCAG-Cas9-mCherry, Actb-gRNA, spacerA-gRNA, spacerK-gRNA together with the donor vector were transfected into 293T cells with Lipofectamine 3000 lipofection reagent from Invitrogen Company. In the negative control group of the HITI system, pCAG-Cas9-mCherry, spacerA-gRNA, spacerK-gRNA together with the donor vector were transfected into 293T cells. After 24 hours of transfection, mCherry-positive cells were sorted by flow cytometry, and after the sorted cells were cultured for 5 days, the ratio of EGFP-positive cells was analyzed by flow cytometry. The comparison of the ratios of EGFP-positive cells of the two different systems could reflect the difference in efficiency of the two systems for site-directed knock-in at the ACTB gene. The comparison results of exogenous gene integration efficiency of different methods were shown in Fig. 6A. The results showed that the ratio of EGFP-positive cells in the HITI system was below 8%, while in the EPTI system, the ratio of EGFP-positive cells exceeded 50%. In addition, in the negative control group with the removal of Actb-gRNA component, the HITI system and the EPTI system could not detect EGFP-positive cells, indicating that EGFP-positive cells could reflect the specific integration of the exogenous gene IRES-EGFP on the donor vector at the target site of human ACTB.

The genomic DNA of EGFP-positive cells was extracted, and then primers ACTB-P1 (SEQ ID NO:72)/ACTB-P2 (SEQ ID NO:73), ACTB-P3 (SEQ ID NO:74)/ACTB-P4 (SEQ ID NO:75) were respectively used to perform the PCR identification and Sanger sequencing analysis on the nucleotide sequence of the junction between the reporter gene IRES-EGFP (5' end and 3' end) and the genomic DNA.

The PCR identification results were shown in Fig. 6B. The results showed that the used primer pairs ACTB-P1/ACTB-P2, ACTB-P3/ACTB-P4 could use the genomic DNA of EGFP-positive cells as a template for the amplification and production of two amplified fragments with expected sizes. The results of Sanger sequencing analysis were shown in Fig. 6C. The results showed that the EPTI method of the present application could efficiently, site-specifically and accurately mediate the ligation of exogenous genes to the cleaved ends of the genomic DNA. In conclusion, the EPTI system described in the present invention could greatly improve the site-directed integration efficiency of exogenous genes.

### Example 3. Site-directed substitution of nucleotide fragment in human GAPDH gene with exogenous gene using EPTI system

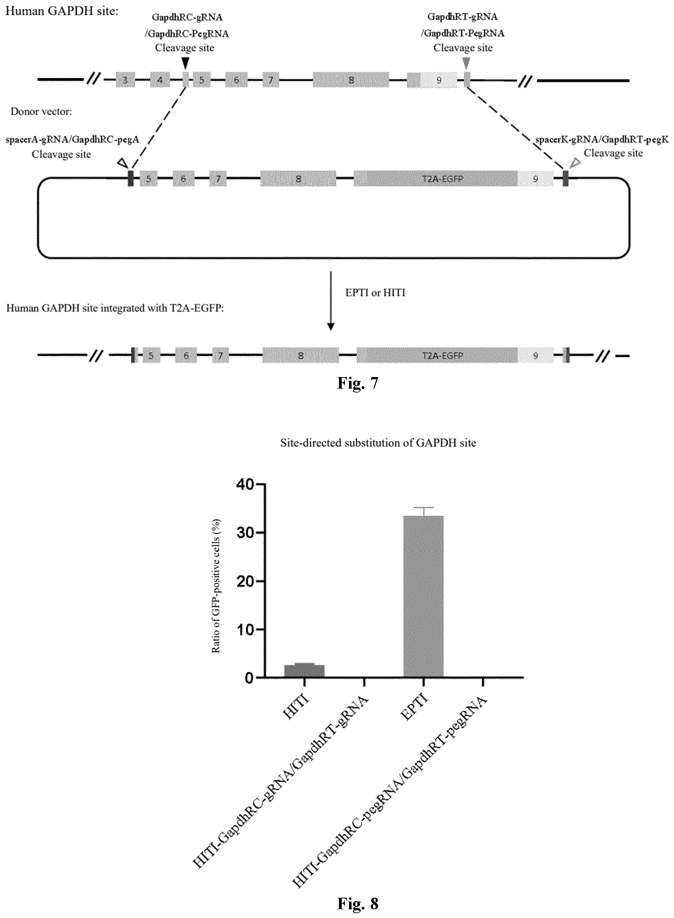

In order to verify the effect of the EPTI system for site-directed substitution of exogenous genes into the genome, the following experiments were designed in this example: the EPTI system was used to perform the site-directed substitution of the DNA fragment (SEQ ID NO: 48) comprising the reporter gene T2A-EGFP for a DNA sequence of the human genome GAPDH gene, and the HITI system was used as a control. A schematic diagram of the principle of the EPTI method to mediate the substitution of specific nucleotide fragment of genome with exogenous gene was shown in Fig. 2. The specific process for site-directed substitution of a nucleotide fragment in GAPDH gene of human cell genome with a target nucleic acid fragment comprising the exogenous gene (T2A-EGFP) was shown in Fig. 7. The synthesis and cleavage steps of the reporter gene were similar to those described in Example 1.

Primers GapdhRC-gRNA-F (SEQ ID NO: 11) and GapdhRC-gRNA-F (SEQ ID NO: 12) were annealed and ligated with T4 ligase to the pUC19-U6-gRNA plasmid digested with BsaI to obtain pUC19-U6-GapdhRC-gRNA plasmid, which could transcribe GapdhRC-gRNA (SEQ ID NO: 13), thereby directing the Cas9 protein to the intron region between exons 4 and 5 of the human GAPDH gene.

Primers GapdhRT-gRNA-F (SEQ ID NO: 14) and GapdhRT-gRNA-R (SEQ ID NO: 15) were annealed and ligated with T4 ligase to the pUC19-U6-gRNA plasmid digested with BsaI to obtain pUC19-U6-GapdhRT-gRNA plasmid, which could transcribe GapdhRT-gRNA (SEQ ID NO: 16), thereby directing the Cas9 protein to target the downstream region of the human GAPDH gene.

Primers GapdhRC-pegRNA-F (SEQ ID NO: 35) and GapdhRC-pegRNA-F (SEQ ID NO: 36) were subjected to overlap extension PCR, and the obtained fragment was recovered and ligated through In-fusion cloning technology to the pUC19-U6-GapdhRC-gRNA plasmid vector digested by HindIII to obtain pUC19-U6-GapdhRC-pegRNA plasmid, which could transcribe GapdhRC-pegRNA (SEQ ID NO: 37), thereby directing the Cas9 protein to target the intron region between exons 4 and 5 of the human GAPDH gene.

Primers GapdhRT-pegRNA-F (SEQ ID NO: 38) and GapdhRT-pegRNA-F (SEQ ID NO: 39) were subjected to overlap extension PCR, and the obtained fragment was recovered and ligated through In-fusion cloning technology to pUC19-U6-GapdhRT-gRNA plasmid vector digested by HindIII to obtain pUC19-U6-GapdhRT-pegRNA plasmid, which could transcribe GapdhRT-pegRNA (SEQ ID NO: 40), thereby directing the Cas9 protein to target the downstream region of the human GAPDH gene.

Primers GapdhRC-pegA-F (SEQ ID NO: 41) and GapdhRC-pegA-R (SEQ ID NO: 42) were subjected to overlap extension PCR, and the obtained fragment was recovered and ligated through In-fusion cloning technology to pUC19-U6-spacerA-gRNA plasmid vector digested by HindIII to obtain pUC19-U6-GapdhRC-pegA plasmid, which could transcribe GapdhRC-pegA (SEQ ID NO: 43), thereby directing the Cas9 protein to target spacerA sequence (SEQ ID NO: 49).

Primers GapdhRT-pegK-F (SEQ ID NO: 44) and GapdhRT-pegK-R (SEQ ID NO: 45) were subjected to overlap extension PCR, and the obtained fragment was recovered and ligated through In-fusion cloning technology to pUC19-U6-spacerK-gRNA plasmid vector digested by HindIII to obtain pUC19-U6-GapdhRT-pegK plasmid, which could transcribe GapdhRT-pegK (SEQ ID NO: 46), thereby directing the Cas9 protein to target spacerK sequence (SEQ ID NO: 50).

The exogenous gene fragment (SEQ ID NO: 48) comprising the reporter gene T2A-EGFP was synthesized by Jierui Company, and ligated through T4 ligase to pGH vector (provided by Jierui Company) digested by EcoRV, which was used as a donor vector. The reporter gene had the recognition and cleavage sites spacerA and spacerK (their sequences were SEQ ID NO: 49 and SEQ ID NO: 50, respectively) of spacerA-gRNA/Gapdh-pegA and spacerK-gRNA/Gapdh-pegK on both sides, respectively.

During the implementation of the EPTI system, pCAG-Cas9-MLV RT-mCherry, GapdhRC-pegRNA, GapdhRT-pegRNA, GapdhRC-pegA, GapdhRT-pegK, together with the donor vector were transfected with Lipofectamine 3000 lipofection reagent from Invitrogen Company into 293T cells. In the negative control group of the EPTI system, pCAG-Cas9-MLV RT-mCherry, GapdhRC-pegA, GapdhRT-pegK together with the donor vector were transfected into 293T cells. After 24 hours of transfection, mCherry-positive cells were sorted by flow cytometry, and after the sorted cells were cultured for 5 days, the ratio of EGFP-positive cells was analyzed by flow cytometry. During the implementation of the HITI system, pCAG-Cas9-mCherry, GapdhRC-gRNA, GapdhRT-gRNA, spacerA-gRNA, spacerK-gRNA together with the donor vector were transfected into 293T cells with Lipofectamine 3000 lipofection reagent of Invitrogen Company. In the negative control group of the HITI system, pCAG-Cas9-mCherry, spacerA-gRNA, spacerK-gRNA together with the donor vector were transfected into 293T cells. After 24 hours of transfection, mCherry-positive cells were sorted by flow cytometry, and after the sorted cells were cultured for 5 days, the ratio of EGFP-positive cells was analyzed by flow cytometry. The comparison of the ratios of EGFP-positive cells between the two systems could reflect the difference in the efficiency of site-directed substitution of the GAPDH gene between the two systems.

The experimental results were shown in Fig. 8. The ratio of EGFP-positive cells in the HITI system was about 3%, while in the EPTI system, the ratio of EGFP-positive cells was higher than 30%. In addition, in the negative control group in which the sgRNA/pegRNA components targeting the GAPDH gene were removed, neither the HITI system nor the EPTI system could detect EGFP-positive cells, indicating that EGFP-positive cells could reflect the occurrence of the site-directed substitution of the exogenous integrated with T2A-EGFP into the donor vector for the genomic fragment at human GAPDH target site. In conclusion, the EPTI system described in the present invention could greatly improve the efficiency of site-directed substitution of exogenous gene at the human GAPDH gene site.

### Example 4. Site-directed insertion of exogenous gene before stop codon of ACTB gene using EPTI system, to express ACTB-T2A-EGFP fusion protein

In order to further verify the effect of the EPTI system for accurate site-directed insertion of exogenous gene into the genome, the following experiment was designed in this example: the EPTI system was used to perform the site-directed knock-in of the reporter gene T2A-EGFP (SEQ ID NO: 82) into 293T cells before the stop codon of ACTB gene, and the HDR system was used as a control. The process of site-directed knock-in of the exogenous gene (T2A-EGFP) in the ACTB gene of the 293T cell genome was shown in Figures 9A and 9B.

As shown in Fig. 9B, the cleavage site to which the ACTB gene was targeted was not between the last coding codon and the stop codon, but between the base "T" and base "A" of the stop codon. In order for the exogenous gene fragment (T2A-EGFP) to be inserted just after the last coding codon of the ACTB gene and fused with the ACTB gene, the base "T" of the stop codon should be removed during the genome editing process. For this purpose, the tag sequence in the pegRNA was designed such that the overhang (the first overhang) on the resulting exogenous gene fragment was complementary to upstream of the site where the ACTB gene was targeted for cleavage; that was, there was a spacer sequence (in this example, the spacer sequence was the base "T" that should be removed) between the genomic sequence to be targeted to the first overhang and the site of the ACTB gene to be targeted for cleavage. With this design, after the first overhang was annealed to the genomic sequence strand to which it was targeted and bound, the spacer sequence would be in free state and excised; thus, the exogenous gene fragment (T2A-EGFP) could be precisely ligated to the last coding codon of ACTB gene.

The pCAG-Cas9-mCherry plasmid and pCAG-Cas9-MLV RT-mCherry used in this example were the same as those in Example 1.

Primers sgACTB2-F (SEQ ID NO: 53) and sgACTB2-R (SEQ ID NO: 54) were annealed and ligated with T4 ligase to the pUC19-U6-gRNA plasmid digested with BsaI to obtain pUC19-U6-sgACTB2 plasmid, which could transcribe sgACTB2 (SEQ ID NO: 55), thereby directing the Cas9 protein to target and cleave the specific site of the ACTB gene in 293T cells (i.e., performing cleavage between the base "T" and the base "A" of the stop codon of the ACTB gene).

Primers ACTB2-sgL-F (SEQ ID NO: 56) and ACTB2-sgL-R (SEQ ID NO: 57) were annealed and ligated with T4 ligase to the pUC19-U6-gRNA plasmid vector digested with BsaI to obtain pUC19-U6-ACTB2-sgL plasmid, which could transcribe ACTB2-sgL (SEQ ID NO: 58).

Primers ACTB2-sgR-F (SEQ ID NO: 59) and ACTB2-sgR-R (SEQ ID NO: 60) were annealed and ligated with T4 ligase to the pUC19-U6-gRNA plasmid vector digested with BsaI to obtain pUC19-U6-ACTB2-sgR plasmid, which could transcribe ACTB2-sgR (SEQ ID NO: 61).

Primers ACTB2-pegL-F (SEQ ID NO: 62) and ACTB2-pegL-R (SEQ ID NO: 63) were subjected to overlap extension PCR, and the obtained fragment was recovered and ligated through In-fusion cloning technology to the pUC19-U6-ACTB2-sgL plasmid vector digested by HindIII, to obtain pUC19-U6-ACTB2-pegL plasmid, which could transcribe ACTB2-pegL (SEQ ID NO:64), thereby directing the Cas9 protein to target spacer L sequence (SEQ ID NO:80).

Primers ACTB2-pegR-F (SEQ ID NO: 65) and ACTB2-pegR-R (SEQ ID NO: 66) were subjected to overlap extension PCR, and the obtained fragment was recovered and ligated through In-fusion cloning technology to pUC19-U6-ACTB-pegR plasmid vector digested by HindIII to obtain pUC19-U6-ACTB-pegR plasmid, which could transcribe ACTB2-pegR (SEQ ID NO: 67), thereby directing the Cas9 protein to target spacerR sequence (SEQ ID NO: 81).

The reporter gene T2A-EGFP (SEQ ID NO: 82) was synthesized by Jierui Company, andwas ligated with T4 ligase to the pGH vector (provided by Jierui Company) digested by EcoRV, which was used as a donor vector. For the donor vector of the EPTI system, the reporter gene T2A-EGFP had on both sides the spacer L sequence (SEQ ID NO:80) which was reverse to the T2A-EGFP gene and the spacer R sequence (SEQ ID NO:81) which was in the same direction as the T2A-EGFP gene, respectively. For the donor vector of the HDR system, the reporter gene T2A-EGFP had on both sides a left homology arm ACTB2 LHA (SEQ ID NO: 83) and a right homology arm ACTB2 RHA (SEQ ID NO: 84), respectively.

In the experiment using the EPTI system, pCAG-Cas9-MLV RT-mCherry, U6-sgACTB2, U6-ACTB2-pegL, U6-ACTB2-pegR were transfected together with the donor vector into 293T cells using Lipofectamine 3000 lipofection reagent from Invitrogen Company. In the negative control group of the EPTI system, pCAG-Cas9-MLV RT-mCherry, U6-ACTB2-pegL, U6-ACTB2-pegR together with the donor vector were transfected into 293T cells. In the experiment using the HDR system, pCAG-Cas9-mCherry and U6-sgACTB2 together with the HDR donor vector were transfected into 293T cells with Lipofectamine 3000 lipofection reagent from Invitrogen Company. In the negative control group of the HDR system, pCAG-Cas9-mCherry together with the HDR donor vector was transfected into 293T cells. The 293T cell line was obtained from the ATCC cell bank. After 24 hours of transfection, mCherry-positive cells sorted by flow cytometer were successfully transfected cells. After the sorted cells were cultured for 5 days, the ratio of EGFP-positive cells was analyzed by flow cytometry. The comparison of the ratios of EGFP-positive cells in the two different systems could reflect the difference in the efficiency of site-directed knock-in of ACTB gene between different systems. The results of the comparison of the efficiency of inserting exogenous genes using different methods were shown in Fig. 9C. The results showed that in the case of using the EPTI system, the ratio of EGFP-positive cells was about 30%, which was significantly higher than the method using the HDR system.

Genomic DNA was extracted from the EGFP-positive cells after 5 days of culture, and then the primers ACTB2-P1 (SEQ ID NO:76)/ACTB2-P2 (SEQ ID NO:77), ACTB2-P3 (SEQ ID NO:78)/ACTB2-P4 (SEQ ID NO: 79) were used to perform PCR identification of the nucleotide sequence at the junction between the reporter gene EGFP (5' end and 3' end) and the genomic DNA.

The PCR identification results were shown in Fig. 9D. The results showed that the used primer pairs ACTB2-P1/ACTB2-P2 and ACTB2-P3/ACTB2-P4 could use the genomic DNA of EGFP-positive cells as a template for the amplification and production of two amplified fragments with expected sizes. These results showed that the EPTI system of the present invention could achieve the site-directed and accurate insertion of the exogenous gene (T2A-EGFP) into the specific position of the ACTB gene in 293T cells. In addition, these results also indicated that the EPTI system of the present invention could still greatly improve the site-directed integration efficiency of exogenous genes in the target genome in the presence of a gap between the genomic sequence to which the overhang is targeted and bound and the site where the genome is targeted for cleavage.

## Claims

1. A system or kit, which comprises the following four components:
(1) a first Cas protein or a nucleic acid molecule A1 comprising a nucleotide sequence encoding the first Cas protein, wherein the first Cas protein is capable of cleaving or fragmenting a first double-stranded target nucleic acid;
(2) a first DNA polymerase that is template-dependent or a nucleic acid molecule B1 comprising a nucleotide sequence encoding the first DNA polymerase;
(3) a first gRNA or a nucleic acid molecule C1 comprising a nucleotide sequence encoding the first gRNA, wherein the first gRNA is capable of binding to the first Cas protein and forming a first functional complex; the first functional complex is capable of fragmenting two strands of the first double-stranded target nucleic acid to form a target nucleic acid fragment;
(4) a first tag primer or a nucleic acid molecule D1 comprising a nucleotide sequence encoding the first tag primer, wherein the first tag primer comprises a first tag sequence and a first target-binding sequence, the first tag sequence is located at the upstream or 5' end of the first target-binding sequence; and, under conditions that allow hybridization or annealing of nucleic acids, the first target-binding sequence is capable of being hybridized or annealed to the 3' end of one nucleic acid strand of the target nucleic acid fragment to form a double-stranded structure, and the first tag sequence does not bind to the target nucleic acid fragment and is in a free single-stranded state.

2. The system or kit according to claim 1, wherein the first Cas protein is selected from the group consisting of Cas9 protein, Cas12a protein, cas12b protein, cas12c protein, cas12d protein, cas12e protein, cas12f protein, cas12g protein, cas12h protein, cas12i protein, Cas14 protein, Cas13a protein, Cas1 protein, Cas1B protein, Cas2 protein, Cas3 protein, Cas4 protein, Cas5 protein, Cas6 protein, Cas7 protein, Cas8 protein, Cas 10 protein, Csy1 protein, Csy2 protein, Csy3 protein, Cse1 protein, Cse2 protein, Csc1 protein, Csc2 protein, Csa5 protein, Csn2 protein, Csm2 protein, Csm3 protein, Csm4 protein, Csm5 protein, Csm6 protein, Cmr1 protein, Cmr3 protein, Cmr4 protein, Cmr5 protein, Cmr6 protein, Csb1 protein, Csb2 protein, Csb3 protein, Csx17 protein, Csx14 protein, Csx10 protein, Csx16 protein, CsaX protein, Csx3 protein, Csx1 protein, Csx15 protein, Csf1 protein, Csf2 protein, Csf3 protein, Csf4 protein and homologue thereof or modified form thereof;
preferably, the first Cas protein is capable of fragmenting the first double-stranded target nucleic acid to produce a sticky end or blunt end;
preferably, the first Cas protein is a Cas9 protein, such as a Cas9 protein (spCas9) of *Streptococcus pyogenes* (*S.pyogenes*);
preferably, the first Cas protein has the amino acid sequence set forth in SEQ ID NO: 1.

3. The system or kit according to claim 1 or 2, wherein the first DNA polymerase is selected from the group consisting of DNA-dependent DNA polymerase and RNA-dependent DNA polymerase;
preferably, the first DNA polymerase is an RNA-dependent DNA polymerase;
preferably, the first DNA polymerase is a reverse transcriptase, such as a reverse transcriptase derived from Moloney murine leukemia virus, human immunodeficiency virus (HIV), avian sarcoma-leukemia virus (ASLV), Rous sarcoma virus (RSV), avian myeloblastosis virus (AMV), avian erythroblastosis virus helper virus, avian granulocytosis virus MC29 helper virus, avian reticuloendotheliosis virus helper virus, avian sarcoma virus UR2 helper virus, avian sarcoma virus Y73 helper virus, Rous-associated virus and myeloblastosis-associated virus (MAV);
preferably, the first DNA polymerase has the amino acid sequence set forth in SEQ ID NO: 4.

4. The system or kit according to any one of claims 1-3, wherein the first Cas protein is linked to the first DNA polymerase;
preferably, the first Cas protein is covalently linked to the first DNA polymerase with or without a linker;
preferably, the linker is a peptide linker, such as a flexible peptide linker; for example, the linker has the amino acid sequence set forth in SEQ ID NO: 51;
preferably, the first Cas protein is fused to the first DNA polymerase through a peptide linker or not through a peptide linker to form a first fusion protein;
preferably, the first Cas protein is linked or fused to the N-terminal of the first DNA polymerase optionally through a linker; or, the first Cas protein is linked or fused to the C-terminal of the first DNA polymerase optionally through a linker;
preferably, the first fusion protein has the amino acid sequence set forth in SEQ ID NO: 52.

5. The system or kit according to any one of claims 1-4, wherein the first gRNA comprises a first guide sequence, and, under conditions that allow hybridization or annealing of nucleic acids, the first guide sequence is capable of being hybridized or annealed to one nucleic acid strand of the first double-stranded target nucleic acid;
preferably, the first guide sequence has a length of at least 5nt, such as 5-10nt, 10-15nt, 15-20nt, 20-25nt, 25-30nt, 30-40nt, 40-50nt, 50-100nt, 100-200nt, or longer;
preferably, the first gRNA also comprises a first scaffold sequence, which can be recognized by and bound to the first Cas protein to form the first functional complex;
preferably, the first scaffold sequence has a length of at least 20nt, such as 20-30nt, 30-40nt, 40-50nt, 50-100nt, 100-200nt, or longer;
preferably, the first guide sequence is located at the upstream or 5' end of the first scaffold sequence;
preferably, the first functional complex is capable of fragmenting two strands of the first double-stranded target nucleic acid after the first guide sequence binds to the first double-stranded target nucleic acid.

6. The system or kit according to any one of claims 1-5, wherein, under conditions that allow hybridization or annealing of nucleic acids, the first target-binding sequence is capable of being hybridized or annealed to the 3' end of one nucleic acid strand of the target nucleic acid fragment, and the 3' end is formed by fragmenting the first double-stranded target nucleic acid by the first functional complex;
preferably, the first target-binding sequence has a length of at least 5nt, such as 5-10nt, 10-15nt, 15-20nt, 20-25nt, 25-30nt, 30-40nt, 40-50nt, 50-100nt, 100-200nt, or more long;
preferably, the first tag sequence has a length of at least 4nt, such as 4-10nt, 10-15nt, 15-20nt, 20-25nt, 25-30nt, 30-40nt, 40-50nt, 50-100nt, 100-200nt, or longer;
preferably, after the first target-binding sequence is hybridized or annealed to the 3' end of one nucleic acid strand of the target nucleic acid fragment, the first DNA polymerase is capable of extending the 3' end of the nucleic acid strand by using the first tag primer as a template; preferably, the extension forms a first overhang;
preferably, the first tag primer is a single-stranded deoxyribonucleic acid or a single-stranded ribonucleic acid;
preferably, the first tag primer is a single-stranded ribonucleic acid, and the first DNA polymerase is an RNA-dependent DNA polymerase; or, the first tag primer is a single-stranded deoxyribonucleic acid, and the first DNA polymerase is a DNA-dependent DNA polymerase;
preferably, the nucleic acid strand bound to the first guide sequence is different from the nucleic acid strand bound to the first target-binding sequence; preferably, the nucleic acid strand bound to the first guide sequence is an opposite strand of the nucleic acid strand bound to the first target-binding sequence.

7. The system or kit according to any one of claims 1-6, wherein the first tag primer is linked to the first gRNA;
preferably, the first tag primer is covalently linked to the first gRNA through a linker or not through a linker;
preferably, the first tag primer is optionally linked to the 3' end of the first gRNA through a linker;
preferably, the linker is a nucleic acid linker (e.g., a ribonucleic acid linker or a deoxyribonucleic acid linker);
preferably, the first tag primer is a single-stranded ribonucleic acid, and is ligated to the 3' end of the first gRNA through a ribonucleic acid linker or not through a ribonucleic acid linker to form a first PegRNA.

8. The system or kit according to any one of claims 1-7, which has one or more technical features selected from the following:
(1) the nucleic acid molecule A1 is capable of expressing the first Cas protein in a cell;
(2) the nucleic acid molecule B1 is capable of expressing the first DNA polymerase in a cell;
(3) the nucleic acid molecule C1 is capable of transcribing the first gRNA in a cell;
(4) the nucleic acid molecule D1 is capable of transcribing the first tag primer in a cell;
preferably, the nucleic acid molecule A1 is comprised in an expression vector (e.g., an eukaryotic expression vector), or the nucleic acid molecule A1 is an expression vector (e.g., an eukaryotic expression vector) comprising a nucleotide sequence encoding the first Cas protein;
preferably, the nucleic acid molecule B1 is comprised in an expression vector (e.g., an eukaryotic expression vector), or the nucleic acid molecule B 1 is an expression vector (e.g., an eukaryotic expression vector) comprising a nucleotide sequence encoding the first DNA polymerase;
preferably, the nucleic acid molecule C1 is comprised in an expression vector (e.g., an eukaryotic expression vector), or the nucleic acid molecule C1 is an expression vector (e.g., an eukaryotic expression vector) comprising a nucleotide sequence encoding the first gRNA;
preferably, the nucleic acid molecule D1 is comprised in an expression vector (e.g., an eukaryotic expression vector), or the nucleic acid molecule D1 is an expression vector (e.g., an eukaryotic expression vector) comprising a nucleotide sequence encoding the first tag primer;
preferably, the nucleic acid molecule A1 and the nucleic acid molecule B1 are comprised in the same or different expression vectors (e.g., eukaryotic expression vectors); preferably, the nucleic acid molecule A1 and the nucleic acid molecule B1 are capable of expressing the first Cas protein and the first DNA polymerase in isolation state in a cell, or capable of expressing a first fusion protein comprising the first Cas protein and the first DNA polymerase in a cell;
preferably, the nucleic acid molecule C1 and the nucleic acid molecule D1 are comprised in the same expression vector (e.g., an eukaryotic expression vector); preferably, the nucleic acid molecule C1 and the nucleic acid molecule D1 are capable of transcribing a first PegRNA comprising the first gRNA and the first tag primer in a cell; preferably, two, three or four of the nucleic acid molecules A1, B1, C1 and D1 are comprised in the same expression vector (e.g., an eukaryotic expression vector).

9. The system or kit according to any one of claims 1-8, wherein the system or kit comprises:
(M1-1) a first fusion protein comprising the first Cas protein and the first DNA polymerase, or, a nucleic acid molecule comprising a nucleotide sequence encoding the first fusion protein; or, (M1-2) the first Cas protein and the first DNA polymerase in isolation state, or, a nucleic acid molecule capable of expressing the first Cas protein and first DNA polymerase in isolation state; and,
(M2) a first PegRNA comprising the first gRNA and the first tag primer, or a nucleic acid molecule comprising a nucleotide sequence encoding the first PegRNA.

10. The system or kit according to any one of claims 1-9, wherein the system or kit further comprises:
(5) a second gRNA or a nucleic acid molecule C2 comprising a nucleotide sequence encoding the second gRNA, wherein the second gRNA is capable of binding to a second Cas protein and forming a second functional complex; the second functional complex is capable of fragmenting two strands of a second double-stranded target nucleic acid to form a target nucleic acid fragment;
preferably, the second Cas protein is the same as or different from the first Cas protein; preferably, the second Cas protein is the same as the first Cas protein;
preferably, the second gRNA comprises a second guide sequence, and under conditions that allow hybridization or annealing of nucleic acid, the second guide sequence is capable of being hybridized or annealed to one nucleic acid strand of the second double-stranded target nucleic acid;
preferably, after the second guide sequence binds to the second double-stranded target nucleic acid, the second functional complex fragments two strands of the second double-stranded target nucleic acid;
preferably, the second guide sequence is different from the first guide sequence;
preferably, the second double-stranded target nucleic acid is the same as or different from the first double-stranded target nucleic acid;
preferably, the second double-stranded target nucleic acid is the same as the first double-stranded target nucleic acid, and the second functional complex and the first functional complex fragment the same double-stranded target nucleic acid at different positions;
preferably, the second functional complex and the first functional complex fragment the same double-stranded target nucleic acid, and the nucleic acid strand bound to the first guide sequence is different from the nucleic acid bound to the second guide sequence; preferably, the nucleic acid strand bound to the first guide sequence is an opposite strand of the nucleic acid strand bound to the second guide sequence;
preferably, the second guide sequence has a length of at least 5nt, such as 5-10nt, 10-15nt, 15-20nt, 20-25nt, 25-30nt, 30-40nt, 40-50nt, 50-100nt, 100-200nt, or longer;
preferably, the second gRNA further comprises a second scaffold sequence, which is capable of being recognized by and bound to the second Cas protein to form the second functional complex;
preferably, the second scaffold sequence has a length of at least 20nt, such as 20-30nt, 30-40nt, 40-50nt, 50-100nt, 100-200nt, or longer;
preferably, the second scaffold sequence is the same as or different from the first scaffold sequence; preferably, the second scaffold sequence is the same as the first scaffold sequence;
preferably, the second guide sequence is located at the upstream or 5' end of the second scaffold sequence;
preferably, the nucleic acid molecule C2 is capable of transcribing the second gRNA in a cell;
preferably, the nucleic acid molecule C2 is comprised in an expression vector (e.g., an eukaryotic expression vector), or the nucleic acid molecule C2 is an expression vector (e.g., an eukaryotic expression vector) comprising a nucleotide sequence encoding the second gRNA.

11. The system or kit according to claim 10, wherein the second Cas protein is different from the first Cas protein; and the system or kit further comprises:
(6) the second Cas protein or a nucleic acid molecule A2 comprising a nucleotide sequence encoding the second Cas protein, wherein the second Cas protein is capable of cleaving or fragmenting a second double-stranded target nucleic acid;
preferably, the second Cas protein is capable of fragmenting the second double-stranded target nucleic acid to produce a sticky end or blunt end;
preferably, the second Cas protein is selected from the group consisting of Cas9 protein, Cas12a protein, cas12b protein, cas12c protein, cas12d protein, cas12e protein, cas12f protein, cas12g protein, cas12h protein, cas12i protein, cas14 protein, Cas 13a protein, Cas1 protein, Cas1B protein, Cas2 protein, Cas3 protein, Cas4 protein, Cas5 protein, Cas6 protein, Cas7 protein, Cas8 protein, Cas10 protein, Csy1 protein, Csy2 protein, Csy3 protein, Cse1 protein, Cse2 protein, Csc1 protein, Csc2 protein, Csa5 protein, Csn2 protein, Csm2 protein, Csm3 protein, Csm4 protein, Csm5 protein, Csm6 protein, Cmr1 protein, Cmr3 protein, Cmr4 protein, Cmr5 protein, Cmr6 protein, Csb1 protein, Csb2 protein, Csb3 protein, Csx17 protein, Csx14 protein, Csx10 protein, Csx16 protein, CsaX protein, Csx3 protein, Csx1 protein, Csx15 protein, Csf1 protein, Csf2 protein, Csf3 protein, Csf4 protein and homologue thereof or modified form thereof;
preferably, the second Cas protein is a Cas9 protein, such as a Cas9 protein (spCas9) of *Streptococcus pyogenes* (*S. pyogenes*);
preferably, the second Cas protein has the amino acid sequence set forth in SEQ ID NO: 1;
preferably, the nucleic acid molecule A2 is capable of expressing the second Cas protein in a cell;
preferably, the nucleic acid molecule A2 is comprised in an expression vector (e.g., an eukaryotic expression vector), or the nucleic acid molecule A2 is an expression vector (e.g., an eukaryotic expression vector) comprising a nucleotide sequence encoding the second Cas protein.

12. The system or kit according to any one of claims 1-11, wherein the system or kit further comprises:
(7) a second tag primer or a nucleic acid molecule D2 comprising a nucleotide sequence encoding the second tag primer, wherein the second tag primer comprises a second tag sequence and a second target-binding sequence, the second tag sequence is located at upstream or 5' end of the second target-binding sequence; and, under conditions that allow hybridization or annealing of nucleic acids, the second target-binding sequence is capable of being hybridized or annealed to the 3' end of one nucleic acid strand of the target nucleic acid fragment to form a double-stranded structure, and the second tag sequence does not bind to the target nucleic acid fragment and is in a free single-stranded state;
preferably, under conditions that allow hybridization or annealing of nucleic acids, the second target-binding sequence is capable of being hybridized or annealed to the 3' end of one nucleic acid strand of the target nucleic acid fragment, and the 3' end is formed by fragmenting the second double-stranded target nucleic acid by the second functional complex;
preferably, the second target-binding sequence has a length of at least 5nt, such as 5-10nt, 10-15nt, 15-20nt, 20-25nt, 25-30nt, 30-40nt, 40-50nt, 50-100nt, 100-200nt, or longer;
preferably, the second target-binding sequence is different from the first target-binding sequence; preferably, the nucleic acid strand bound to the second target-binding sequence is different from the nucleic acid strand bound to the first target-binding sequence; preferably, the nucleic acid strand bound to the second target-binding sequence is an opposite strand of the nucleic acid strand bound to the first target-binding sequence;
preferably, the second tag sequence has a length of at least 4nt, such as 4-10nt, 10-15nt, 15-20nt, 20-25nt, 25-30nt, 30-40nt, 40-50nt, 50-100nt, 100-200nt, or longer;
preferably, the second tag sequence is the same as or different from the first tag sequence; preferably, the second tag sequence is different from the first tag sequence;
preferably, after the second target-binding sequence is hybridized or annealed to the 3' end of one nucleic acid strand of the target nucleic acid fragment, a second DNA polymerase is capable of extending the 3' end of the nucleic acid strand by using the second tag primer as a template; preferably, the extension forms a second overhang;
preferably, the second DNA polymerase is the same as or different from the first DNA polymerase; preferably, the second DNA polymerase is the same as the first DNA polymerase;
preferably, the second tag primer is a single-stranded deoxyribonucleic acid or a single-stranded ribonucleic acid;
preferably, the second tag primer is a single-stranded ribonucleic acid, and the second DNA polymerase is an RNA-dependent DNA polymerase; or, the second tag primer is a single-stranded deoxyribonucleic acid, and the second DNA polymerase is a DNA-dependent DNA polymerase;
preferably, the nucleic acid strand bound to the second guide sequence is different from the nucleic acid strand bound to the second target-binding sequence; preferably, the nucleic acid strand bound to the second guide sequence is an opposite strand of the nucleic acid strand bound to the second target-binding sequence;
preferably, the second guide sequence and the first target-binding sequence bind to the same nucleic acid strand, and the binding position of the second guide sequence is located at upstream or 5' end of the binding position of the first target-binding sequence;
preferably, the first guide sequence and the second target-binding sequence bind to the same nucleic acid strand, and the binding position of the first guide sequence is located at upstream or 5' end of the binding position of the second target-binding sequence;
preferably, the first overhang and the second overhang are comprised in the same target nucleic acid fragment and are located on nucleic acid strands opposite to each other;
preferably, the nucleic acid molecule D2 is capable of transcribing the second tag primer in a cell;
preferably, the nucleic acid molecule D2 is comprised in an expression vector (e.g., an eukaryotic expression vector), or the nucleic acid molecule D2 is an expression vector (e.g., an eukaryotic expression vector) comprising a nucleotide sequence encoding the second tag primer.

13. The system or kit according to claim 12, wherein the second DNA polymerase is different from the first DNA polymerase; and the system or kit further comprises:
(8) the second DNA polymerase or a nucleic acid molecule B2 comprising a nucleotide sequence encoding the second DNA polymerase;
preferably, the second DNA polymerase is selected from the group consisting of DNA-dependent DNA polymerase and RNA-dependent DNA polymerase;
preferably, the second DNA polymerase is an RNA-dependent DNA polymerase;
preferably, the second DNA polymerase is a reverse transcriptase, such as a reverse transcriptase derived from Moloney murine leukemia virus, human immunodeficiency virus (HIV), avian sarcoma-leukemia virus (ASLV), Rous sarcoma virus (RSV), avian myeloblastosis virus (AMV), avian erythroblastosis virus helper virus, avian granulocytosis virus MC29 helper virus, avian reticuloendotheliosis virus helper virus, avian sarcoma virus UR2 helper virus, avian sarcoma virus Y73 helper virus, Rous-associated virus and myeloblastosis-associated virus (MAV);
preferably, the second DNA polymerase has the amino acid sequence set forth in SEQ ID NO: 4;
preferably, the nucleic acid molecule B2 is capable of expressing the second DNA polymerase in a cell;
preferably, the nucleic acid molecule B2 is comprised in an expression vector (e.g., an eukaryotic expression vector), or the nucleic acid molecule B2 is an expression vector (e.g., eukaryotic expression vectors) comprising a nucleotide sequence encoding the second DNA polymerase.

14. The system or kit according to claim 12 or 13, wherein the second tag primer is ligated to the second gRNA;
preferably, the second tag primer is covalently linked to the second gRNA through a linker or not through a linker;
preferably, the second tag primer is optionally linked to the 3' end of the second gRNA through a linker;
preferably, the linker is a nucleic acid linker (e.g., a ribonucleic acid linker or a deoxyribonucleic acid linker);
preferably, the second tag primer is a single-stranded ribonucleic acid, and is ligated to the 3' end of the second gRNA through a ribonucleic acid linker or not through a ribonucleic acid linker to form a second PegRNA;
preferably, the nucleic acid molecule C2 and the nucleic acid molecule D2 are comprised in the same expression vector (e.g., an eukaryotic expression vector); preferably, the nucleic acid molecule C2 and the nucleic acid molecule D2 are capable of transcribing a second PegRNA comprising the second gRNA and the second tag primer in a cell;
preferably, the system or kit comprises: a second PegRNA comprising the second gRNA and the second tag primer, or a nucleic acid molecule comprising a nucleotide sequence encoding the second PegRNA.

15. The system or kit according to claim 13 or 14, wherein the second Cas protein is isolated from or linked to the second DNA polymerase;
preferably, the second Cas protein is covalently linked to the second DNA polymerase with or without a linker;
preferably, the linker is a peptide linker, such as a flexible peptide linker; for example, the linker has the amino acid sequence set forth in SEQ ID NO: 51;
preferably, the second Cas protein is fused to the second DNA polymerase through a peptide linker or not through a peptide linker to form a second fusion protein;
preferably, the second Cas protein is optionally linked or fused to the N-terminal of the second DNA polymerase through a linker; or, the second Cas protein is optionally linked or fused to the C-terminal of the second DNA polymerase through a linker;
preferably, the second fusion protein has the amino acid sequence set forth in SEQ ID NO: 52;
preferably, the nucleic acid molecule A2 and the nucleic acid molecule B2 are comprised in the same or different expression vectors (e.g., eukaryotic expression vectors); preferably, the nucleic acid molecule A2 and the nucleic acid molecule B2 are capable of expressing the second Cas protein and the second DNA polymerase in isolation state in a cell, or capable of expressing a second fusion protein comprising the second Cas protein and the second DNA polymerase in a cell;
preferably, the system or kit comprises a second fusion protein comprising the second Cas protein and the second DNA polymerase, or a nucleic acid molecule comprising a nucleotide sequence encoding the second fusion protein; or, the second Cas protein and the second DNA polymerase in isolation state, or, a nucleic acid molecule capable of expressing the second Cas protein and the second DNA polymerase in isolation state;
preferably, the first and second Cas proteins are the same Cas protein, the first and second DNA polymerases are the same DNA polymerase; and, the system or kit comprises:
(M1-1) a first fusion protein comprising the first Cas protein and the first DNA polymerase, or, a nucleic acid molecule comprising a nucleotide sequence encoding the first fusion protein; or, (M1-2) the first Cas protein and the first DNA polymerase in isolation state, or a nucleic acid molecule capable of expressing the first Cas protein and the first DNA polymerase in isolation state;
(M2) a first PegRNA comprising the first gRNA and the first tag primer, or a nucleic acid molecule comprising a nucleotide sequence encoding the first PegRNA;
(M3) a second PegRNA comprising the second gRNA and the second tag primer, or, a nucleic acid molecule comprising a nucleotide sequence encoding the second PegRNA.

16. The system or kit according to any one of claims 1-15, wherein the system or kit further comprises a nucleic acid vector;
preferably, the nucleic acid vector is double-stranded;
preferably, the nucleic acid vector is a circular double-stranded vector;
preferably, the nucleic acid vector comprises a first guide binding sequence capable of being hybridized or annealed to the first guide sequence (e.g., a complementary sequence to the first guide sequence), and/or, a second guide binding sequence capable of being hybridized or annealed to the second guide sequence (e.g., a complementary sequence of the second guide sequence); optionally, the nucleic acid vector further comprises a restriction enzyme cleavage site between the first guide binding sequence and the second guide binding sequence;
preferably, the first guide binding sequence and the second guide binding sequence are located on an opposite strand of the nucleic acid vector;
preferably, the nucleic acid vector further comprises a first PAM sequence recognized by the first Cas protein, and/or a second PAM sequence recognized by the second Cas protein;
preferably, the first functional complex is capable of binding and fragmenting the nucleic acid vector through the first guide binding sequence and the first PAM sequence; and/or, the second functional complex is capable of binding and fragmenting the nucleic acid vector through the second guide binding sequence and the second PAM sequence.

17. The system or kit of claim 16, wherein the nucleic acid vector further comprises a gene of interest;
preferably, the gene of interest is located between the first guide binding sequence and the second guide binding sequence;
preferably, the first functional complex and the second functional complex cleave the nucleic acid vector, resulting in the production of a nucleic acid fragment comprising the gene of interest;
preferably, under conditions that allow hybridization or annealing of nucleic acids, the first tag primer is capable of being hybridized or annealed to the 3' end of one nucleic acid strand of the nucleic acid fragment through the first target-binding sequence to form a double-stranded structure, and, the first tag sequence of the first tag primer is in a free state; preferably, the nucleic acid strand to which the first target-binding sequence is hybridized or annealed is an opposite strand of the nucleic acid strand comprising the first guide binding sequence;
preferably, under conditions that allow hybridization or annealing of nucleic acids, the second tag primer is capable of being hybridized or annealed to the 3' end of one nucleic acid strand of the nucleic acid fragment through the second target-binding sequence to form a double-stranded structure, and, the second tag sequence of the second tag primer is in a free state; preferably, the nucleic acid strand to which the second target-binding sequence is hybridized or annealed is an opposite strand of the nucleic acid strand comprising the second guide binding sequence;
preferably, the nucleic acid strand to which the first target-binding sequence is hybridized or annealed is an opposite strand of the nucleic acid strand to which the second target-binding sequence is hybridized or annealed.

18. The system or kit according to claim 16 or 17, wherein the nucleic acid vector further comprises a first target sequence; wherein, under conditions that allow hybridization or annealing of nucleic acids, the first tag primer is capable of being hybridized or annealed to the first target sequence through the first target-binding sequence to form a double-stranded structure, and the first tag sequence of the first tag primer is in a free state; preferably, the first target sequence is located between the first guide binding sequence and the second guide binding sequence; preferably, the first target sequence is located on an opposite strand of the first guide binding sequence; preferably, after the nucleic acid vector is cleaved by the first functional complex, the nucleic acid strand comprising the first target sequence can be extended (preferably, forming a first overhang) by using the first tag primer annealed to the first target sequence as a template; preferably, the first functional complex cleaves the nucleic acid vector at a site located at the 3' end or 3' portion of the first target sequence; preferably, the first target sequence is located at the 3' end of one nucleic acid strand of the nucleic acid fragment comprising the gene of interest;
and / or,
the nucleic acid vector further comprises a second target sequence; wherein, under conditions that allow hybridization or annealing of nucleic acids, the second tag primer can hybridized or annealed to the second target sequence through the second target-binding sequence to form a double-stranded structure, and the second tag sequence of the second tag primer is in a free state; preferably, the second target sequence is located between the first guide binding sequence and the second guide binding sequence; preferably, the second target sequence is located at an opposite strand of the second guide binding sequence; preferably, after cleavage of the nucleic acid vector by the second functional complex, the nucleic acid strand comprising the second target sequence is capable of extending (preferably, forming a second overhang) by using the second tag primer annealed to the second target sequence as a template; preferably, the second functional complex cleaves the nucleic acid vector at a site located at the 3' end or 3' portion of the second target sequence; preferably, the second target sequence is located at the 3' end of one nucleic acid strand of the nucleic acid fragment comprising the gene of interest;
preferably, the nucleic acid strand comprising the first target sequence is located at an opposite strand of the nucleic acid strand comprising the second target sequence;
preferably, the nucleic acid vector further comprises a restriction enzyme cleavage site between the first target sequence and the second target sequence;
preferably, the nucleic acid vector further comprises a gene of interest between the first target sequence and the second target sequence.

19. The system or kit according to any one of claims 1-18, wherein the system or kit further comprises:
(9) a third gRNA or a nucleic acid molecule C3 comprising a nucleotide sequence encoding the third gRNA, wherein the third gRNA is capable of binding to a third Cas protein and forming a third functional complex; the third functional complex is capable of fragmenting two strands of a third double-stranded target nucleic acid to form fragmented nucleotide fragments a1 and a2;
preferably, the third Cas protein is the same as or different from the first Cas protein or the second Cas protein; preferably, the first, second and third Cas proteins are the same Cas protein;
preferably, the third gRNA comprises a third guide sequence, and, under conditions that allow hybridization or annealing of nucleic acids, the third guide sequence is capable of being hybridized or annealed to one nucleic acid strand of the third double-stranded target nucleic acid;
preferably, the third functional complex fragments two strands of the third double-stranded target nucleic acid after the third guide sequence binds to the third double-stranded target nucleic acid;
preferably, the third guide sequence is the same as or different from the first guide sequence or the second guide sequence; preferably, the first, second and third guide sequences are different from each other;
preferably, the third double-stranded target nucleic acid is the same as or different from the first double-stranded target nucleic acid or the second double-stranded target nucleic acid; preferably, the second double-stranded target nucleic acid is the same as the first double-stranded target nucleic acid, and the third double-stranded target nucleic acid is different from the first and second double-stranded target nucleic acids; preferably, the third double-stranded target nucleic acid is a genomic DNA;
preferably, the third guide sequence has a length of at least 5nt, such as 5-10nt, 10-15nt, 15-20nt, 20-25nt, 25-30nt, 30-40nt, 40-50nt, 50-100nt, 100-200nt, or longer;
preferably, the third gRNA further comprises a third scaffold sequence, which is capable of being recognized by and bound to the third Cas protein to form a third functional complex;
preferably, the third scaffold sequence has a length of at least 20nt, such as 20-30nt, 30-40nt, 40-50nt, 50-100nt, 100-200nt, or longer;
preferably, the third scaffold sequence is the same as or different from the first scaffold sequence or the second scaffold sequence; preferably, the first, second and third scaffold sequences are the same;
preferably, the third guide sequence is located at the upstream or 5' end of the third scaffold sequence;
preferably, the nucleic acid molecule C3 is capable of transcribing the third gRNA in a cell;
preferably, the nucleic acid molecule C3 is comprised in an expression vector (e.g., an eukaryotic expression vector), or the nucleic acid molecule C3 is an expression vector (e.g., an eukaryotic expression vector) comprising a nucleotide sequence encoding the third gRNA.

20. The system or kit according to claim 19, wherein the third functional complex is capable of fragmenting two strands of the third double-stranded target nucleic acid to form fragmented nucleotide fragments a1 and a2;
preferably, under conditions that allow hybridization or annealing of nucleic acid, the first tag sequence or complementary sequence thereof or the first overhang is capable of being hybridized or annealed to the fragmented nucleotide fragment a1;
preferably, the first tag sequence or complementary sequence thereof or the first overhang is capable of being hybridized or annealed to the fragmented nucleotide fragment a1 at the end formed by fragmenting the third double-stranded target nucleic acid by the third functional complex;
preferably, the complementary sequence of the first tag sequence or the first overhang is capable of being hybridized or annealed to the 3' end or 3' portion of one nucleic acid strand of the fragmented nucleotide fragment a1, and the 3' end or 3' portion is formed by fragmenting the third double-stranded target nucleic acid by the third functional complex;
preferably, the complementary sequence of the first tag sequence or the first overhang is capable of being hybridized or annealed to the 3' portion of one nucleic acid strand of the fragmented nucleotide fragment a1, and there is a first spacer region between the 3' portion of the nucleotide fragment a1 and the cleaved end formed by the third double-stranded target nucleic acid;
preferably, the first spacer region has a length of 1nt to 200nt, such as 1-10nt, 10-20nt, 20-30nt, 30-40nt, 40-50nt, 50-100nt or 100-200nt;
preferably, under conditions that allow hybridization or annealing of nucleic acids, the second tag sequence or complementary sequence thereof or the second overhang is capable of being hybridized or annealed to the fragmented nucleotide fragment a2;
preferably, the second tag sequence or complementary sequence thereof or the second overhang is capable of being hybridized or annealed to the fragmented nucleotide fragment a2 at the end formed by fragmenting the third double-stranded target nucleic acid by the third functional complex;
preferably, the complementary sequence of the second tag sequence or the second overhang is capable of being hybridized or annealed to the 3' end or 3' portion of one nucleic acid strand of the fragmented nucleotide fragment a2, and the 3' end or 3' portion is formed by fragmenting the third double-stranded target nucleic acid by the third functional complex;
preferably, the complementary sequence of the second tag sequence or the second overhang is capable of being hybridized or annealed to the 3' portion of one nucleic acid strand of the fragmented nucleotide fragment a2, and there is a second spacer region between the 3' portion of the nucleotide fragment a2 and the cleaved end formed by the third double-stranded target nucleic acid;
preferably, the second spacer region has a length of 1 nt to 200 nt, such as 1-10 nt, 10-20 nt, 20-30 nt, 30-40 nt, 40-50 nt, 50-100 nt or 100-200 nt.

21. The system or kit according to claim 19 or 20, wherein the third Cas protein is different from the first Cas protein or the second Cas protein; and the system or kit further comprises:
(10) the third Cas protein or a nucleic acid molecule A3 comprising a nucleotide sequence encoding the third Cas protein, wherein the third Cas protein is capable of cleaving or fragmenting the third double-stranded target nucleic acid;
preferably, the third Cas protein is selected from the group consisting of Cas9 protein, Cas12a protein, cas12b protein, cas12c protein, cas12d protein, cas12e protein, cas12f protein, cas12g protein, cas12h protein, cas12i protein, cas14 protein, Cas13a protein, Cas1 protein, Cas1B protein, Cas2 protein, Cas3 protein, Cas4 protein, Cas5 protein, Cas6 protein, Cas7 protein, Cas8 protein, Cas10 protein, Csy1 protein, Csy2 protein, Csy3 protein, Cse1 protein, Cse2 protein, Csc1 protein, Csc2 protein, Csa5 protein, Csn2 protein, Csm2 protein, Csm3 protein, Csm4 protein, Csm5 protein, Csm6 protein, Cmr1 protein, Cmr3 protein, Cmr4 protein, Cmr5 protein, Cmr6 protein, Csb1 protein, Csb2 protein, Csb3 protein, Csx17 protein, Csx14 protein, Csx10 protein, Csx16 protein, CsaX protein, Csx3 protein, Csx1 protein, Csx15 protein, Csf1 protein, Csf2 protein, Csf3 protein, Csf4 protein and homologue thereof or modified form thereof;
preferably, the third Cas protein is capable of fragmenting the third double-stranded target nucleic acid and producing a sticky end or blunt end;
preferably, the third Cas protein is a Cas9 protein, such as a Cas9 protein (spCas9) of *Streptococcus pyogenes (S. pyogenes);*
preferably, the third Cas protein has the amino acid sequence set forth in SEQ ID NO: 1;
preferably, the nucleic acid molecule A3 is capable of expressing the third Cas protein in a cell;
preferably, the nucleic acid molecule A3 is comprised in an expression vector (e.g., an eukaryotic expression vector), or the nucleic acid molecule A3 is an expression vector (e.g., an eukaryotic expression vector) comprising a nucleotide sequence encoding the third Cas protein.

22. The system or kit according to claim 19 or 20, wherein the first, second and third Cas proteins are the same Cas protein, and the first and second DNA polymerases are the same DNA polymerase; and, the system or kit comprises:
(M1-1) a first fusion protein comprising the first Cas protein and the first DNA polymerase, or, a nucleic acid molecule comprising a nucleotide sequence encoding the first fusion protein; or, (M1-2) the first Cas protein and the first DNA polymerase in isolation state, or a nucleic acid molecule capable of expressing the first Cas protein and the first DNA polymerase in isolation state;
(M2) a first PegRNA comprising the first gRNA and the first tag primer, or a nucleic acid molecule comprising a nucleotide sequence encoding the first PegRNA;
(M3) a second PegRNA comprising the second gRNA and the second tag primer, or a nucleic acid molecule comprising a nucleotide sequence encoding the second PegRNA;
(M4) the third gRNA or a nucleic acid molecule comprising a nucleotide sequence encoding the third gRNA;
preferably, the system or kit further comprises: a nucleic acid vector as defined in any one of claims 16-18.

23. The system or kit according to any one of claims 19-22, wherein the system or kit further comprises:
(11) a third tag primer or a nucleic acid molecule D3 comprising a nucleotide sequence encoding the third tag primer, wherein the third tag primer comprises a third tag sequence and a third target-binding sequence, the third tag sequence is located at the upstream or 5' end of the third target-binding sequence; and, under conditions that allow hybridization or annealing of nucleic acids, the third target-binding sequence is capable of being hybridized or annealed to the 3' end of one nucleic acid strand of the fragmented nucleotide fragment a1 or a2 to form a double-stranded structure, and, the third tag sequence does not bind to the nucleotide fragment a1 or a2, and is in a free single-stranded state;
preferably, under conditions that allow hybridization or annealing of nucleic acids, the third target-binding sequence is capable of being hybridized or annealed to the 3' end of one nucleic acid strand of the fragmented nucleotide fragment a1 or a2, and the 3' end is formed by fragmenting the third double-stranded target nucleic acid by the third functional complex;
preferably, the nucleic acid strand bound to the third target-binding sequence is different from the nucleic acid strand bound to the third guide sequence; preferably, the nucleic acid strand bound to the third target-binding sequence is an opposite strand of the nucleic acid strand bound to the third guide sequence;
preferably, the third target-binding sequence has a length of at least 5nt, such as 5-10nt, 10-15nt, 15-20nt, 20-25nt, 25-30nt, 30-40nt, 40-50nt, 50-100nt, 100-200nt, or longer;
preferably, the third target-binding sequence is different from the first or second target-binding sequence;
preferably, after the third target-binding sequence is hybridized or annealed to the 3' end of one nucleic acid strand of the fragmented nucleotide fragment a1 or a2, the third DNA polymerase is capable of extending the 3' end of the nucleic acid strand by using the third tag primer as a template; preferably, the extension forms a third overhang;
preferably, the third tag sequence has a length of at least 4nt, such as 4-10nt, 10-15nt, 15-20nt, 20-25nt, 25-30nt, 30-40nt, 40-50nt, 50-100nt, 100-200nt, or longer;
preferably, the third tag sequence is the same as or different from the first or second tag sequence; preferably, the third tag sequence is different from the first or second tag sequence;
preferably, under conditions that allow hybridization or annealing of nucleic acids, the complementary sequence of the third tag sequence or the third overhang is capable of being hybridized or annealed to the nucleic acid strand comprising the first overhang or the second overhang; preferably, the complementary sequence of the third tag sequence or the third overhang is hybridized or annealed to the first overhang or the second overhang or its upstream nucleotide sequence;
preferably, the third DNA polymerase is the same as or different from the first or second DNA polymerase; preferably, the first, second and third DNA polymerases are the same DNA polymerase;
preferably, the third tag primer is a single-stranded deoxyribonucleic acid or a single-stranded ribonucleic acid;
preferably, the third tag primer is a single-stranded ribonucleic acid, and the third DNA polymerase is an RNA-dependent DNA polymerase; or, the third tag primer is a single-stranded deoxyribonucleic acid, and the third DNA polymerase is a DNA-dependent DNA polymerase;
preferably, the nucleic acid molecule D3 is capable of transcribing the third tag primer in a cell;
preferably, the nucleic acid molecule D3 is comprised in an expression vector (e.g., an eukaryotic expression vector), or the nucleic acid molecule D3 is an expression vector (e.g., an eukaryotic expression vector) comprising a nucleotide sequence encoding the third tag primer.

24. The system or kit according to claim 23, wherein the third DNA polymerase is different from the first or second DNA polymerase; and the system or kit further comprises:
(12) the third DNA polymerase or a nucleic acid molecule B3 comprising a nucleotide sequence encoding the third DNA polymerase;
preferably, the third DNA polymerase is selected from the group consisting of DNA-dependent DNA polymerase and RNA-dependent DNA polymerase;
preferably, the third DNA polymerase is an RNA-dependent DNA polymerase;
preferably, the third DNA polymerase is a reverse transcriptase, for example a reverse transcriptase derived from Moloney murine leukemia virus, human immunodeficiency virus (HIV), avian sarcoma-leukemia virus (ASLV), Rous sarcoma virus (RSV), avian myeloblastosis virus (AMV), avian erythroblastosis virus helper virus, avian granulocytosis virus MC29 helper virus, avian reticuloendotheliosis virus helper virus, avian sarcoma virus UR2 helper virus, avian sarcoma virus Y73 helper virus, Rous-associated virus and myeloblastosis-associated virus (MAV);
preferably, the third DNA polymerase has the amino acid sequence set forth in SEQ ID NO: 4;
preferably, the nucleic acid molecule B3 is capable of expressing the third DNA polymerase in a cell;
preferably, the nucleic acid molecule B3 is comprised in an expression vector (e.g., an eukaryotic expression vector), or the nucleic acid molecule B3 is an expression vector (e.g., eukaryotic expression vectors) comprising a nucleotide sequence encoding the third DNA polymerase.

25. The system or kit according to claim 23 or 24, wherein the third tag primer is ligated to the third gRNA;
preferably, the third tag primer is covalently linked to the third gRNA through a linker or not through a linker;
preferably, the third tag primer is optionally linked to the 3' end of the third gRNA through a linker;
preferably, the linker is a nucleic acid linker (e.g., a ribonucleic acid linker or a deoxyribonucleic acid linker);
preferably, the third tag primer is a single-stranded ribonucleic acid, and is linked to the 3' end of the third gRNA through a ribonucleic acid linker or not through a ribonucleic acid linker to form a third PegRNA;
preferably, the nucleic acid molecule C3 and the nucleic acid molecule D3 are comprised in the same expression vector (e.g., an eukaryotic expression vector); preferably, the nucleic acid molecule C3 and the nucleic acid molecule D3 are capable of transcribing the third PegRNA comprising the third gRNA and the third tag primer in a cell;
preferably, the system or kit comprises: a third PegRNA comprising the third gRNA and the third tag primer, or a nucleic acid molecule comprising a nucleotide sequence encoding the third PegRNA.

26. The system or kit according to claim 24 or 25, wherein the third Cas protein is isolated from or linked to the third DNA polymerase;
preferably, the third Cas protein is covalently linked to the third DNA polymerase through a linker or without a linker;
preferably, the linker is a peptide linker, such as a flexible peptide linker; for example, the linker has the amino acid sequence set forth in SEQ ID NO: 51;
preferably, the third Cas protein is fused to the third DNA polymerase through a peptide linker or not through a peptide linker to form a third fusion protein;
preferably, the third Cas protein is optionally linked or fused to the N-terminal of the third DNA polymerase through a linker; or, the third Cas protein is optionally linked or fused to the C-terminal of the third DNA polymerase through a linker;
preferably, the third fusion protein has the amino acid sequence set forth in SEQ ID NO: 52;
preferably, the nucleic acid molecule A3 and the nucleic acid molecule B3 are comprised in the same or different expression vectors (e.g., eukaryotic expression vectors); preferably, the nucleic acid molecule A3 and the nucleic acid molecule B3 are capable of expressing the third Cas protein and the third DNA polymerase in isolation state in a cell, or capable of expressing a third fusion protein comprising the third Cas protein and the third DNA polymerase in a cell;
preferably, the system or kit comprises a third fusion protein comprising the third Cas protein and the third DNA polymerase, or a nucleic acid molecule comprising a nucleotide sequence encoding the third fusion protein; or, the third Cas protein and the third DNA polymerase in isolation state, or, a nucleic acid molecule capable of expressing the third Cas protein and the third DNA polymerase in isolation state.

27. The system or kit according to claim 23 or 25, wherein the first, second and third Cas proteins are the same Cas protein, and the first, second and third DNA polymerases are the same DNA polymerase; and, the system or kit comprises:
(M1-1) a first fusion protein comprising the first Cas protein and the first DNA polymerase, or, a nucleic acid molecule comprising a nucleotide sequence encoding the first fusion protein; or, (M1-2) the first Cas protein and the first DNA polymerase in isolated state, or a nucleic acid molecule capable of expressing the first Cas protein and the first DNA polymerase in isolated state;
(M2) a first PegRNA comprising the first gRNA and the first tag primer, or a nucleic acid molecule comprising a nucleotide sequence encoding the first PegRNA;
(M3) a second PegRNA comprising the second gRNA and the second tag primer, or a nucleic acid molecule comprising a nucleotide sequence encoding the second PegRNA;
(M4) a third PegRNA comprising the third gRNA and the third tag primer, or a nucleic acid molecule comprising a nucleotide sequence encoding the third PegRNA;
preferably, the system or kit further comprises: a nucleic acid vector as defined in any one of claims 16-18.

28. The system or kit according to any one of claims 1-27, wherein the system or kit further comprises:
(13) a fourth gRNA or a nucleic acid molecule C4 comprising a nucleotide sequence encoding the fourth gRNA, wherein the fourth gRNA is capable of binding to the fourth Cas protein and forming a fourth functional complex; the fourth functional complex is capable of fragmenting two strands of a fourth double-stranded target nucleic acid to form target nucleic acid fragments b1 and b2;
preferably, the fourth Cas protein is the same as or different from the first, second or third Cas protein; preferably, the first, second, third and fourth Cas proteins are the same Cas protein;
preferably, the fourth gRNA comprises a fourth guide sequence, and, under conditions that allow hybridization or annealing of nucleic acids, the fourth guide sequence is capable of being hybridized or annealed to one nucleic acid strand of the fourth double-stranded target nucleic acid;
preferably, after the fourth guide sequence binds to the fourth double-stranded target nucleic acid, the fourth functional complex fragments two strands of the fourth double-stranded target nucleic acid;
preferably, the fourth guide sequence is the same as or different from the first, second or third guide sequence; preferably, the first, second, third and fourth guide sequences are different from each other;
preferably, the fourth double-stranded target nucleic acid is the same as or different from the first, second or third double-stranded target nucleic acid; preferably, the second double-stranded target nucleic acid is the same as the first double-stranded target nucleic acid, and the fourth double-stranded target nucleic acid is the same as the third double-stranded target nucleic acid, but different from the first or second double-stranded target nucleic acid; preferably, the fourth functional complex and the third functional complex fragment the same double-stranded target nucleic acid at different positions;
preferably, the fourth functional complex and the third functional complex fragment the same double-stranded target nucleic acid, and the nucleic acid strand bound to the fourth guide sequence is different from the nucleic acid strand bound to the third guide sequence; preferably, the nucleic acid strand bound to the fourth guide sequence is an opposite strand of the nucleic acid strand bound to the third guide sequence;
preferably, the fourth double-stranded target nucleic acid is a genomic DNA;
preferably, the fourth guide sequence has a length of at least 5nt, such as 5-10nt, 10-15nt, 15-20nt, 20-25nt, 25-30nt, 30-40nt, 40-50nt, 50-100nt, 100-200nt, or longer;
preferably, the fourth gRNA further comprises a fourth scaffold sequence, which is capable of being recognized by and bound to the fourth Cas protein, thereby forming a fourth functional complex;
preferably, the fourth scaffold sequence has a length of at least 20nt, such as 20-30nt, 30-40nt, 40-50nt, 50-100nt, 100-200nt, or longer;
preferably, the fourth scaffold sequence is the same as or different from the first, second or third scaffold sequence; preferably, the first, second, third and fourth scaffold sequences are the same;
preferably, the fourth guide sequence is located at the upstream or 5' end of the fourth scaffold sequence;
preferably, the nucleic acid molecule C4 is capable of transcribing the fourth gRNA in a cell;
preferably, the nucleic acid molecule C4 is comprised in an expression vector (e.g., an eukaryotic expression vector), or the nucleic acid molecule C4 is an expression vector (e.g., an eukaryotic expression vector) comprising a nucleotide sequence encoding the fourth gRNA.

29. The system or kit according to claim 28, wherein the fourth double-stranded target nucleic acid is the same as the third double-stranded target nucleic acid, and the third and fourth functional complexes fragment the same double-stranded target nucleic acid at different positions to form fragmented nucleotide fragments a1, a2 and a3; wherein, prior to the fragmentation, in the same double-stranded target nucleic acid, the nucleotide fragments a1, a2 and a3 are arranged in sequence (i.e., the nucleotide fragment a1 is linked to the nucleotide fragment a3 via the nucleotide fragment a2); preferably, the third and fourth functional complexes result in the isolation of the nucleotide fragments a1 and a2, and the isolation of the nucleotide fragments a2 and a3, respectively;
preferably, under conditions that allow hybridization or annealing of nucleic acids, the first tag sequence or complementary sequence thereof or the first overhang is capable of being hybridized or annealed to the fragmented nucleotide fragment a1; preferably, the first tag sequence or complementary sequence thereof or the first overhang is capable of being hybridized or annealed to the fragmented nucleotide fragment a1 at the end formed by fragmenting the third double-stranded target nucleic acid by the third functional complex; preferably, the complementary sequence of the first tag sequence or the first overhang is capable of being hybridized or annealed to the 3' end or 3' portion of one nucleic acid strand of the fragmented nucleotide fragment a1, and the 3' end or 3' portion is formed by fragmenting the third double-stranded target nucleic acid by the third functional complex;
preferably, under conditions that allow hybridization or annealing of nucleic acids, the second tag sequence or complementary sequence thereof or the second overhang is capable of being hybridized or annealed to the fragmented nucleotide fragment a3; preferably, the second tag sequence or complementary sequence thereof or the second overhang is capable of being hybridized or annealed to the fragmented nucleotide fragment a3 at the end formed by fragmenting the third double-stranded target nucleic acid by the fourth functional complex; preferably, the complementary sequence of the second tag sequence or the second overhang is capable of being hybridized or annealed to the 3' end or 3' portion of one nucleic acid strand of the fragmented nucleotide fragment a3, and the 3' end or 3' portion is formed by fragmenting the third double-stranded target nucleic acid by the fourth functional complex.

30. The system or kit according to claim 28 or 29, wherein the fourth Cas protein is different from the first, second or third Cas protein; and the system or kit further comprises:
(14) the fourth Cas protein or a nucleic acid molecule A4 comprising a nucleotide sequence encoding the fourth Cas protein, wherein the fourth Cas protein is capable of cleaving or fragmenting the fourth double-stranded target nucleic acid;
preferably, the fourth Cas protein is selected from the group consisting of Cas9 protein, Cas 12a protein, cas12b protein, cas12c protein, cas12d protein, cas12e protein, cas12f protein, cas12g protein, cas12h protein, cas12i protein, cas14 protein, Cas13a protein, Cas1 protein, Cas1B protein, Cas2 protein, Cas3 protein, Cas4 protein, Cas5 protein, Cas6 protein, Cas7 protein, Cas8 protein, Cas10 protein, Csy1 protein, Csy2 protein, Csy3 protein, Cse1 protein, Cse2 protein, Csc1 protein, Csc2 protein, Csa5 protein, Csn2 protein, Csm2 protein, Csm3 protein, Csm4 protein, Csm5 protein, Csm6 protein, Cmr1 protein, Cmr3 protein, Cmr4 protein, Cmr5 protein, Cmr6 protein, Csb1 protein, Csb2 protein, Csb3 protein, Csx17 protein, Csx14 protein, Csx10 protein, Csx16 protein, CsaX protein, Csx3 protein, Csx1 protein, Csx15 protein, Csf1 protein, Csf2 protein, Csf3 protein, Csf4 protein and their homologue thereof or modified form thereof;
preferably, the fourth Cas protein is capable of fragmenting the fourth double-stranded target nucleic acid to produce a sticky end or blunt end;
preferably, the fourth Cas protein is a Cas9 protein, such as a Cas9 protein (spCas9) of *Streptococcus pyogenes* (*S. pyogenes*);
preferably, the fourth Cas protein has the amino acid sequence set forth in SEQ ID NO: 1;
preferably, the nucleic acid molecule A4 is capable of expressing the fourth Cas protein in a cell;
preferably, the nucleic acid molecule A4 is comprised in an expression vector (e.g., an eukaryotic expression vector), or the nucleic acid molecule A4 is an expression vector (e.g., an eukaryotic expression vector) comprising a nucleotide sequence encoding the fourth Cas protein.

31. The system or kit according to claim 28 or 29, wherein the first, second, third and fourth Cas proteins are the same Cas protein, the first and second DNA polymerases (and optionally the third DNA polymerase) are the same DNA polymerase; and, the system or kit comprises:
(M1-1) a first fusion protein comprising the first Cas protein and the first DNA polymerase, or, a nucleic acid molecule comprising a nucleotide sequence encoding the first fusion protein; or, (M1-2) the first Cas protein and the first DNA polymerase in isolation state, or a nucleic acid molecule capable of expressing the first Cas protein and the first DNA polymerase in isolation state;
(M2) a first PegRNA comprising the first gRNA and the first tag primer, or a nucleic acid molecule comprising a nucleotide sequence encoding the first PegRNA;
(M3) a second PegRNA comprising the second gRNA and the second tag primer, or a nucleic acid molecule comprising a nucleotide sequence encoding the second PegRNA;
(M4) the third gRNA or a nucleic acid molecule comprising a nucleotide sequence encoding the third gRNA; or, a third PegRNA comprising the third gRNA and the third tag primer, or, a nucleic acid molecule comprising a nucleotide sequence encoding the third pegRNA;
(M5) the fourth gRNA or a nucleic acid molecule comprising a nucleotide sequence encoding the fourth gRNA;
preferably, the system or kit further comprises: a nucleic acid vector as defined in any one of claims 16-18.

32. The system or kit according to any one of claims 28-31, wherein the system or kit further comprises:
(15) a fourth tag primer or a nucleic acid molecule D4 comprising a nucleotide sequence encoding the fourth tag primer, wherein the fourth tag primer comprises a fourth tag sequence and a fourth target-binding sequence, the fourth tag sequence is located at the upstream or 5' end of the fourth target-binding sequence; and, under conditions that allow hybridization or annealing of nucleic acids, the fourth target-binding sequence is capable of being hybridized or annealed to the 3' end of one nucleic acid strand of the target nucleic acid fragment b1 or b2, to form a double-stranded structure, and the fourth tag sequence does not bind to the target nucleic acid fragment b1 or b2, and is in a free single-stranded state;
preferably, under conditions that allow hybridization or annealing of nucleic acids, the fourth target-binding sequence is capable of being hybridized or annealed to the 3' end of one nucleic acid strand of the target nucleic acid fragment b1 or b2, and the 3' end is formed by fragmenting the fourth double-stranded target nucleic acid by the fourth functional complex;
preferably, the nucleic acid strand bound to the fourth target-binding sequence is different from the nucleic acid strand bound to the fourth guide sequence; preferably, the nucleic acid strand bound to the fourth target-binding sequence is an opposite strand of the nucleic acid strand bound to the fourth guide sequence;
preferably, the fourth target-binding sequence has a length of at least 5nt, such as 5-10nt, 10-15nt, 15-20nt, 20-25nt, 25-30nt, 30-40nt, 40-50nt, 50-100nt, 100-200nt, or longer;
preferably, the fourth target-binding sequence is different from the first, second or third target-binding sequence; preferably, the nucleic acid strand bound to the fourth target-binding sequence is different from the nucleic acid strand bound to the third target-binding sequence; preferably, the nucleic acid strand bound to the fourth target-binding sequence is an opposite strand of the nucleic acid strand bound to the third target-binding sequence;
preferably, after the fourth target-binding sequence is hybridized or annealed to the 3' end of one nucleic acid strand of the target nucleic acid fragment b1 or b2, the fourth DNA polymerase is capable of extending the 3' end of the nucleic acid strand by using the fourth tag primer as a template; preferably, the extension forms a fourth overhang;
preferably, the fourth tag sequence has a length of at least 4nt, such as 4-10nt, 10-15nt, 15-20nt, 20-25nt, 25-30nt, 30-40nt, 40-50nt, 50-100nt, 100-200nt, or longer;
preferably, the fourth tag sequence is the same as or different from the first, second or third tag sequence; preferably, the fourth tag sequence is different from the first, second or third tag sequence;
preferably, the fourth DNA polymerase is the same as or different from the first, second or third DNA polymerase; preferably, the first, second, third and fourth DNA polymerases are the same DNA polymerase;
preferably, the fourth tag primer is a single-stranded deoxyribonucleic acid or a single-stranded ribonucleic acid;
preferably, the fourth tag primer is a single-stranded ribonucleic acid, and the fourth DNA polymerase is an RNA-dependent DNA polymerase; or, the fourth tag primer is a single-stranded deoxyribonucleic acid, and the fourth DNA polymerase is a DNA-dependent DNA polymerase;
preferably, the fourth guide sequence and the third target-binding sequence bind to the same nucleic acid strand, and the binding position of the third target-binding sequence is located at the upstream or 5' end of the binding position of the fourth guide sequence;
preferably, the third guide sequence and the fourth target-binding sequence bind to the same nucleic acid strand, and the binding position of the fourth target-binding sequence is located at the upstream or 5' end of the binding position of the third guide sequence;
preferably, the third overhang and the fourth overhang are comprised in different target nucleic acid fragments, and preferably, are located at nucleic acid strands opposite to each other;
preferably, under conditions that allow hybridization or annealing of nucleic acids, the complementary sequence of the fourth tag sequence or the fourth overhang is capable of being hybridized or annealed to the nucleic acid strand comprising the first overhang or the second overhang; preferably, the complementary sequence of the fourth tag sequence or the fourth overhang is capable of being hybridized or annealed to the first overhang or the second overhang or its upstream nucleotide sequence;
preferably, under conditions that allow hybridization or annealing of nucleic acids, the complementary sequence of the third tag sequence or the third overhang is capable of being hybridized or annealed to the first overhang or a nucleotide sequence at the upstream thereof, and, the complementary sequence of the fourth tag sequence or the fourth overhang is capable of being hybridized or annealed to the second overhang or its upstream nucleotide sequence; or, under conditions that allow hybridization or annealing of nucleic acids, the complementary sequence of the third tag sequence or the third overhang is capable of being hybridized or annealed to the second overhang or its upstream nucleotide sequence, and the complementary sequence of the fourth tag sequence or the fourth overhang is capable of being hybridized or annealed to the first overhang or a nucleotide sequence at the upstream thereof;
preferably, the nucleic acid molecule D4 is capable of transcribing the fourth tag primer in a cell;
preferably, the nucleic acid molecule D4 is comprised in an expression vector (e.g., an eukaryotic expression vector), or the nucleic acid molecule D4 is an expression vector (e.g., an eukaryotic expression vector) comprising a nucleotide sequence encoding the fourth tag primer.

33. The system or kit according to claim 32, wherein the fourth DNA polymerase is different from the first, second or third DNA polymerase; and the system or kit further comprises:
(16) the fourth DNA polymerase or a nucleic acid molecule B4 comprising a nucleotide sequence encoding the fourth DNA polymerase;
preferably, the fourth DNA polymerase is selected from the group consisting of DNA-dependent DNA polymerase and RNA-dependent DNA polymerase;
preferably, the fourth DNA polymerase is an RNA-dependent DNA polymerase;
preferably, the fourth DNA polymerase is a reverse transcriptase, such as a reverse transcriptase derived from Moloney murine leukemia virus, human immunodeficiency virus (HIV), avian sarcoma-leukemia virus (ASLV), Rous sarcoma virus (RSV), avian myeloblastosis virus (AMV), avian erythroblastosis virus helper virus, avian granulocytosis virus MC29 helper virus, avian reticuloendotheliosis virus helper virus, avian sarcoma virus UR2 helper virus, avian sarcoma virus Y73 helper virus, Rous-associated virus and myeloblastosis-associated virus (MAV);
preferably, the fourth DNA polymerase has the amino acid sequence set forth in SEQ ID NO: 4;
preferably, the nucleic acid molecule B4 is capable of expressing the fourth DNA polymerase in a cell;
preferably, the nucleic acid molecule B4 is comprised in an expression vector (e.g., an eukaryotic expression vector), or the nucleic acid molecule B4 is an expression vector (e.g., eukaryotic expression vectors) comprising a nucleotide sequence encoding the fourth DNA polymerase.

34. The system or kit according to claim 32 or 33, wherein the fourth tag primer is ligated to the fourth gRNA;
preferably, the fourth tag primer is covalently linked to the fourth gRNA through a linker or not through a linker;
preferably, the fourth tag primer is optionally linked to the 3' end of the fourth gRNA through a linker;
preferably, the linker is a nucleic acid linker (e.g., a ribonucleic acid linker or a deoxyribonucleic acid linker);
preferably, the fourth tag primer is a single-stranded ribonucleic acid, and is ligated to the 3' end of the fourth gRNA through a ribonucleic acid linker or not through a ribonucleic acid linker to form a fourth PegRNA;
preferably, the nucleic acid molecule C4 and the nucleic acid molecule D4 are comprised in the same expression vector (e.g., an eukaryotic expression vector); preferably, the nucleic acid molecule C4 and the nucleic acid molecule D4 are capable of transcribing a fourth PegRNA comprising the fourth gRNA and the fourth tag primer in a cell;
preferably, the system or kit comprises: a fourth PegRNA comprising the fourth gRNA and the fourth tag primer, or a nucleic acid molecule comprising a nucleotide sequence encoding the fourth PegRNA.

35. The system or kit according to claim 33 or 34, wherein the fourth Cas protein is isolated from or linked to the fourth DNA polymerase;
preferably, the fourth Cas protein is covalently linked to the fourth DNA polymerase through a linker or not through a linker;
preferably, the linker is a peptide linker, such as a flexible peptide linker; for example, the linker has the amino acid sequence set forth in SEQ ID NO: 51;
preferably, the fourth Cas protein is fused to the fourth DNA polymerase through a peptide linker or not through a peptide linker to form a fourth fusion protein;
preferably, the fourth Cas protein is optionally linked or fused to the N-terminal of the fourth DNA polymerase through a linker; or, the fourth Cas protein is optionally linked or fused to the C-terminal of the fourth DNA polymerase through a linker;
preferably, the fourth fusion protein has the amino acid sequence set forth in SEQ ID NO: 52;
preferably, the nucleic acid molecule A4 and the nucleic acid molecule B4 are comprised in the same or different expression vectors (e.g., eukaryotic expression vectors); preferably, the nucleic acid molecule A4 and the nucleic acid molecule B4 are capable of expressing the fourth Cas protein and the fourth DNA polymerase in isolation state in a cell, or capable of expressing a fourth fusion protein comprising the fourth Cas protein and the fourth DNA polymerase in a cell;
preferably, the system or kit comprises a fourth fusion protein comprising the fourth Cas protein and the fourth DNA polymerase, or a nucleic acid molecule comprising a nucleotide sequence encoding the fourth fusion protein; or, the fourth Cas protein and the fourth DNA polymerase in isolation state, or, a nucleic acid molecule capable of expressing the fourth Cas protein and the fourth DNA polymerase in isolation state.

36. The system or kit according to claim 32 or 34, wherein the first, second, third and fourth Cas proteins are the same Cas protein, the first, second, third and fourth DNA polymerase are the same DNA polymerase; and, the system or kit comprises:
(M1-1) a first fusion protein comprising the first Cas protein and the first DNA polymerase, or, a nucleic acid molecule comprising a nucleotide sequence encoding the first fusion protein; or, (M1-2) the first Cas protein and the first DNA polymerase in isolation state, or a nucleic acid molecule capable of expressing the first Cas protein and the first DNA polymerase in isolation state;
(M2) a first PegRNA comprising the first gRNA and the first tag primer, or a nucleic acid molecule comprising a nucleotide sequence encoding the first PegRNA;
(M3) a second PegRNA comprising the second gRNA and the second tag primer, or a nucleic acid molecule comprising a nucleotide sequence encoding the second PegRNA;
(M4) a third PegRNA comprising the third gRNA and the third tag primer, or a nucleic acid molecule comprising a nucleotide sequence encoding the third PegRNA;
(M5) a fourth PegRNA comprising the fourth gRNA and the fourth tag primer, or a nucleic acid molecule comprising a nucleotide sequence encoding the fourth PegRNA;
preferably, the system or kit further comprises: a nucleic acid vector as defined in any one of claims 16-18.

37. The system or kit according to any one of claims 32-36, wherein the fourth double-stranded target nucleic acid is the same as the third double-stranded target nucleic acid, and the third and fourth functional complexes fragment the same double-stranded target nucleic acid at different positions to form fragmented nucleotide fragments a1, a2 and a3; wherein, in the same double-stranded target nucleic acid, the nucleotide fragment a1 is linked to the nucleotide fragment a3 through the nucleotide fragment a2;
preferably, the third and fourth functional complexes result in the isolation of the nucleotide fragments a1 and a2 and the isolation of the nucleotide fragments a2 and a3, respectively;
preferably, the nucleotide fragment a1 has a third overhang formed by extension using the third tag primer as a template; and the nucleotide fragment a3 has a fourth overhang formed by extension using the fourth tag primer as a template;
preferably, under conditions that allow hybridization or annealing of nucleic acids, the first tag sequence or complementary sequence thereof or the first overhang is capable of being hybridized or annealed to the fragmented nucleotide fragment a1; preferably, the first tag sequence or complementary sequence thereof or the first overhang is capable of being hybridized or annealed to the fragmented nucleotide fragment a1 at the end formed by fragmenting the third double-stranded target nucleic acid by the third functional complex; preferably, the complementary sequence of the first tag sequence or the first overhang is capable of being hybridized or annealed to the 3' end or 3' portion of one nucleic acid strand of the fragmented nucleotide fragment a1, and the 3' end or 3' portion is formed by fragmenting the third double-stranded target nucleic acid by the third functional complex; preferably, the first overhang is capable of being hybridized or annealed to the third overhang of the fragmented nucleotide fragment a1 or or an upstream nucleotide sequence thereof;
preferably, the complementary sequence of the third tag sequence or the third overhang is capable of being hybridized or annealed to the first overhang or its upstream nucleotide sequence under conditions that allow hybridization or annealing of nucleic acids;
preferably, under conditions that allow hybridization or annealing of nucleic acids, the second tag sequence or complementary sequence thereof or the second overhang is capable of being hybridized or annealed to the fragmented nucleotide fragment a3; preferably, the second tag sequence or complementary sequence thereof or the second overhang is capable of being hybridized or annealed to the fragmented nucleotide fragment a3 at the end formed by fragmenting the third double-stranded target nucleic acid by the fourth functional complex; preferably, the complementary sequence of the second tag sequence or the second overhang is capable of being hybridized or annealed to the 3' end or 3' portion of one nucleic acid strand of the fragmented nucleotide fragment a3, and the 3' end or 3' portion is formed by fragmenting the third double-stranded target nucleic acid by the fourth functional complex; preferably, the second overhang is capable of being hybridized or annealed to the fourth overhang of the fragmented nucleotide fragment a3 or an upstream nucleotide sequence thereof;
preferably, under conditions that allow hybridization or annealing of nucleic acids, the complementary sequence of the fourth tag sequence or the fourth overhang is capable of being hybridized or annealed to the second overhang or an upstream nucleotide sequence thereof.

38. The system or kit according to any one of claims 1-37, the kit further comprising an additional component;
preferably, the additional component comprises one or more selected from the group consisting of:
(1) one or more (e.g., 2, 3, 4, 5, 10, 15, 20, or more) additional gRNAs or a nucleic acid molecule comprising a nucleotide sequence encoding the additional gRNA, wherein the additional gRNA is capable of binding to a Cas protein and form a functional complex; preferably, the functional complex is capable of fragmenting two strands of a double-stranded target nucleic acid to form a target nucleic acid fragment;
(2) one or more (e.g., 2, 3, 4, 5, 10, 15, 20, or more) additional Cas proteins or a nucleic acid molecule comprising a nucleotide sequence encoding the additional Cas protein; preferably, the Cas protein is capable of cleaving or fragmenting a double-stranded target nucleic acid;
(3) one or more (e.g., 2, 3, 4, 5, 10, 15, 20, or more) additional tag primers or a nucleic acid molecule comprising a nucleotide sequence encoding the additional tag primer, wherein the additional tag primer comprises a tag sequence and a target-binding sequence, the tag sequence is located at the upstream or 5' end of the target-binding sequence; preferably, under conditions allowing hybridization or annealing of nucleic acids, the target-binding sequence is capable of being hybridized or annealed to the 3' end of one nucleic acid strand of the target nucleic acid fragment to form a double-stranded structure, and the tag sequence does not bind to the target nucleic acid fragment, and is in a free single-chain state;
(4) one or more (e.g., 2, 3, 4, 5, 10, 15, 20, or more) additional DNA polymerases or a nucleic acid molecule comprising a nucleotide sequence encoding the additional DNA polymerase; preferably, the additional DNA polymerase is selected from the group consisting of DNA-dependent DNA polymerase and RNA-dependent DNA polymerase; preferably, the additional DNA polymerase is an RNA-dependent DNA polymerase, such as a reverse transcriptase.

39. A fusion protein, which comprises a Cas protein and a template-dependent DNA polymerase, wherein the Cas protein is capable of fragmenting a double-stranded target nucleic acid;
preferably, the Cas protein is capable of fragmenting the double-stranded target nucleic acid and producing a sticky end or blunt end;
preferably, the Cas protein is selected from the group consisting of Cas9 protein, Cas12a protein, cas12b protein, cas12c protein, cas12d protein, cas12e protein, cas12f protein, cas12g protein, cas12h protein, cas12i protein, cas14 protein, Cas13a protein, Cas1 protein, Cas1B protein, Cas2 protein, Cas3 protein, Cas4 protein, Cas5 protein, Cas6 protein, Cas7 protein, Cas8 protein, Cas10 protein, Csy1 protein, Csy2 protein, Csy3 protein, Cse1 protein, Cse2 protein, Csc1 protein, Csc2 protein, Csa5 protein, Csn2 protein, Csm2 protein, Csm3 protein, Csm4 protein, Csm5 protein, Csm6 protein, Cmr1 protein, Cmr3 protein, Cmr4 protein, Cmr5 protein, Cmr6 protein, Csb1 protein, Csb2 protein, Csb3 protein, Csx17 protein, Csx14 protein, Csx10 protein, Csx16 protein, CsaX protein, Csx3 protein, Csx1 protein, Csx15 protein, Csf1 protein, Csf2 protein, Csf3 protein, Csf4 protein and homologue thereof or modified form thereof;
preferably, the Cas protein is a Cas9 protein, such as a Cas9 protein (spCas9) of *Streptococcus pyogenes (S. pyogenes);*
preferably, the Cas protein has the amino acid sequence set forth in SEQ ID NO: 1;
preferably, the DNA polymerase is selected from the group consisting of DNA-dependent DNA polymerase and RNA-dependent DNA polymerase;
preferably, the DNA polymerase is an RNA-dependent DNA polymerase;
preferably, the DNA polymerase is a reverse transcriptase, such as a reverse transcriptase derived from Moloney murine leukemia virus, human immunodeficiency virus (HIV), avian sarcoma-leukemia virus (ASLV), Rous sarcoma virus (RSV), avian myeloblastosis virus (AMV), avian erythroblastosis virus helper virus, avian granulocytosis virus MC29 helper virus, avian reticuloendotheliosis virus helper virus, avian sarcoma virus UR2 helper virus, avian sarcoma virus Y73 helper virus, Rous-associated virus and myeloblastosis-associated virus (MAV);
preferably, the DNA polymerase has the amino acid sequence set forth in SEQ ID NO: 4;
preferably, the Cas protein is covalently linked to the DNA polymerase with or without a linker;
preferably, the linker is a peptide linker, such as a flexible peptide linker; for example, the linker has the amino acid sequence set forth in SEQ ID NO: 51;
preferably, the Cas protein is optionally linked or fused to the N-terminal of the DNA polymerase through a linker; or, the Cas protein is optionally linked or fused to the C-terminal of the DNA polymerase through a linker;
preferably, the fusion protein has the amino acid sequence set forth in SEQ ID NO: 52.

40. A nucleic acid molecule, which comprises a polynucleotide encoding the fusion protein according to claim 39.

41. A vector, which comprises the nucleic acid molecule according to claim 40;
preferably, the vector is an expression vector;
preferably, the vector is an eukaryotic expression vector.

42. A host cell, which comprises the nucleic acid molecule according to claim 40 or the vector according to claim 41;
preferably, the host cell is an prokaryotic cell, such as E. coli cell; or the host cell is an eukaryotic cell, such as yeast cell, fungal cell, plant cell, animal cell;
preferably, the host cell is a mammalian cell, such as a human cell.

43. A method for preparing the fusion protein according to claim 39, comprising, (1) culturing the host cell according to claim 42 under conditions that allow protein expression; and (2) isolating the fusion protein expressed by the host cell.

44. A complex, which comprises a first Cas protein and a first DNA polymerase that is template-dependent, wherein the first Cas protein has the ability of fragmenting a double-stranded target nucleic acid, and the first Cas protein is complexed with the first DNA polymerase in a covalent or non-covalent manner;
preferably, the first Cas protein is capable of fragmenting the double-stranded target nucleic acid and producing a sticky end or blunt end;
preferably, the first Cas protein is selected from the group consisting of Cas9 protein, Cas 12a protein, cas12b protein, cas12c protein, cas12d protein, cas12e protein, cas12f protein, cas12g protein, cas12h protein, cas12i protein, cas14 protein, Cas13a protein, Cas1 protein, Cas1B protein, Cas2 protein, Cas3 protein, Cas4 protein, Cas5 protein, Cas6 protein, Cas7 protein, Cas8 protein, Cas10 protein, Csy1 protein, Csy2 protein, Csy3 protein, Cse1 protein, Cse2 protein, Csc1 protein, Csc2 protein, Csa5 protein, Csn2 protein, Csm2 protein, Csm3 protein, Csm4 protein, Csm5 protein, Csm6 protein, Cmr1 protein, Cmr3 protein, Cmr4 protein, Cmr5 protein, Cmr6 protein, Csb1 protein, Csb2 protein, Csb3 protein, Csx17 protein, Csx14 protein, Csx10 protein, Csx16 protein, CsaX protein, Csx3 protein, Csx1 protein, Csx15 protein, Csf1 protein, Csf2 protein, Csf3 protein, Csf4 protein and their homologue thereof or modified form thereof;
preferably, the first Cas protein is a Cas9 protein, such as a Cas9 protein (spCas9) of *Streptococcus pyogenes* (*S. pyogenes*);
preferably, the first Cas protein has the amino acid sequence set forth in SEQ ID NO: 1;
preferably, the first DNA polymerase is selected from the group consisting of DNA-dependent DNA polymerase and RNA-dependent DNA polymerase;
preferably, the first DNA polymerase is an RNA-dependent DNA polymerase;
preferably, the first DNA polymerase is a reverse transcriptase, such as a reverse transcriptase derived from Moloney murine leukemia virus, human immunodeficiency virus (HIV), avian sarcoma-leukemia virus (ASLV), Rous sarcoma virus (RSV), avian myeloblastosis virus (AMV), avian erythroblastosis virus helper virus, avian granulocytosis virus MC29 helper virus, avian reticuloendotheliosis virus helper virus, avian sarcoma virus UR2 helper virus, avian sarcoma virus Y73 helper virus, Rous-associated virus and myeloblastosis-associated virus (MAV);
preferably, the first DNA polymerase has the amino acid sequence set forth in SEQ ID NO: 4;
preferably, the first Cas protein is covalently linked to the first DNA polymerase with or without a linker;
preferably, the linker is a peptide linker, such as a flexible peptide linker; for example, the linker has the amino acid sequence set forth in SEQ ID NO: 51;
preferably, the first Cas protein is fused to the first DNA polymerase through a peptide linker or not through a peptide linker to form a first fusion protein;
preferably, the first Cas protein is optionally linked or fused to the N-terminal of the first DNA polymerase through a linker; or, the first Cas protein is optionally linked or fused to the C-terminal of the first DNA polymerase through a linker;
preferably, the first fusion protein has the amino acid sequence set forth in SEQ ID NO: 52.

45. The complex according to claim 44, wherein the complex further comprises a first gRNA;
preferably, the first gRNA is capable of binding to the first Cas protein to form a first functional unit; the first functional unit is capable of binding to a double-stranded target nucleic acid and fragmenting two strands thereof to form fragmented the target nucleic acid fragments;
preferably, the first gRNA comprises a first guide sequence, and, under conditions that allow hybridization or annealing of nucleic acids, the first guide sequence is capable of being hybridized or annealed to one nucleic acid strand of the double-stranded target nucleic acid;
preferably, the first guide sequence has a length of at least 5nt, such as 5-10nt, 10-15nt, 15-20nt, 20-25nt, 25-30nt, 30-40nt, 40-50nt, 50-100nt, 100-200nt, or longer;
preferably, the first gRNA further comprises a first scaffold sequence, which is capable of being recognized by and bound to the first Cas protein, thereby forming a first functional unit;
preferably, the first scaffold sequence has a length of at least 20nt, such as 20-30nt, 30-40nt, 40-50nt, 50-100nt, 100-200nt, or longer;
preferably, the first guide sequence is located at the upstream or 5' end of the first scaffold sequence;
preferably, after the first guide sequence binds to the double-stranded target nucleic acid, the complex or the first functional unit is capable of fragmenting two strands of the double-stranded target nucleic acid to form a target nucleic acid fragment.

46. The complex according to claim 45, wherein the complex further comprises a double-stranded target nucleic acid,
preferably, the double-stranded target nucleic acid comprises a first PAM sequence recognized by the first Cas protein and a first guide binding sequence capable of being hybridized or annealed to the first guide sequence, whereby the first functional unit binds to the double-stranded target nucleic acid through the first guide binding sequence and the first PAM sequence.

47. The complex according to claim 46, wherein the complex further comprises a first tag primer that is hybridized or annealed to the double-stranded target nucleic acid; wherein the first tag primer comprises a first target-binding sequence capable of being hybridized or annealed to the double-stranded target nucleic acid;
preferably, the tag primer comprises a first tag sequence and a first target-binding sequence, the first tag sequence is located at the upstream or 5' end of the first target-binding sequence; and, under conditions that allow hybridization or annealing of nucleic acids, the first target-binding sequence is capable of being hybridized or annealed to the double-stranded target nucleic acid; preferably, the first target-binding sequence is capable of being hybridized or annealed to the double-stranded target nucleic acid at the position fragmented by the first functional unit; preferably, the first target-binding sequence is capable of being hybridized or annealed to the 3' end of one nucleic acid strand of the target nucleic acid fragment to form a double-stranded structure; preferably, the 3' end is formed by fragmenting the double-stranded target nucleic acid by the first functional unit; preferably, the first tag sequence does not bind to the target nucleic acid fragment and is in a free single-stranded state;
preferably, the first target-binding sequence has a length of at least 5nt, such as 5-10nt, 10-15nt, 15-20nt, 20-25nt, 25-30nt, 30-40nt, 40-50nt, 50-100nt, 100-200nt, or longer;
preferably, the first tag sequence has a length of at least 4nt, such as 4-10nt, 10-15nt, 15-20nt, 20-25nt, 25-30nt, 30-40nt, 40-50nt, 50-100nt, 100-200nt, or longer;
preferably, the first tag primer binds to the target nucleic acid fragment through the first target-binding sequence; preferably, the first DNA polymerase binds to the target nucleic acid fragment and the first tag primer;
preferably, the first tag primer is a single-stranded deoxyribonucleic acid or a single-stranded ribonucleic acid;
preferably, the first tag primer is a single-stranded ribonucleic acid, and the first DNA polymerase is an RNA-dependent DNA polymerase; or, the first tag primer is a single-stranded deoxyribonucleic acid, and the first DNA polymerase is a DNA-dependent DNA polymerase;
preferably, the target nucleic acid fragment is extended by the first DNA polymerase using the first tag primer as a template to form a first overhang;
preferably, the nucleic acid strand bound to the first gRNA is different from the nucleic acid strand bound to the first tag primer; preferably, the nucleic acid strand to which the first gRNA binds is an opposite strand of the nucleic acid strand to which the first tag primer binds.

48. The complex according to claim 47, wherein the first tag primer is linked to the first gRNA; preferably, the first tag primer is covalently linked to the first gRNA through a linker or not through a linker;
preferably, the first tag primer is optionally linked to the 3' end of the first gRNA through a linker;
preferably, the linker is a nucleic acid linker (e.g., a ribonucleic acid linker or a deoxyribonucleic acid linker);
preferably, the first tag primer is a single-stranded ribonucleic acid, and is ligated to the 3' end of the first gRNA through a ribonucleic acid linker or not through a ribonucleic acid linker to form a first PegRNA.

49. The complex according to claim 47 or 48, wherein the complex further comprises a second Cas protein and a second gRNA, wherein the second Cas protein has the ability to fragment a double-stranded target nucleic acid, the second gRNA is capable of binding to the second Cas protein to form a second functional unit; the second functional unit is capable of binding to a double-stranded target nucleic acid, and fragmenting two strands thereof to form a target nucleic acid fragment;
preferably, the second Cas protein is the same as or different from the first Cas protein; preferably, the second Cas protein is the same as the first Cas protein;
preferably, the second Cas protein is capable of fragmenting the double-stranded target nucleic acid and producing a sticky end or blunt end;
preferably, the second Cas protein is selected from the group consisting of Cas9 protein, Cas12a protein, cas12b protein, cas12c protein, cas12d protein, cas12e protein, cas12f protein, cas12g protein, cas12h protein, cas12i protein, cas14 protein, Cas1 protein, Cas1B protein, Cas2 protein, Cas3 protein, Cas4 protein, Cas5 protein, Cas6 protein, Cas7 protein, Cas8 protein, Cas10 protein, Csy1 protein, Csy2 protein, Csy3 protein, Cse1 protein, Cse2 protein, Csc1 protein, Csc2 protein, Csa5 protein, Csn2 protein, Csm2 protein, Csm3 protein, Csm4 protein, Csm5 protein, Csm6 protein, Cmr1 protein, Cmr3 protein, Cmr4 protein, Cmr5 protein, Cmr6 protein, Csb1 protein, Csb2 protein, Csb3 protein, Csx17 protein, Csx14 protein, Csx10 protein, Csx16 protein, CsaX protein, Csx3 protein, Csx1 protein, Csx15 protein, Csf1 protein, Csf2 protein, Csf3 protein, Csf4 protein and homologue thereof or modified form thereof;
preferably, the second Cas protein is a Cas9 protein, such as a Cas9 protein (spCas9) of *Streptococcus pyogenes* (*S. pyogenes*);
preferably, the second Cas protein has the amino acid sequence set forth in SEQ ID NO: 1;
preferably, the second gRNA comprises a second guide sequence, and under conditions that allow hybridization or annealing of nucleic acids, the second guide sequence is capable of being hybridized or annealed to one nucleic acid strand of the double-stranded target nucleic acid;
preferably, the second guide sequence is different from the first guide sequence; preferably, the nucleic acid strand bound to the first guide sequence is different from the nucleic acid strand bound to the second guide sequence; preferably, the nucleic acid strand to which the first guide sequence binds is an opposite strand of the nucleic acid strand to which the second guide sequence binds;
preferably, the second guide sequence has a length of at least 5nt, such as 5-10nt, 10-15nt, 15-20nt, 20-25nt, 25-30nt, 30-40nt, 40-50nt, 50-100nt, 100-200nt, or longer;
preferably, the second gRNA further comprises a second scaffold sequence, which is capable of being recognized by and bound to the second Cas protein, thereby forming a second functional unit;
preferably, the second scaffold sequence is the same as or different from the first scaffold sequence; preferably, the second scaffold sequence is the same as the first scaffold sequence;
preferably, the second scaffold sequence has a length of at least 20nt, such as 20-30nt, 30-40nt, 40-50nt, 50-100nt, 100-200nt, or longer;
preferably, the second guide sequence is located at the upstream or 5' end of the second scaffold sequence;
preferably, the double-stranded target nucleic acid comprises a second PAM sequence recognized by the second Cas protein and a second guide binding sequence capable of being hybridized or annealed to the second guide sequence, whereby the second functional unit binds to the double-stranded target nucleic acid through the second guide binding sequence and the second PAM sequence.

50. The complex according to claim 49, wherein the complex further comprises a template-dependent second DNA polymerase, the second DNA polymerase is complexed with a second Cas protein in a covalent or non-covalent manner;
preferably, the second DNA polymerase is selected from the group consisting of DNA-dependent DNA polymerase and RNA-dependent DNA polymerase;
preferably, the second DNA polymerase is an RNA-dependent DNA polymerase;
preferably, the second DNA polymerase is a reverse transcriptase, such as a reverse transcriptase derived from Moloney murine leukemia virus, human immunodeficiency virus (HIV), avian sarcoma-leukemia virus (ASLV), Rous sarcoma virus (RSV), avian myeloblastosis virus (AMV), avian erythroblastosis virus helper virus, avian granulocytosis virus MC29 helper virus, avian reticuloendotheliosis virus helper virus, avian sarcoma virus UR2 helper virus, avian sarcoma virus Y73 helper virus, Rous-associated virus and myeloblastosis-associated virus (MAV);
preferably, the second DNA polymerase has the amino acid sequence set forth in SEQ ID NO: 4;
preferably, the second DNA polymerase is the same as or different from the first DNA polymerase; preferably, the second DNA polymerase is the same as the first DNA polymerase;
preferably, the second Cas protein is covalently linked to the second DNA polymerase with or without a linker;
preferably, the linker is a peptide linker, such as a flexible peptide linker; for example, the linker has the amino acid sequence set forth in SEQ ID NO: 51;
preferably, the second Cas protein is fused to the second DNA polymerase through a peptide linker or not through a peptide linker to form a fusion second protein;
Preferably, the second Cas protein is optionally linked or fused to the N-terminal of the second DNA polymerase through a linker; or, the second Cas protein is optionally linked or fused to the C-terminal of the second DNA polymerase through a linker;
preferably, the second fusion protein has the amino acid sequence set forth in SEQ ID NO: 52.

51. The complex according to claim 50, wherein the complex further comprises a second tag primer that is hybridized or annealed to the double-stranded target nucleic acid; wherein the second tag primer comprises a second target-binding sequence capable of being hybridized or annealed to the double-stranded target nucleic acid;
preferably, the tag primer comprises a second tag sequence and a second target-binding sequence, the second tag sequence is located at the upstream or 5' end of the second target-binding sequence; and, under conditions that allow hybridization or annealing of nucleic acids, the second target-binding sequence is capable of being hybridized or annealed to the double-stranded target nucleic acid; preferably, the second target-binding sequence is capable of being hybridized or annealed to the double-stranded target nucleic acid at the position fragmented by the second functional unit; preferably, the second target-binding sequence is capable of being hybridized or annealed to the 3' end of one nucleic acid strand of the target nucleic acid fragment to form a double-stranded structure; preferably, the 3' end is formed by fragmenting the double-stranded target nucleic acid by the second functional unit; preferably, the second tag sequence does not bind to the target nucleic acid fragment, and is in a free single-stranded state;
preferably, the second target-binding sequence has a length of at least 5nt, such as 5-10nt, 10-15nt, 15-20nt, 20-25nt, 25-30nt, 30-40nt, 40-50nt, 50-100nt, 100-200nt, or longer;
preferably, the second target-binding sequence is different from the first target-binding sequence; preferably, the nucleic acid strand bound to the second target-binding sequence is different from the nucleic acid strand bound to the first target-binding sequence; preferably, the nucleic acid strand bound to the second target-binding sequence is an opposite strand of the nucleic acid strand bound to the first target-binding sequence;
preferably, the second tag sequence has a length of at least 4nt, such as 4-10nt, 10-15nt, 15-20nt, 20-25nt, 25-30nt, 30-40nt, 40-50nt, 50-100nt, 100-200nt, or longer;
preferably, the second tag sequence is the same as or different from the first tag sequence; preferably, the second tag sequence is different from the first tag sequence;
preferably, the second tag primer binds to the target nucleic acid fragment through the second target-binding sequence; preferably, the second DNA polymerase binds to the target nucleic acid fragment and the second tag primer;
preferably, the second tag primer is a single-stranded deoxyribonucleic acid or a single-stranded ribonucleic acid;
preferably, the second tag primer is a single-stranded ribonucleic acid, and the second DNA polymerase is an RNA-dependent DNA polymerase; or, the second tag primer is a single-stranded deoxyribonucleic acid, and the second DNA polymerase is a DNA-dependent DNA polymerase;
preferably, the target nucleic acid fragment is extended by the second DNA polymerase using the second tag primer as a template to form a second overhang;
preferably, the nucleic acid strand to which the second gRNA binds is different from the nucleic acid strand to which the second tag primer binds; preferably, the nucleic acid strand to which the second gRNA binds is an opposite strand of the nucleic acid strand to which the second tag primer binds.

52. The complex according to claim 51, wherein the second tag primer is ligated to the second gRNA; preferably, the second tag primer is covalently linked to the second gRNA through a linker or not through a linker;
preferably, the second tag primer is optionally linked to the 3' end of the second gRNA through a linker;
preferably, the linker is a nucleic acid linker (e.g., a ribonucleic acid linker or a deoxyribonucleic acid linker);
preferably, the second tag primer is a single-stranded ribonucleic acid, and is ligated to the 3' end of the second gRNA through a ribonucleic acid linker or not through a ribonucleic acid linker to form a second PegRNA.

53. The complex according to claim 52, wherein the first and second functional units bind to the double-stranded target nucleic acid in a predetermined positional relationship;
preferably, the second guide sequence and the first target-binding sequence bind to the same nucleic acid strand; and/or, the first guide sequence and the second target-binding sequence bind to the same nucleic acid strand;
preferably, the binding position of the second guide sequence is located at the upstream or 5' end of the binding position of the first target-binding sequence; and/or, the binding position of the first guide sequence is located at the upstream or 5' end of the binding position of the second target-binding sequence;
preferably, the binding position of the second guide sequence is located at the downstream or 3' end of the binding position of the first target-binding sequence; and/or, the binding position of the first guide sequence is located at the downstream or 3' end of the binding position of the second target-binding sequence;
preferably, the double-stranded target nucleic acid is selected from the group consisting of genomic DNA and nucleic acid vector DNA.

54. A method for fragmenting a double-stranded target nucleic acid and adding an overhang at its 3' end, wherein the method comprises: using the system or kit according to any one of claims 1-9.

55. The method according to claim 54, wherein the method comprises the steps of:
i. providing a double-stranded target nucleic acid; and
providing the first Cas protein, the first gRNA, the first DNA polymerase and the first tag primer;
ii. contacting the double-stranded target nucleic acid with the first Cas protein, the first gRNA, the first DNA polymerase and the first tag primer;
preferably, in step ii:
the first Cas protein binds to the first gRNA to form a first functional complex, and the first functional complex binds to and fragments the double-stranded target nucleic acid to form a target nucleic acid fragment; and,
the first tag primer is hybridized or annealed to the 3' end of one nucleic acid strand of the target nucleic acid fragment through the first target-binding sequence; and,
the first DNA polymerase extends the target nucleic acid fragment to form a first overhang by using the first tag primer annealed to the target nucleic acid fragment as a template;
preferably, the method is performed in a cell;
preferably, in step i, the first Cas protein or nucleic acid molecule A1, the first DNA polymerase or nucleic acid molecule B1, the first gRNA or nucleic acid molecule C1 and the first tag primer or nucleic acid molecule D1 are delivered into a cell to provide the first Cas protein, the first gRNA, the first DNA polymerase and the first tag primer in the cell;
preferably, in step i, the nucleic acid molecule A1, the nucleic acid molecule B1, the first gRNA or nucleic acid molecule C1 and the first tag primer or nucleic acid molecule D1 are delivered into a cell to provide the first Cas protein, the first gRNA, the first DNA polymerase and the first tag primer in the cell;
preferably, in step i, the nucleic acid molecules A1, B1, C1 and D1 are delivered into a cell to provide the first Cas protein, the first gRNA, the first DNA polymerase and the first tag primer in the cell;
preferably, the nucleic acid molecule A1 and the nucleic acid molecule B1 are comprised in the same or different expression vectors (e.g., eukaryotic expression vectors); preferably, the nucleic acid molecule A1 and the nucleic acid molecule B1 are capable of expressing the first Cas protein and the first DNA polymerase in isolation state in a cell, or are capable of expressing a first fusion protein comprising the first Cas protein and the first DNA polymerase in a cell; preferably, in step i, a nucleic acid molecule comprising a nucleotide sequence encoding the first Cas protein and the first DNA polymerase in isolation state or a nucleic acid molecule comprising a nucleotide sequence encoding the first fusion protein is delivered into a cell, and expressed in the cell, to provide the first Cas protein and the first DNA polymerase in the cell;
preferably, the nucleic acid molecule C1 and the nucleic acid molecule D1 are comprised in the same expression vector (e.g., an eukaryotic expression vector); preferably, the nucleic acid molecule C1 and the nucleic acid molecule D1 are capable of transcribing a first PegRNA comprising the first gRNA and the first tag primer in a cell; preferably, in step i, the first PegRNA is delivered into a cell to provide the first gRNA and the first tag primer in the cell; or, a nucleic acid molecule comprising a nucleotide sequence encoding the first PegRNA is delivered into a cell, and the first PegRNA is transcribed in the cell, to provide the first gRNA and the first tag primer in the cell;
preferably, in step i, a nucleic acid molecule capable of expressing the first Cas protein and the first DNA polymerase in isolation state or a nucleic acid molecule comprising a nucleotide sequence encoding the first fusion protein, and a nucleic acid molecule comprising a nucleotide sequence encoding the first PegRNA is delivered into a cell, and transcribed and expressed in the cell, thereby providing the first Cas protein, the first gRNA, the first DNA polymerase and the first tag primer in the cell;
preferably, in step i, the double-stranded target nucleic acid or a nucleic acid molecule T comprising the double-stranded target nucleic acid is delivered into a cell, to provide the double-stranded target nucleic acid in the cell;
preferably, the first Cas protein, the first gRNA, the first DNA polymerase and the first tag primer are as defined in any one of claims 1-9;
preferably, the double-stranded target nucleic acid or nucleic acid molecule T comprises a first PAM sequence recognized by the first Cas protein; preferably, in step ii, the first functional complex binds to the double-stranded target nucleic acid or nucleic acid molecule T through the first PAM sequence and the first gRNA, and fragments double-stranded target nucleic acid or nucleic acid molecule T.

56. A method for fragmenting a double-stranded target nucleic acid into a target nucleic acid fragment, and adding an overhang respectively at two 3' ends of the target nucleic acid fragment, wherein the method comprises, using the system or kit according to any one of claims 10-18; wherein the first double-stranded target nucleic acid and the second double-stranded target nucleic acid are the same.

57. The method according to claim 56, wherein the method comprises the steps of:
i. providing a double-stranded target nucleic acid; and
providing the first Cas protein, the first gRNA, the first DNA polymerase, the first tag primer, the second Cas protein, the second gRNA, the second DNA polymerase and the second tag primer;
ii. contacting the double-stranded target nucleic acid with the first Cas protein, the first gRNA, the first DNA polymerase, the first tag primer, the second Cas protein, the second gRNA, the second DNA polymerase, and the second tag primer;
preferably, in step ii:
the first Cas protein binds to the first gRNA to form a first functional complex, and the second Cas protein binds to the second gRNA to form a second functional complex; and, the first and second functional complexes bind and fragment the double-stranded target nucleic acid to form a target nucleic acid fragment F1; and,
the first tag primer is hybridized or annealed to the 3' end of one nucleic acid strand of the target nucleic acid fragment F1 through the first target-binding sequence; and the second tag primer hybridizes or annealed to the 3' end of the other nucleic acid strand of the target nucleic acid fragment F1 through the second target-binding sequence; and,
the first DNA polymerase and the second DNA polymerase extend the target nucleic acid fragment F1 by respectively using the first tag primer and the second tag primer annealed to the target nucleic acid fragment F1 as templates, to form a target nucleic acid fragment F2 having a first overhang and a second overhang;
preferably, the method is performed in a cell;
preferably, in step i, the first Cas protein or nucleic acid molecule A1, the first DNA polymerase or nucleic acid molecule B 1, the first gRNA or nucleic acid molecule C1, the first tag primer or nucleic acid molecule D1, the second Cas protein or nucleic acid molecule A2, the second DNA polymerase or nucleic acid molecule B2, the second gRNA or nucleic acid molecule C2, and the second tag primer or nucleic acid molecule D2 are delivered into the cell to provide the first Cas protein, the first gRNA, the first DNA polymerase, the first tag primer, the second Cas protein, the second gRNA, the second DNA polymerase and the second tag primer in the cell;
preferably, in step i, the nucleic acid molecule A1, the nucleic acid molecule B1, the first gRNA or nucleic acid molecule C1, the first tag primer or nucleic acid molecule D1, the nucleic acid molecule A2, the nucleic acid molecule B2, the second gRNA or nucleic acid molecule C2 and the second tag primer or nucleic acid molecule D2 are delivered into the cell to provide the first Cas protein, the first gRNA, the first DNA polymerase, the first tag primer, the second Cas protein, the second gRNA, the second DNA polymerase, and the second tag primer in the cell;
preferably, in step i, the nucleic acid molecules A1, B1, C1, D1, A2, B2, C2 and D2 are delivered into the cell to provide the first Cas protein, the first gRNA, the first DNA polymerase, the first tag primer, the second Cas protein, the second gRNA, the second DNA polymerase, and the second tag primer in the cell;
preferably, the nucleic acid molecule A1 and the nucleic acid molecule B1 are comprised in the same or different expression vectors (e.g., eukaryotic expression vectors); preferably, the nucleic acid molecule A1 and the nucleic acid molecule B1 are capable of expressing the first Cas protein and the first DNA polymerase in isolation state, or capable of expressing a first fusion protein comprising the first Cas protein and the first DNA polymerase; preferably, in step i, a nucleic acid molecule comprising a nucleotide sequence encoding the first Cas protein and the first DNA polymerase in isolation state or a nucleic acid molecule comprising a nucleotide sequence encoding the first fusion protein is delivered into the cell, and expressed in the cell to provide the first Cas protein and the first DNA polymerase in the cell;
preferably, the nucleic acid molecule A2 and the nucleic acid molecule B2 are comprised in the same or different expression vectors (e.g., eukaryotic expression vectors); preferably, the nucleic acid molecule A2 and the nucleic acid molecule B2 are capable of expressing the second Cas protein and the second DNA polymerase in isolation state, or capable of expressing a second fusion protein comprising the second Cas protein and the second DNA polymerase; preferably, in step i, a nucleic acid molecule comprising a nucleotide sequence encoding the second Cas protein and the second DNA polymerase in isolation state or a nucleic acid molecule comprising a nucleotide sequence encoding the second fusion protein is delivered into the cell, and expressed in the cell to provide the second Cas protein and the second DNA polymerase in the cell;
preferably, the nucleic acid molecule C1 and the nucleic acid molecule D1 are comprised in the same expression vector (e.g., an eukaryotic expression vector); preferably, the nucleic acid molecule C1 and the nucleic acid molecule D1 are capable of transcribing in a cell a first PegRNA comprising the first gRNA and the first tag primer; preferably, in step i, the first PegRNA is delivered into the cell to provide the first gRNA and the first tag primer in the cell, or, a nucleic acid molecule comprising a nucleotide sequence encoding the first PegRNA is delivered into the cell, and the first PegRNA is transcribed in the cell to provide the first gRNA and the first tag primer in the cell;
preferably, the nucleic acid molecule C2 and the nucleic acid molecule D2 are comprised in the same expression vector (e.g., an eukaryotic expression vector); preferably, the nucleic acid molecule C2 and the nucleic acid molecule D2 are capable of transcribing in a cell a second PegRNA comprising the second gRNA and the second tag primer; preferably, in step i, the second PegRNA is delivered into the cell to provide the second gRNA and the second tag primer in the cell, or, a nucleic acid molecule comprising a nucleotide sequence encoding the second PegRNA is delivered into the cell, and the second PegRNA is transcribed in the cell to provide the second gRNA and the second tag primer in the cell;
preferably, in step i, the double-stranded target nucleic acid or a nucleic acid molecule T comprising the double-stranded target nucleic acid is delivered into a cell to provide the double-stranded target nucleic acid in the cell;
preferably, the first Cas protein, the first gRNA, the first DNA polymerase or the first tag primer is as defined in any one of claims 1-9;
preferably, the second Cas protein, the second gRNA, the second DNA polymerase or the second tag primer is as defined in any one of claims 10-18;
preferably, the double-stranded target nucleic acid or nucleic acid molecule T comprises a first PAM sequence recognized by the first Cas protein and a second PAM sequence recognized by the second Cas protein; preferably, in step ii, the first functional complex binds to the double-stranded target nucleic acid or nucleic acid molecule T through the first PAM sequence and the first gRNA, and fragments it; and the second functional complex binds to the double-stranded target nucleic acid or nucleic acid molecule T through the second PAM sequence and the second gRNA, and fragments it.

58. The method according to claim 57, wherein the second Cas protein is the same as the first Cas protein, and the second DNA polymerase is the same as the first DNA polymerase; wherein the first Cas protein forms first and second functional complexes with the first and second gRNAs, respectively, and the first DNA polymerase extends the target nucleic acid fragment F1 by respectively using the first tag primer and the second tag primer annealed to the target nucleic acid fragment F1 as templates, to form a target nucleic acid fragment F2 having a first overhang and a second overhang;
preferably, in step i, the first Cas protein or nucleic acid molecule A1, the first DNA polymerase or nucleic acid molecule B 1, the first gRNA or nucleic acid molecule C1, the first tag primer or nucleic acid molecule D1, the second gRNA or nucleic acid molecule C2 and the second tag primer or nucleic acid molecule D2 are delivered into the cell to provide the first Cas protein, the first gRNA, the first DNA polymerase, the first tag primer, the second gRNA, and the second tag primer in the cell;
preferably, in step i, the nucleic acid molecule A1, the nucleic acid molecule B1, the first gRNA or nucleic acid molecule C1, the first tag primer or nucleic acid molecule D1, the second gRNA or nucleic acid molecule C2 and the second tag primer or nucleic acid molecule D2 are delivered into the cell to provide the first Cas protein, the first gRNA, the first DNA polymerase, the first tag primer, the second gRNA and the second tag primer in the cell;
preferably, in step i, the nucleic acid molecules A1, B1, C1, D1, C2 and D2 are delivered into the cell to provide the first Cas protein, the first gRNA, the first DNA polymerase, the first tag primer, the second gRNA, and the second tag primer in the cell;
preferably, the nucleic acid molecule A1 and the nucleic acid molecule B1 are comprised in the same or different expression vectors (e.g., eukaryotic expression vectors); preferably, the nucleic acid molecule A1 and the nucleic acid molecule B1 are capable of expressing the first Cas protein and the first DNA polymerase in isolation state in the cell, or capable of expressing a first fusion protein comprising the first Cas protein and the first DNA polymerase in the cell; preferably, in step i, a nucleic acid molecule capable of expressing the first Cas protein and the first DNA polymerase in isolation state or a nucleic acid molecule comprising a nucleotide sequence encoding the first fusion protein is delivered into the cell, and expressed in the cell to provide the first Cas protein and the first DNA polymerase in the cell;
preferably, the nucleic acid molecule C1 and the nucleic acid molecule D1 are comprised in the same expression vector (e.g., an eukaryotic expression vector); preferably, the nucleic acid molecule C1 and the nucleic acid molecule D1 are capable of transcribing a first PegRNA comprising the first gRNA and the first tag primer in a cell; preferably, in step i, the first PegRNA is delivered into the cell to provide the first gRNA and the first tag primer in the cell, or, a nucleic acid molecule comprising a nucleotide sequence encoding the first PegRNA is delivered into the cell, and the first PegRNA is transcribed in the cell to provide the first gRNA and the first tag primer in the cell;
preferably, the nucleic acid molecule C2 and the nucleic acid molecule D2 are comprised in the same expression vector (e.g., an eukaryotic expression vector); preferably, the nucleic acid molecule C2 and the nucleic acid molecule D2 are capable of transcribing a second PegRNA comprising the second gRNA and the second tag primer in a cell; preferably, in step i, the second PegRNA is delivered into the cell to provide the second gRNA and the second tag primer in the cell, or, a nucleic acid molecule comprising a nucleotide sequence encoding the second PegRNA is delivered into the cell, and the second PegRNA is transcribed in the cell to provide the second gRNA and the second tag primer in the cell;
preferably, in step i, a nucleic acid molecule capable of expressing the first Cas protein and the first DNA polymerase in isolation state or a nucleic acid molecule comprising a nucleotide sequence encoding the first fusion protein, a nucleic acid molecule comprising a nucleotide sequence encoding the first PegRNA and a nucleic acid molecule comprising a nucleotide sequence encoding the second PegRNA are delivered into the cell, and transcribed and expressed in the cell, thereby providing the first Cas protein, the first gRNA, the first DNA polymerase, the first tag primer, the second gRNA, and the second tag primer in the cell.

59. A method for inserting a target nucleic acid fragment into a nucleic acid molecule of interest; wherein the method comprises, using the system or kit according to any one of claims 19-27; wherein the first double-stranded target nucleic acid is the same as the second double-stranded target nucleic acid for providing the target nucleic acid fragment; and the third double-stranded target nucleic acid is the nucleic acid molecule of interest.

60. The method according to claim 59, wherein the method comprises:
a. by the method according to any one of claims 56-58, the first double-stranded target nucleic acid is fragmented into a target nucleic acid fragment F1, and overhangs are added to the two 3' ends of the target nucleic acid fragment F1, respectively, thereby forming a target nucleic acid fragment F2 having a first overhang and a second overhang;
b. the nucleic acid molecule of interest is fragmented with the third functional complex to form fragmented nucleotide fragments a1 and a2; and,
c. the nucleotide fragments a1 and a2 are ligated with the target nucleic acid fragment F2, thereby inserting the target nucleic acid fragment into the nucleic acid molecule of interest;
preferably, the method comprises the following steps:
i. providing the double-stranded target nucleic acid and the nucleic acid molecule of interest; and providing the first Cas protein, the first gRNA, the first DNA polymerase, the first tag primer, the second Cas protein, the second gRNA, the second DNA polymerase, the second tag primer, the third Cas protein, and the third gRNA;
ii. contacting the double-stranded target nucleic acid with the first Cas protein, the first gRNA, the first DNA polymerase, the first tag primer, the second Cas protein, the second gRNA, the second DNA polymerase, and the second tag primer, and contacting the nucleic acid molecule of interest with the third Cas protein and the third gRNA;
preferably, in step ii:
the first Cas protein binds to the first gRNA to form a first functional complex, the second Cas protein binds to the second gRNA to form a second functional complex, and the third Cas protein binds to the third gRNAs to form a third functional complex; and,
the first and second functional complexes bind and fragment the double-stranded target nucleic acid to form a target nucleic acid fragment F1, and the third functional complex binds and fragments the nucleic acid molecule of interest to form fragmented nucleotide fragments a1 and a2 of; and,
the first tag primer is hybridized or annealed to the 3' end of one nucleic acid strand of the target nucleic acid fragment F1 through the first target-binding sequence; and the second tag primer is hybridized or annealed to the 3' end of the other nucleic acid strand of the target nucleic acid fragment F1 through the second target-binding sequence; and,
the first DNA polymerase and the second DNA polymerase extend the target nucleic acid fragment F1 by respectively using the first tag primer and the second tag primer annealed to the target nucleic acid fragment F1 as templates to form a target nucleic acid fragment F2 having a first overhang and a second overhang; wherein the first overhang and the second overhang are capable of being hybridized or annealed to the fragmented nucleotide fragments a1 and a2, respectively; and,
the target nucleic acid fragment F2 is hybridized or annealed to the nucleotide fragments a1 and a2 through the first overhang and the second overhang, respectively, and then inserted or ligated between the nucleotide fragments a1 and a2, thereby inserting the target nucleic acid fragment into the nucleic acid molecule of interest;
preferably, the first overhang is capable of being hybridized or annealed to the 3' end or 3' portion of one nucleic acid strand of the nucleotide fragment a1, and the 3' end or 3' portion is formed by fragmenting the nucleic acid molecule of interest by the third functional complex;
preferably, the complementary sequence of the first tag sequence or the first overhang is capable of being hybridized or annealed to the 3' portion of one nucleic acid strand of the fragmented nucleotide fragment a1, and there is a first spacer region between the 3' portion of the nucleotide fragment a1 and the cleaved end formed by the third double-stranded target nucleic acid;
preferably, the first spacer region has a length of 1nt to 200nt, such as 1-10nt, 10-20nt, 20-30nt, 30-40nt, 40-50nt, 50-100nt or 100-200nt;
preferably, the second overhang is capable of being hybridized or annealed to the 3' end or 3' portion of one nucleic acid strand of the nucleotide fragment a2, and the 3' end or 3' portion is formed by fragmenting the nucleic acid molecule of interest by the third functional complex;
preferably, the complementary sequence of the second tag sequence or the second overhang is capable of being hybridized or annealed to the 3' portion of one nucleic acid strand of the fragmented nucleotide fragment a2, and there is a second spacer region between the 3' portion of the nucleotide fragment a2 and the cleaved end formed by the third double-stranded target nucleic acid;
preferably, the second spacer region has a length of 1nt to 200nt, such as 1-10nt, 10-20nt, 20-30nt, 30-40nt, 40-50nt, 50-100nt or 100-200nt;
preferably, the method is performed in a cell;
preferably, in step i, the first Cas protein or nucleic acid molecule A1, the first DNA polymerase or nucleic acid molecule B1, the first gRNA or nucleic acid molecule C1, the first tag primer or nucleic acid molecule D1, the second Cas protein or nucleic acid molecule A2, the second DNA polymerase or nucleic acid molecule B2, the second gRNA or nucleic acid molecule C2, the second tag primer or nucleic acid molecule D2, the third Cas protein or nucleic acid molecule A3 and the third gRNA or nucleic acid molecule C3 are delivered into the cell to provide the first Cas protein, the first gRNA, the first DNA polymerase, the first tag primer, the second Cas proteins, the second gRNA, the second DNA polymerase, the second tag primer, the third Cas protein, and the third gRNA;
preferably, in step i, the nucleic acid molecule A1, the nucleic acid molecule B1, the first gRNA or nucleic acid molecule C1, the first tag primer or nucleic acid molecule D1, the nucleic acid molecule A2, the nucleic acid molecule B2, the second gRNA or nucleic acid molecule C2, the second tag primer or nucleic acid molecule D2, the nucleic acid molecule A3 and the third gRNA or nucleic acid molecule C3 are delivered into the cell, to provide the first Cas protein, the first gRNA, the first DNA polymerase, the first tag primer, the second Cas protein, the second gRNA, the second DNA polymerase, the second tag primer, the third Cas protein, and the third gRNA in the cell;
preferably, in step i, the nucleic acid molecules A1, B1, C1, D1, A2, B2, C2, D2, A3 and C3 are delivered into the cell to provide the first Cas protein, the first gRNA, the first DNA polymerase, the first tag primer, the second Cas protein, the second gRNA, the second DNA polymerase, the second tag primer, the third Cas protein, and the third gRNA in the cell;
preferably, in step i, the double-stranded target nucleic acid or a nucleic acid molecule T comprising the double-stranded target nucleic acid is delivered into the cell to provide the double-stranded target nucleic acid in the cell;
preferably, the double-stranded target nucleic acid or nucleic acid molecule T comprises a first PAM sequence recognized by the first Cas protein and a second PAM sequence recognized by the second Cas protein; preferably, in step ii, the first functional complex binds to the double-stranded target nucleic acid or nucleic acid molecule T through the first PAM sequence and the first gRNA, and fragments it; and the second functional complex binds to the double-stranded target nucleic acid or nucleic acid molecule T through the second PAM sequence and the second gRNA, and fragments it;
preferably, the nucleic acid molecule of interest comprises a third PAM sequence recognized by the third Cas protein; preferably, in step ii, the third functional complex binds to the nucleic acid molecule of interest through the third PAM sequence and the third gRNA, and fragments it;
preferably, the nucleic acid molecule of interest is a genomic DNA of the cell;
preferably, the first Cas protein, the first gRNA, the first DNA polymerase or the first tag primer is as defined in any one of claims 1-9;
preferably, the second Cas protein, the second gRNA, the second DNA polymerase or the second tag primer is as defined in any one of claims 10-18;
preferably, the third Cas protein and the third gRNA are as defined in any one of claims 19-27.

61. The method according to claim 60, wherein the first, second and third Cas proteins are the same Cas protein, and the second DNA polymerase is the same as the first DNA polymerase; wherein the first Cas protein forms first, second and third functional complexes with the first, second and third gRNAs, respectively, and the first DNA polymerase extends the target nucleic acid fragment F1 by respectively using the first tag primer and the second tag primer annealed to the target nucleic acid fragment F1 as templates, to form a target nucleic acid fragment F2 having a first overhang and a second overhang;
preferably, in step i, the first Cas protein or nucleic acid molecule A1, the first DNA polymerase or nucleic acid molecule B1, the first gRNA or nucleic acid molecule C1, the first tag primer or nucleic acid molecule D1, the second gRNA or nucleic acid molecule C2, the second tag primer or nucleic acid molecule D2, and the third gRNA or nucleic acid molecule C3 are delivered into a cell to provide the first Cas protein, the first gRNA, the first DNA polymerase, the first tag primer, the second gRNA, the second tag primer and the third gRNA in the cell;
preferably, in step i, the nucleic acid molecule A1, the nucleic acid molecule B1, the first gRNA or nucleic acid molecule C1, the first tag primer or nucleic acid molecule D1, the second gRNA or nucleic acid molecule C2, the second tag primer or nucleic acid molecule D2 and the third gRNA or nucleic acid molecule C3 are delivered into a cell to provide the first Cas protein, the first gRNA, the first DNA polymerase, the first tag primer, the second gRNA, the second tag primer and the third gRNA in the cell;
preferably, in step i, the nucleic acid molecules A1, B1, C1, D1, C2, D2 and C3 are delivered into a cell to provide the first Cas protein, the first gRNA, the first DNA polymerase, the first tag primer, the second gRNA, the second tag primer, and the third gRNA in the cell;
preferably, the nucleic acid molecule A1 and the nucleic acid molecule B1 are comprised in the same or different expression vectors (e.g., eukaryotic expression vectors); preferably, the nucleic acid molecule A1 and the nucleic acid molecule B1 are capable of expressing the first Cas protein and the first DNA polymerase in isolation state in a cell, or capable of expressing a first fusion protein comprising the first Cas protein and the first DNA polymerase in a cell; preferably, in step i, a nucleic acid molecule capable of expressing the first Cas protein and the first DNA polymerase in isolation state or a nucleic acid molecule comprising a nucleotide sequence encoding the first fusion protein is delivered into a cell, and expressed in the cell to provide the first Cas protein and the first DNA polymerase in the cell;
preferably, the nucleic acid molecule C1 and the nucleic acid molecule D1 are comprised in the same expression vector (e.g., an eukaryotic expression vector); preferably, the nucleic acid molecule C1 and the nucleic acid molecule D1 are capable of transcribing a first PegRNA comprising the first gRNA and the first tag primer in a cell; preferably, in step i, the first PegRNA is delivered into a cell to provide the first gRNA and the first tag primer in the cell, or, a nucleic acid molecule comprising a nucleotide sequence encoding the first PegRNA is delivered into a cell, and the first PegRNA is transcribed in the cell to provide the first gRNA and the first tag primer in the cell;
preferably, the nucleic acid molecule C2 and the nucleic acid molecule D2 are comprised in the same expression vector (e.g., an eukaryotic expression vector); preferably, the nucleic acid molecule C2 and the nucleic acid molecule D2 are capable of transcribing a second PegRNA comprising the second gRNA and the second tag primer in a cell; preferably, in step i, the second PegRNA is delivered into a cell to provide the second gRNA and the second tag primer in the cell, or, a nucleic acid molecule comprising a nucleotide sequence encoding the second PegRNA is delivered into a cell, and the second PegRNA is transcribed in the cell to provide the second gRNA and the second tag primer in the cell;
preferably, in step i, a nucleic acid molecule capable of expressing the first Cas protein and the first DNA polymerase in isolation state or a nucleic acid molecule comprising a nucleotide sequence encoding the first fusion protein, a nucleic acid molecule comprising a nucleotide sequence encoding the first PegRNA, a nucleic acid molecule comprising a nucleotide sequence encoding the second PegRNA, and a nucleic acid molecule comprising a nucleotide sequence encoding the third gRNA are delivered into a cell, and transcribed and expressed in the cell, thereby providing the first Cas protein, the first gRNA, the first DNA polymerase, the first tag primer, the second gRNA, the second tag primer, and the third gRNA in the cell.

62. A method for substituting a nucleic acid fragment in a nucleic acid molecule of interest with a target nucleotide fragment; wherein the method comprises, using the system or kit according to any one of claims 28-37; wherein, the first double-stranded target nucleic acid is the same as the second double-stranded target nucleic acid for providing the target nucleic acid fragment; and the third double-stranded target nucleic acid is the same as the fourth double-stranded target nucleic acid, used as the nucleic acid molecule of interest;
preferably, the method comprises:
a. by the method according to any one of claims 56-58, the first double-stranded target nucleic acid is fragmented to form a target nucleic acid fragment F1, and overhangs are added to the two 3' ends of the target nucleic acid fragment F1, respectively, to form a target nucleic acid fragment F2 having a first overhang and a second overhang;
b. the nucleic acid molecule of interest is fragmented with the third and fourth functional complexes to form fragmented nucleotide fragments a1, a2 and a3; wherein, prior to the fragmentation, in the nucleic acid molecule of interest, the nucleotide fragments a1, a2 and a3 are arranged in sequence (i.e., the nucleotide fragment a1 is ligated to the nucleotide fragment a3 through the nucleotide fragment a2); and,
c. the nucleotide fragments a1 and a3 are ligated to the target nucleic acid fragment F2, thereby substituting the nucleotide fragment a2 in the nucleic acid molecule of interest with the target nucleic acid fragment.

63. The method according to claim 62, wherein the method comprises the steps of:
i. providing the double-stranded target nucleic acid and the nucleic acid molecule of interest; and providing the first Cas protein, the first gRNA, the first DNA polymerase, the first tag primer, the second Cas protein, the second gRNA, the second DNA polymerase, the second tag primer, the third Cas protein, the third gRNA, the fourth Cas protein and the fourth gRNA;
ii. contacting the double-stranded target nucleic acid with the first Cas protein, the first gRNA, the first DNA polymerase, the first tag primer, the second Cas protein, the second gRNA, the second DNA polymerase, and the second tag primer, and contacting the nucleic acid molecule of interest with the third Cas protein, the third gRNA, the fourth Cas protein, and the fourth gRNA;
preferably, in step ii:
the first Cas protein binds to the first gRNA to form a first functional complex, the second Cas protein binds to the second gRNA to form a second functional complex, the third Cas protein binds to the third gRNA to form a third functional complex, and the fourth Cas protein binds to the fourth gRNA to form a fourth functional complex; and,
the first and second functional complexes bind and fragment the double-stranded target nucleic acid to form a target nucleic acid fragment F1, and the third and fourth functional complexes bind and fragment the nucleic acid molecule of interest to form fragmented nucleotide fragments a1, a2, and a3; and,
the first tag primer is hybridized or annealed to the 3' end of one nucleic acid strand of the target nucleic acid fragment F1 through the first target-binding sequence; and the second tag primer is hybridized or annealed to the 3' end of the other nucleic acid strand of the target nucleic acid fragment F1 through the second target-binding sequence; and,
the first DNA polymerase and the second DNA polymerase extend the target nucleic acid fragment F1 by respectively using the first tag primer and the second tag primer annealed to the target nucleic acid fragment F1 as templates to form a target nucleic acid fragment F2 having a first overhang and a second overhang; wherein the first overhang and the second overhang are capable of being hybridized or annealed to the fragmented nucleotide fragments a1 and a3, respectively; and,
the target nucleic acid fragment F2 is hybridized or annealed to the nucleotide fragments a1 and a3 through the first overhang and the second overhang, respectively, and is then ligated between the nucleotide fragments a1 and a3, thereby the nucleotide fragment a2 in the nucleic acid molecule of interest is substituted with the target nucleic acid fragment;
preferably, the first overhang is capable of being hybridized or annealed to the 3' end or 3' portion of one nucleic acid strand of the nucleotide fragment a1, and the 3' end or 3' portion is formed by fragmenting the nucleic acid molecule of interest by the third functional complex;
preferably, the second overhang is capable of being hybridized or annealed to the 3' end or 3' portion of one nucleic acid strand of the nucleotide fragment a3, and the 3' end or 3' portion is formed by fragmenting the nucleic acid molecule of interest by the fourth functional complex;
preferably, the method is performed in a cell;
preferably, in step i, the first Cas protein or nucleic acid molecule A1, the first DNA polymerase or nucleic acid molecule B 1, the first gRNA or nucleic acid molecule C1, the first tag primer or nucleic acid molecule D1, the second Cas protein or nucleic acid molecule A2, the second DNA polymerase or nucleic acid molecule B2, the second gRNA or nucleic acid molecule C2, the second tag primer or nucleic acid molecule D2, the third Cas protein or nucleic acid molecule A3, the third gRNA or nucleic acid molecule C3, the fourth Cas protein or nucleic acid molecule A4, the fourth gRNA or nucleic acid molecule C4 are delivered into a cell, to provide the first Cas protein, the first gRNA, the first DNA polymerase, the first tag primer, the second Cas protein, the second gRNA, the second DNA polymerase, the second tag primer, the third Cas protein, the third gRNA, the fourth Cas proteins and the fourth gRNA in the cell;
preferably, in step i, the nucleic acid molecule A1, the nucleic acid molecule B1, the first gRNA or nucleic acid molecule C1, the first tag primer or nucleic acid molecule D1, the nucleic acid molecule A2, the nucleic acid molecule B2, the second gRNA or nucleic acid molecule C2, the second tag primer or nucleic acid molecule D2, the nucleic acid molecule A3, the third gRNA or nucleic acid molecule C3, the nucleic acid molecule A4 and the fourth gRNA or nucleic acid molecule C4 are delivered into a cell, to provide the first Cas protein, the first gRNA, the first DNA polymerase, the first tag primer, the second Cas protein, the second gRNA, the second DNA polymerase, the second tag primer, the third Cas protein, the third gRNA, the fourth Cas protein, and the fourth gRNA in the cell;
preferably, in step i, the nucleic acid molecules A1, B1, C1, D1, A2, B2, C2, D2, A3, C3, A4 and C4 are delivered into a cell to provide the first Cas protein, the first gRNA, the first DNA polymerase, the first tag primer, the second Cas protein, the second gRNA, the second DNA polymerase, the second tag primer, the third Cas protein, the third gRNA, the fourth Cas protein and the fourth gRNA in the cell;
preferably, in step i, the double-stranded target nucleic acid or a nucleic acid molecule T comprising the double-stranded target nucleic acid is delivered into a cell to provide the double-stranded target nucleic acid in the cell;
preferably, the double-stranded target nucleic acid or nucleic acid molecule T comprises a first PAM sequence recognized by the first Cas protein and a second PAM sequence recognized by the second Cas protein; preferably, in step ii, the first functional complex binds to the double-stranded target nucleic acid or nucleic acid molecule T through the first PAM sequence and the first gRNA, and fragments it; and the second functional complex binds to the double-stranded target nucleic acid or nucleic acid molecule T through the second PAM sequence and the second gRNA, and fragments it;
preferably, the nucleic acid molecule of interest comprises a third PAM sequence recognized by the third Cas protein and a fourth PAM sequence recognized by the fourth Cas protein; preferably, in step ii, the third functional complex binds to the nucleic acid molecule of interest through the third PAM sequence and the third gRNA, and fragment it; and the fourth functional complex binds to the nucleic acid molecule of interest through the fourth PAM sequence and the fourth gRNA, and fragment it;
preferably, the nucleic acid molecule of interest is a genomic DNA of the cell;
preferably, the first Cas protein, the first gRNA, the first DNA polymerase or the first tag primer is as defined in any one of claims 1-9;
preferably, the second Cas protein, the second gRNA, the second DNA polymerase or the second tag primer is as defined in any one of claims 10-18;
preferably, the third Cas protein and the third gRNA are as defined in any one of claims 19-27;
preferably, the fourth Cas protein and the fourth gRNA are as defined in any one of claims 28-37.

64. The method according to claim 63, wherein the first, second, third and fourth Cas proteins are the same Cas protein, and the second DNA polymerase is the same as the first DNA polymerase; wherein, the first Cas protein forms the first, second, third and fourth functional complexes with the first, second, third and fourth gRNAs, respectively, and the first DNA polymerase extends the target nucleic acid fragment F1 by respectively using the first tag primer and the second tag primer annealed to the target nucleic acid fragment F1 as templates, to form a target nucleic acid fragment F2 having a first overhang and a second overhang;
preferably, in step i, the first Cas protein or nucleic acid molecule A1, the first DNA polymerase or nucleic acid molecule B 1, the first gRNA or nucleic acid molecule C1, the first tag primer or nucleic acid molecule D1, the second gRNA or nucleic acid molecule C2, the second tag primer or nucleic acid molecule D2, the third gRNA or nucleic acid molecule C3 and the fourth gRNA or nucleic acid molecule C4 are delivered into a cell, to provide the first Cas protein, the first gRNA, the first DNA polymerase, the first tag primer, the second gRNA, the second tag primer, the third gRNA and the fourth gRNA in the cell;
preferably, in step i, the nucleic acid molecule A1, the nucleic acid molecule B1, the first gRNA or nucleic acid molecule C1, the first tag primer or nucleic acid molecule D1, the second gRNA or nucleic acid molecule C2, the second tag primer or nucleic acid molecule D2, the third gRNA or nucleic acid molecule C3 and the fourth gRNA or nucleic acid molecule C4 are delivered into a cell to provide the first Cas protein, the first gRNA, the first DNA polymerase, the first tag primer, the second gRNA, the second tag primer, the third gRNA, and the fourth gRNA in the cell;
preferably, in step i, the nucleic acid molecules A1, B1, C1, D1, C2, D2, C3 and C4 are delivered into a cell to provide the first Cas protein, the first gRNA, the first DNA polymerase, the first tag primer, the second gRNA, the second tag primer, the third gRNA and the fourth gRNA in the cell;
preferably, the nucleic acid molecule A1 and the nucleic acid molecule B1 are comprised in the same or different expression vectors (e.g., eukaryotic expression vectors); preferably, the nucleic acid molecule A1 and the nucleic acid molecule B1 are capable of expressing the first Cas protein and the first DNA polymerase in isolation state, or capable of expressing a first fusion protein comprising the first Cas protein and the first DNA polymerase; preferably, in step i, a nucleic acid molecule capable expressing the first Cas protein and the first DNA polymerase in isolation state or a nucleic acid molecule comprising a nucleotide sequence encoding the first fusion protein is delivered into a cell, and expressed in the cell to provide the first Cas protein and the first DNA polymerase in the cell;
preferably, the nucleic acid molecule C1 and the nucleic acid molecule D1 are comprised in the same expression vector (e.g., an eukaryotic expression vector); preferably, the nucleic acid molecule C1 and the nucleic acid molecule D1 are capable transcribing a first PegRNA comprising the first gRNA and the first tag primer; preferably, in step i, the first PegRNA is delivered into a cell to provide the first gRNA and the first tag primer in the cell, or, a nucleic acid molecule comprising a nucleotide sequence encoding the first PegRNA is delivered into a cell, and the first PegRNA is transcribed in the cell to provide the first gRNA and the first tag primer in the cell;
preferably, the nucleic acid molecule C2 and the nucleic acid molecule D2 are comprised in the same expression vector (e.g., an eukaryotic expression vector); preferably, the nucleic acid molecule C2 and the nucleic acid molecule D2 are capable of transcribing a second PegRNA comprising the second gRNA and the second tag primer in a cell; preferably, in step i, the second PegRNA is delivered into a cell to provide the second gRNA and the second tag primer in the cell, or, a nucleic acid molecule comprising a nucleotide sequence encoding the second PegRNA is delivered into a cell, and the second PegRNA is transcribed in the cell to provide the second gRNA and the second tag primer in the cell;
preferably, in step i, a nucleic acid molecule capable of expressing the first Cas protein and the first DNA polymerase in isolation state or a nucleic acid molecule comprising a nucleotide sequence encoding the first fusion protein, a nucleic acid molecule comprising a nucleotide sequence encoding the first PegRNA, a nucleic acid molecule comprising a nucleotide sequence encoding the second PegRNA, a nucleic acid molecule comprising a nucleotide sequence encoding the third gRNA, and a nucleic acid molecule comprising a nucleotide sequence encoding the fourth gRNA are delivered into a cell, and transcribed and expressed in the cell, thereby providing the first Cas protein, the first gRNA, the first DNA polymerase, the first tag primer, the second gRNA, the second tag primer, the third gRNA, and the fourth gRNA in the cell.

65. The method according to claim 62, wherein the method comprises the steps of:
i. providing the double-stranded target nucleic acid and the nucleic acid molecule of interest; and
providing the first, second, third and fourth Cas proteins, the first, second, third and fourth gRNAs, the first, second, third and fourth DNA polymerases, and the first, second, third and fourth tag primers;
ii. contacting the double-stranded target nucleic acid with the first and second Cas proteins, the first and second gRNAs, the first and second DNA polymerases, and the first and second tag primers, and contacting the nucleic acid molecule of interest with the third and fourth Cas proteins, the third and fourth gRNAs, the third and fourth DNA polymerases, and the third and fourth tag primers;
preferably, in step ii:
the first Cas protein binds to the first gRNA to form a first functional complex, the second Cas protein binds to the second gRNA to form a second functional complex, the third Cas protein binds to the third gRNA to form a third functional complex, and the fourth Cas protein binds to the fourth gRNA to form a fourth functional complex; and,
the first and second functional complexes bind and fragment the double-stranded target nucleic acid to form a target nucleic acid fragment F 1, and the third and fourth functional complexes bind and fragment the nucleic acid molecule of interest to form fragmented nucleotide fragments a1, a2, and a3; and,
the first tag primer is hybridized or annealed to the 3' end of one nucleic acid strand of the target nucleic acid fragment F 1 through the first target-binding sequence; and the second tag primer is hybridized or annealed to the 3' end of the other nucleic acid strand of the target nucleic acid fragment F1 through the second target-binding sequence; and,
the first DNA polymerase and the second DNA polymerase extend the target nucleic acid fragment F1 by respectively using the first tag primer and the second tag primer annealed to the target nucleic acid fragment F 1 as templates to form a target nucleic acid fragment F2 having a first overhang and a second overhang; wherein the first overhang and the second overhang are capable of being hybridized or annealed to the fragmented nucleotide fragments a1 and a3, respectively; and,
the third tag primer is hybridized or annealed to the 3' end of one nucleic acid strand of the nucleotide fragment a1 through the third target-binding sequence, wherein the 3' end is formed by fragmenting the nucleic acid molecule of interest by the third functional complex; and, the fourth tag primer is hybridized or annealed to the 3' end of the other nucleic acid strand of the nucleotide fragment a3 through the fourth target-binding sequence, wherein the 3' end is formed by fragmenting the nucleic acid molecule of interest by the fourth functional complex; and,
the third DNA polymerase extends the nucleotide fragment a1 by using the third tag primer annealed to the nucleotide fragment a1 as a template to form a nucleotide fragment a1 having a third overhang; and, the fourth DNA polymerase extends the nucleotide fragment a3 by using the fourth tag primer annealed to the nucleotide fragment a3 as a template to form a nucleotide fragment a3 having a fourth overhang; wherein the third overhang and the fourth overhang are capable of being hybridized or annealed to the target nucleic acid fragment F2, respectively; and,
through the first, second, third and fourth overhangs, the target nucleic acid fragment F2 is hybridized or annealed to the nucleotide fragments a1 and a3, respectively, and then ligated between the nucleotide fragments a1 and a3, thereby substituting the nucleotide fragment a2 in the nucleic acid molecule of interest with the target nucleic acid fragment;
preferably, the first overhang is capable of being hybridized or annealed to the 3' end or 3' portion of one nucleic acid strand of the nucleotide fragment a1, and the 3' end or 3' portion is formed by fragmenting the nucleic acid molecule of interest by the third functional complex; preferably, the first overhang is capable of being hybridized or annealed to the third overhang of the nucleotide fragment a1 or its upstream nucleotide sequence;
preferably, the second overhang is capable of being hybridized or annealed to the 3' end or 3' portion of one nucleic acid strand of the nucleotide fragment a3, and the 3' end or 3' portion is formed by fragmenting the nucleic acid molecule of interest by the fourth functional complex; preferably, the second overhang is capable of being hybridized or annealed to the fourth overhang of the nucleotide fragment a3 or its upstream nucleotide sequence;
preferably, the third overhang is capable of being hybridized or annealed to the first overhang or its upstream nucleotide sequence;
preferably, the fourth overhang is capable of being hybridized or annealed to the second overhang or its upstream nucleotide sequence;
preferably, the method is performed in a cell;
preferably, in step i, the first Cas protein or nucleic acid molecule A1, the first DNA polymerase or nucleic acid molecule B 1, the first gRNA or nucleic acid molecule C1, the first tag primer or nucleic acid molecule D1, the second Cas protein or nucleic acid molecule A2, the second DNA polymerase or nucleic acid molecule B2, the second gRNA or nucleic acid molecule C2, the second tag primer or nucleic acid molecule D2, the third Cas protein or nucleic acid molecule A3, the third DNA polymerase or nucleic acid molecule B3, the third gRNA or nucleic acid molecule C3, the third tag primer or nucleic acid molecule D3, the fourth Cas protein or nucleic acid molecule A4, the fourth DNA polymerase or nucleic acid molecule B4, the fourth gRNA or nucleic acid molecule C4, and the fourth tag primer or nucleic acid molecule D4 are delivered into a cell, to provide the first, second, third and fourth Cas proteins, the first, second, third and fourth gRNAs, the first, second, third and fourth DNA polymerases, and the first, second, third and fourth tag primers in the cell;
preferably, in step i, the nucleic acid molecule A1, the nucleic acid molecule B1, the first gRNA or nucleic acid molecule C1, the first tag primer or nucleic acid molecule D1, the nucleic acid molecule A2, the nucleic acid molecule B2, the second gRNA or nucleic acid molecule C2, the second tag primer or nucleic acid molecule D2, the nucleic acid molecule A3, the nucleic acid molecule B3, the third gRNA or nucleic acid molecule C3, the third tag primer or nucleic acid molecule D3, the nucleic acid molecule A4, the nucleic acid molecule B4, the fourth gRNA or nucleic acid molecule C4, and the fourth tag primer or nucleic acid molecule D4 are delivered into a cell to provide the first, second, third and fourth Cas proteins, the first, second, third and fourth gRNAs, the first, second, third and fourth DNA polymerases, and the first, second, third and fourth tag primers in the cell;
preferably, in step i, the nucleic acid molecules A1, B1, C1, D1, A2, B2, C2, D2, A3, B3, C3, D3, A4, B4, C4, D4 are delivered into a cell to providing the first, second, third and fourth Cas proteins, the first, second, third and fourth gRNAs, the first, second, third and fourth DNA polymerases, and the first, second, third and fourth tag primers in the cell;
preferably, in step i, the double-stranded target nucleic acid or a nucleic acid molecule T comprising the double-stranded target nucleic acid is delivered into a cell to provide the double-stranded target nucleic acid in the cell;
preferably, the double-stranded target nucleic acid or nucleic acid molecule T comprises a first PAM sequence recognized by the first Cas protein and a second PAM sequence recognized by the second Cas protein; preferably, in step ii, the first functional complex binds to the double-stranded target nucleic acid or nucleic acid molecule T through the first PAM sequence and the first gRNA, and fragments it; and the second functional complex binds to the double-stranded target nucleic acid or nucleic acid molecule T through the second PAM sequence and the second gRNA, and fragments it;
preferably, the nucleic acid molecule of interest comprises a third PAM sequence recognized by the third Cas protein and a fourth PAM sequence recognized by the fourth Cas protein; preferably, in step ii, the third functional complex binds to the nucleic acid molecule of interest through the third PAM sequence and the third gRNA, and fragment it; and the fourth functional complex binds to the nucleic acid molecule of interest through the fourth PAM sequence and the fourth gRNA, and fragment it;
preferably, the nucleic acid molecule of interest is a genomic DNA of the cell;
preferably, the first Cas protein, the first gRNA, the first DNA polymerase or the first tag primer is as defined in any one of claims 1-9;
preferably, the second Cas protein, the second gRNA, the second DNA polymerase or the second tag primer is as defined in any one of claims 10-18;
preferably, the third Cas protein, the third gRNA, the third DNA polymerase or the third tag primer is as defined in any one of claims 19-27;
preferably, the fourth Cas protein, the fourth gRNA, the fourth DNA polymerase or the fourth tag primer is as defined in any one of claims 28-37.

66. The method according to claim 65, wherein the first, second, third and fourth Cas proteins are the same Cas protein, and the first, second, third and fourth DNA polymerases are the same DNA polymerase; wherein, the first Cas protein forms the first, second, third and fourth functional complexes with the first, second, third and fourth gRNAs, respectively; and, the first DNA polymerase extends the target nucleic acid fragment F1 by respectively using the first tag primer and the second tag primer annealed to the target nucleic acid fragment F1 as templates to form a target nucleic acid fragment F2 having a first overhang and a second overhang; and, the first DNA polymerase extends the nucleotide fragments a1 and a3 by using the third tag primer and the fourth tag primer as templates to form third and fourth overhangs;
preferably, in step i, the first Cas protein or nucleic acid molecule A1, the first DNA polymerase or nucleic acid molecule B 1, the first gRNA or nucleic acid molecule C1, the first tag primer or nucleic acid molecule D1, the second gRNA or nucleic acid molecule C2, the second tag primer or nucleic acid molecule D2, the third gRNA or nucleic acid molecule C3, the third tag primer or nucleic acid molecule D3, the fourth gRNA or nucleic acid molecule C4, and the fourth tag primer or nucleic acid molecule D4 are delivered into a cell to provide the first Cas protein, the first DNA polymerase, the first, second, third and fourth gRNAs, and the first, second, third, and fourth tag primers in the cell;
preferably, in step i, the nucleic acid molecule A1, the nucleic acid molecule B1, the first gRNA or nucleic acid molecule C1, the first tag primer or nucleic acid molecule D1, the second gRNA or nucleic acid molecule C2, the second tag primer or nucleic acid molecule D2, the third gRNA or nucleic acid molecule C3, the third tag primer or nucleic acid molecule D3, the fourth gRNA or nucleic acid molecule C4, and the fourth tag primer or nucleic acid molecule D4 are delivered into a cell to provide the first Cas protein, the first DNA polymerase, the first, second, third and fourth gRNAs, and the first, second, third and fourth tag primers in the cell;
preferably, in step i, the nucleic acid molecules A1, B1, C1, D1, C2, D2, C3, D3, C4 and D4 are delivered into a cell to provide the first Cas protein, the first a DNA polymerase, the first, second, third and fourth gRNAs, and the first, second, third and fourth tag primers in the cell;
preferably, the nucleic acid molecule A1 and the nucleic acid molecule B1 are comprised in the same or different expression vectors (e.g., eukaryotic expression vectors); preferably, the nucleic acid molecule A1 and the nucleic acid molecule B1 are capable of expressing the first Cas protein and the first DNA polymerase in isolation state, or capable of expressing a first fusion protein comprising the first Cas protein and the first DNA polymerase; preferably, in step i, a nucleic acid molecule capable of expressing the first Cas protein and the first DNA polymerase in isolation state or a nucleic acid molecule comprising a nucleotide sequence encoding the first fusion protein is delivered into a cell, and expressed in the cell to provide the first Cas protein and the first DNA polymerase in the cell;
preferably, the nucleic acid molecule C1 and the nucleic acid molecule D1 are comprised in the same expression vector (e.g., an eukaryotic expression vector); preferably, the nucleic acid molecule C1 and the nucleic acid molecule D1 are capable of transcribing a first PegRNA comprising the first gRNA and the first tag primer in a cell; preferably, in step i, the first PegRNA is delivered into a cell to provide the first gRNA and the first tag primer in the cell, or, a nucleic acid molecule comprising a nucleotide sequence encoding the first PegRNA is delivered into a cell, and the first PegRNA is transcribed in the cell to provide the first gRNA and the first tag primer in the cell;
preferably, the nucleic acid molecule C2 and the nucleic acid molecule D2 are comprised in the same expression vector (for example, an eukaryotic expression vector); preferably, the nucleic acid molecule C2 and the nucleic acid molecule D2 are capable of transcribing a second PegRNA comprising the second gRNA and the second tag primer in a cell; preferably, in step i, the second PegRNA is delivered into a cell to provide the second gRNA and the second tag primer in the cell, or, a nucleic acid molecule comprising a nucleotide sequence encoding the second PegRNA is delivered into a cell, and the second PegRNA is transcribed in the cell to provide the second gRNA and the second tag primer in the cell;
preferably, the nucleic acid molecule C3 and the nucleic acid molecule D3 are comprised in the same expression vector (e.g., an eukaryotic expression vector); preferably, the nucleic acid molecule C3 and the nucleic acid molecule D3 are capable of transcribing a third PegRNA comprising the third gRNA and the third tag primer in a cell; preferably, in step i, the third PegRNA is delivered into a cell to provide the third gRNA and the third tag primer in the cell, or, a nucleic acid molecule comprising a nucleotide sequence encoding the third PegRNA is delivered into a cell, and the third PegRNA is transcribed in the cell to provide the third gRNA and the third tag primer in the cell;
preferably, the nucleic acid molecule C4 and the nucleic acid molecule D4 are comprised in the same expression vector (e.g., an eukaryotic expression vector); preferably, the nucleic acid molecule C4 and the nucleic acid molecule D4 are capable of transcribing a fourth PegRNA comprising the fourth gRNA and the fourth tag primer in a cell; preferably, in step i, the fourth PegRNA is delivered into a cell to provide the fourth gRNA and the fourth tag primer in the cell, or, a nucleic acid molecule comprising a nucleotide sequence encoding the fourth PegRNA is delivered into a cell, and the fourth PegRNA is transcribed in the cell to provide the fourth gRNA and the fourth tag primer in the cell;
preferably, in step i, a nucleic acid molecule capable of expressing the first Cas protein and the first DNA polymerase in isolation state or a nucleic acid molecule comprising a nucleotide sequence encoding the first fusion protein, a nucleic acid molecule comprising a nucleotide sequence encoding the first PegRNA, a nucleic acid molecule comprising a nucleotide sequence encoding the second PegRNA, a nucleic acid molecule comprising a nucleotide sequence encoding the third PegRNA, and a nucleic acid molecule comprising a nucleotide sequence encoding the fourth PegRNA are delivered into a cell, to express the first fusion protein and transcribe the first, second, third and fourth PegRNAs in the cell, thereby providing the first Cas protein, the first DNA polymerase, the first, second, third and fourth gRNAs, and the first, second, third and fourth tag primers in the cell.
